# EUROPEAN PATENT SPECIFICATION

(11) **EP 3 548 066 B1**
(45) Date of publication and mention of the grant of the patent: **25.11.2026**
(21) Application number: 17832386.1
(22) Date of filing: 01.12.2017
(51) Int. Cl.: A61K 38/48, A61K 38/37, A61P 7/04

(54) **CHIMERIC FACTOR VIII FOR USE IN TREATING HEMOPHIIC ARTHROPATHY**
CHIMÄRER FAKTOR VIII ZUR VERWENDUNG BEI DER BEHANDLUNG DER HÄMOPHILEN ARTHROPATHIE
FACTEUR VIII CHIMÉRIQUE POUR UTILISATION DANS LE TRAITEMENT DE L'ARTHROPATHIE HÉMOPHILIQUE

(30) Priority: 02.12.2016 US 201662429509 P; 07.07.2017 US 201762529896 P; 25.08.2017 US 201762550488 P; 14.09.2017 US 201762558793 P
(43) Date of publication of application: 09.10.2019
(73) Proprietor: Bioverativ Therapeutics Inc., Waltham, MA 02451 (US)
(72) Inventor: DUMONT, Jennifer, Waltham, Massachusetts 02451 (US); JAIN, Nisha, Waltham, Massachusetts 02451 (US); GLAZEBROOK, Desilu, Waltham, Massachusetts 02451 (US)
(74) Representative: Zwicker, Jörk
(86) International application number: PCT/US2017/064302
(87) International publication number: WO 2018/102743

(56) References cited:
- WO-A1-2014/070953
- WO-A1-2014/144549
- G. YOUNG ET AL: "Recombinant factor VIII Fc fusion protein for the prevention and treatment of bleeding in children with severe hemophilia A", JOURNAL OF THROMBOSIS AND HAEMOSTASIS, vol. 13, no. 6, 23 April 2015 (2015-04-23), GB, pages 967 - 977, XP055460215, ISSN: 1538-7933, DOI: 10.1111/jth.12911
- KERLIN BRYCE A ET AL: "Long-Term Efficacy of rFVIIIFc Prophylaxis in Pediatric, Adolescent, and Adult Subjects with Target Joints and Severe Hemophilia A", BLOOD, vol. 126, no. 23, December 2015 (2015-12-01), & 57TH ANNUAL MEETING OF THE AMERICAN-SOCIETY-OF-HEMATOLOGY; ORLANDO, FL, USA; DECEMBER 05 -08, 2015, XP009504173

## Description

### FIELD OF THE DISCLOSURE

The present disclosure relates generally to the field of therapeutics for hemostatic disorders.

### BACKGROUND

Hemophilia is an X-linked bleeding disorder caused by mutations and/or deletions in genes encoding coagulation proteins, in particular the Factor VIII (FVIII) gene, resulting in a deficiency of FVIII activity (hemophilia A), or the Factor IX gene, resulting in a deficiency of FIX activity (hemophilia B) (*see, e.g.,* Peyvandi, F. et al. Haemophilia 12:82-89 (2006)). The disease is characterized by spontaneous hemorrhage and excessive bleeding after trauma. Treatment of hemophilia is by replacement therapy targeting restoration of FVIII and/or FIX activity to prevent spontaneous bleeding (*see, e.g.,* Mannucci, P.M., et al., N. Engl. J. Med. 344:1773-1779 (2001)). Young et al. (Journal of Thrombosis and Hemostasis 13:967-977 (2015)) report prevention and treatment of bleeding in children with severe hemophilia A using recombinant Factor VIII Fc over a course of about 28 weeks. Kerlin et al. (Blood 126(23):3520 (2015)) present results of the phase 3 A-LONG and Kids A-LONG studies and report long-term efficacy of rFVIIIFc in prophylaxis in pediatric, adolescent and adult subjects with target joints and severe hemophilia A.

Over time, the repeated bleeding into muscles and joints, which often begins in early childhood, results in hemophilic arthropathy and joint damage. Hemophilic arthropathy is a common and severe complication associated with hemophilia, which often results in pain, deformity, and disability. The most common patients to suffer from hemophilic arthropathy are young males, between the ages of 3 and 15 years old. While the knees are the most likely joints to be affected, hemophilic arthropathy can also present in the elbow, ankles, shoulders, and the spine. Hemophilic arthropathy is known to be irreversible. Currently available treatment for hemophilic arthropathy is thus limited.

Accordingly, there is a need to develop new ways of treating hemophilic arthropathy.

### BRIEF SUMMARY

One aspect of the present disclosure provides a chimeric protein comprising a factor VIII (FVIII) and a first Fc region derived from human IgG1, for use in reversing hemophilic arthropathy of a target joint in a human subject having hemophilia A, wherein the hemophilic arthropathy comprises synovitis, and wherein the chimeric protein is administered at a dose of from 20 IU/kg to 300 IU/kg, wherein the hemophilic arthropathy is reversed in the subject without surgical intervention, and wherein the administration improves a joint health score in the human subject.

In some embodiments, the administration reduces joint pain in the human.

In certain embodiments, the Fc region specifically binds to a low affinity immunoglobulin gamma Fc region receptor II-b (FcyRIIB). In some embodiments, the Fc region specifically binds to dendritic cell-specific intercellular adhesion molecule-3-grabbing non-integrin (DC-SIGN).

In some embodiments, the human subject is identified using an imaging system. In certain embodiments, the imaging system comprises a radiography, a magnetic resonance imaging, an ultrasonography, power Doppler sonography, or any combination thereof. In some embodiments, the human expresses one or more biomarkers associated with joint inflammation.

In some embodiments, the chimeric protein comprising FVIII-Fc is administered at a dosing interval of about two days, about three days, about four days, about five days, about six days, about seven days, about eight days, about nine days, about ten days, about 11 days, about 12 days, about 13 days, about 14 days, about 15 days, about 16 days, about 17 days, about 18 days, about 19 days, about 20 days, about 21 days, about 22 days, about 23 days, or about 24 days.

In other aspects, the effective amount of the chimeric protein comprising FVIII-Fc is from about 20 IU/kg to about 100 IU/kg. In some embodiments, the chimeric protein comprising FVIII-Fc is administered at a dosing interval of about three days, four days, five days, six days, seven days, eight days, nine days, ten days, 11 days, 12 days, 13 days, 14 days, 15 days, 16 days, 17 days, 18 days, 19 days, 20 days, 21 days, 22 days, 23 days, 24 days, 25 days, 26 days, 27 days, or 28 days.

The invention is defined by the appended claims. Further aspects and embodiments are disclosed in the appended set of claims. Any part of the description which does not fall under the scope of the appended claims is for illustrative purposes only. Any reference to methods of treatment refer to the chimeric protein for use in a method of treatment of the human (or animal) body by therapy.

### BRIEF DESCRIPTION OF THE FIGURES

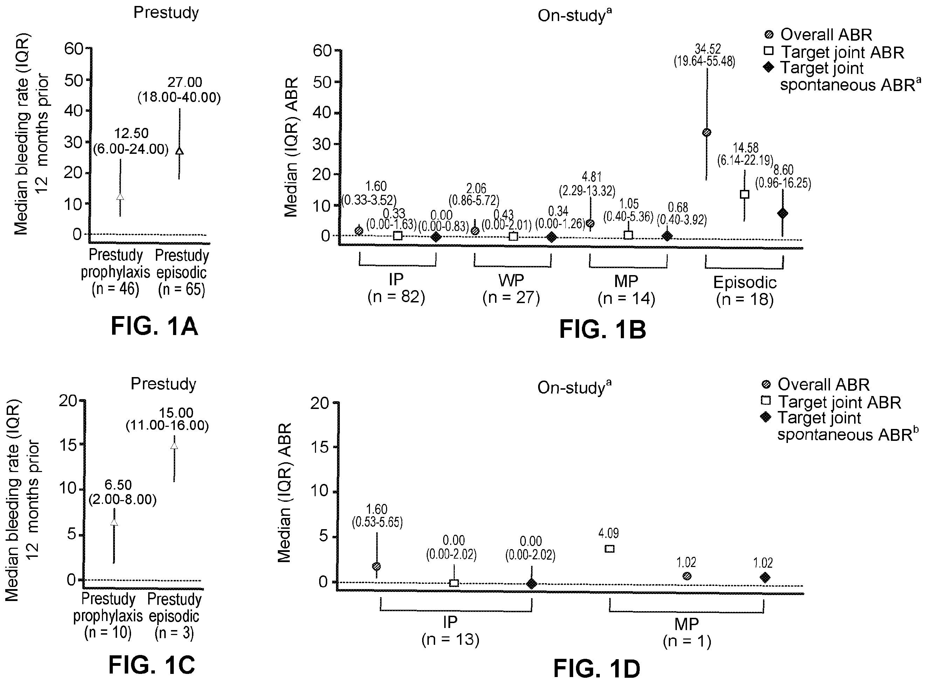

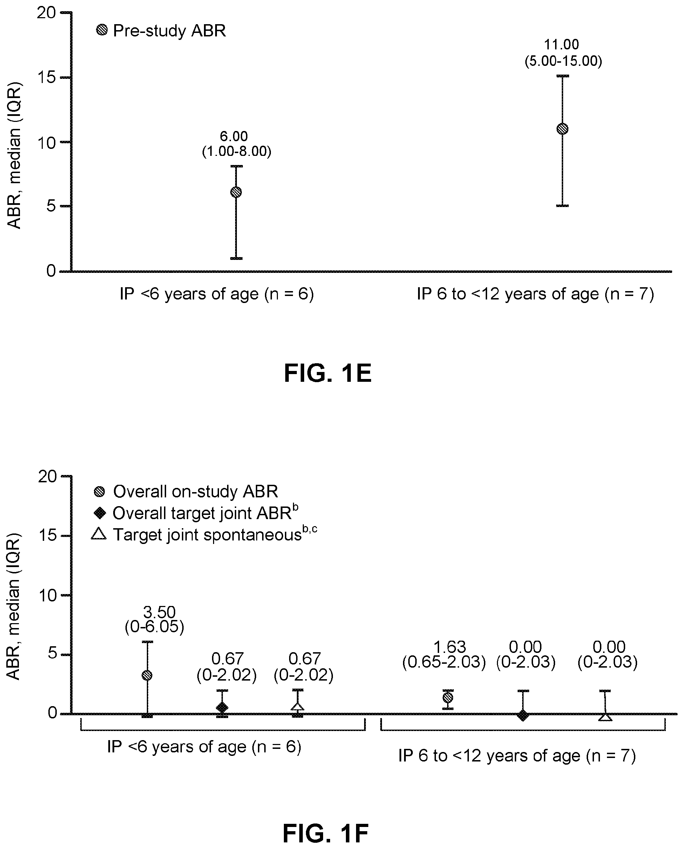

FIGs. 1A-1F show prestudy (FIGs. 1A, 1C, and 1E) and on-study median (FIGs. 1B, 1D, and 1F) (IQR) annualized bleeding rates (ABRs) for subjects from FVIII-Fc study (FIGs 1A and 1B) and FVIII-Fc study for kids (FIGs. 1C-1F) with target joints at baseline. FIGs. 1C-1D show combined data from the FVIII-Fc study for kids, whereas FIGs. 1E-1F show the same data stratified based on the age of the subject (less than 6 years of age and between 6 and less than 12 years of age).

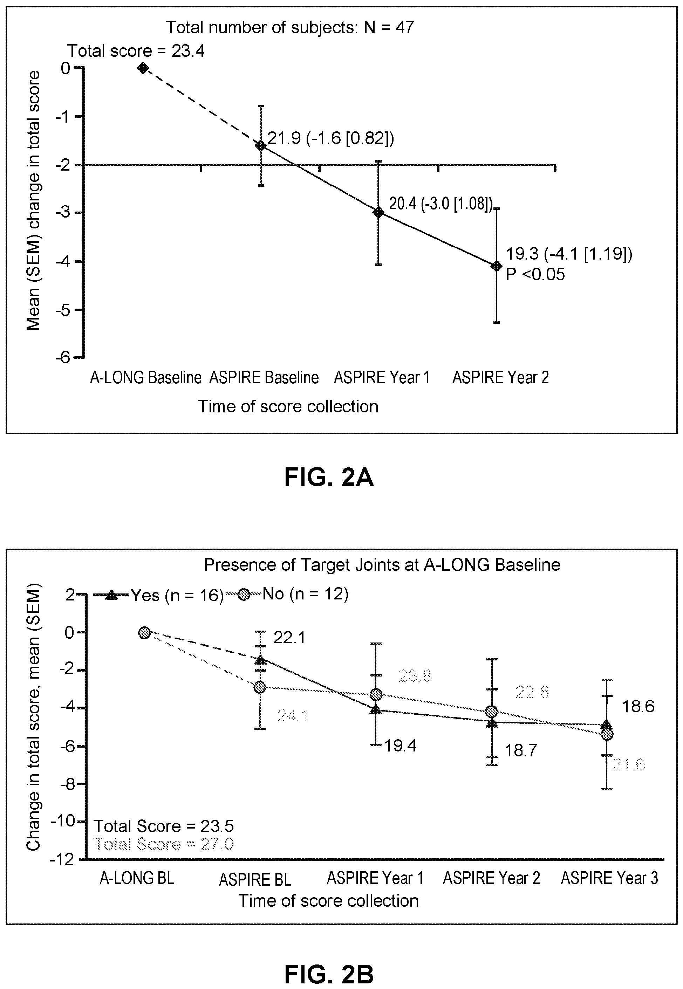

FIGs. 2A-2B show the mean change in total modified Hemophilia Joint Health Score (mHJHS; y-axis) from FVIII-Fc study base line through extension study year 2 (FIG. 2A; x-axis) and through extension study year 3 (FIG. 2B; x-axis). FIG. 2B distinguishes between the presence (yes; triangles) and absence (no; circles) of target joints at baseline.

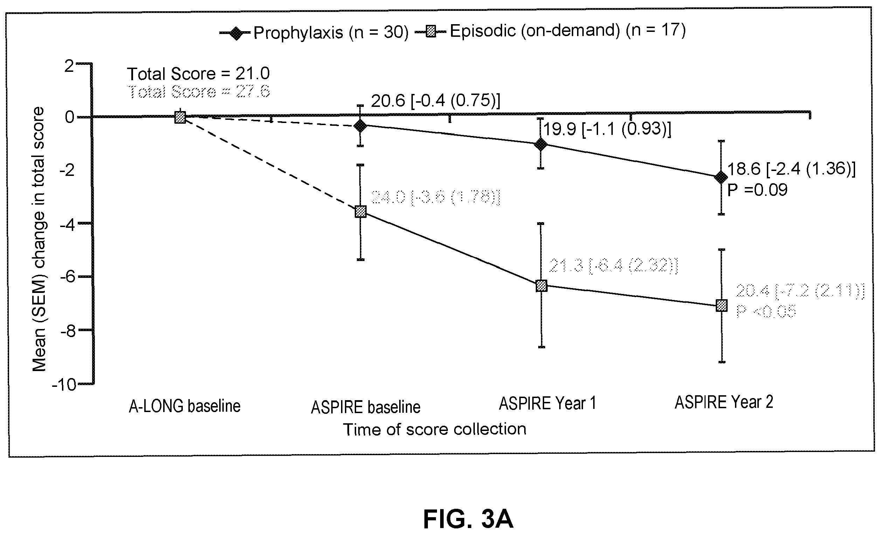

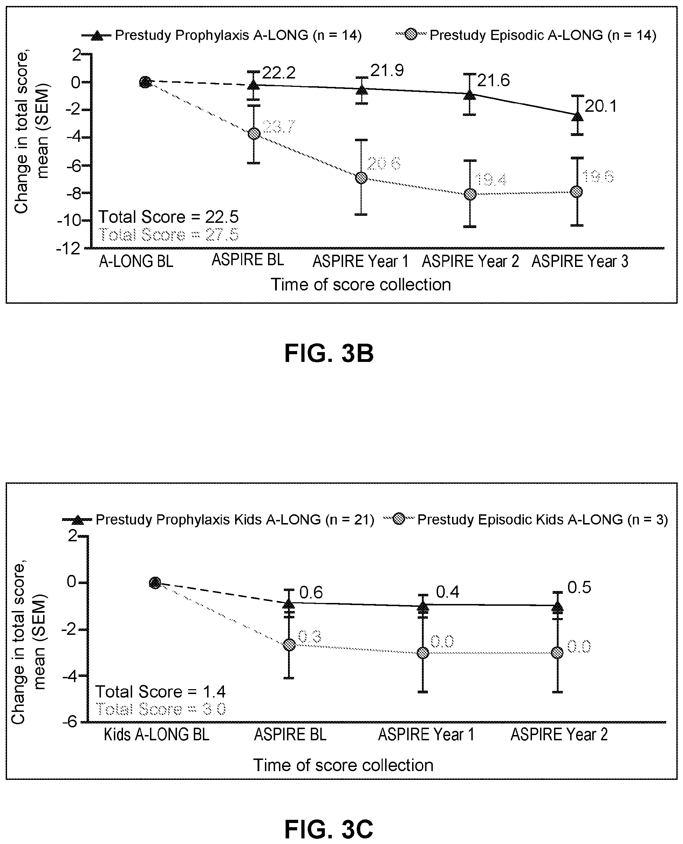

FIGs. 3A-3C show the mean change in total mHJHS (y-axis) from FVIII-Fc study base line through extension study year 2 (FIG. 3A; x-axis) and extension study year 3 (FIG. 3B) and from the FVIII-Fc study for kids through extension year 2 (FIG. 3C; x-axis) for subjects receiving prestudy prophylactic treatment and subjects receiving prestudy episodic (on-demand) treatment.

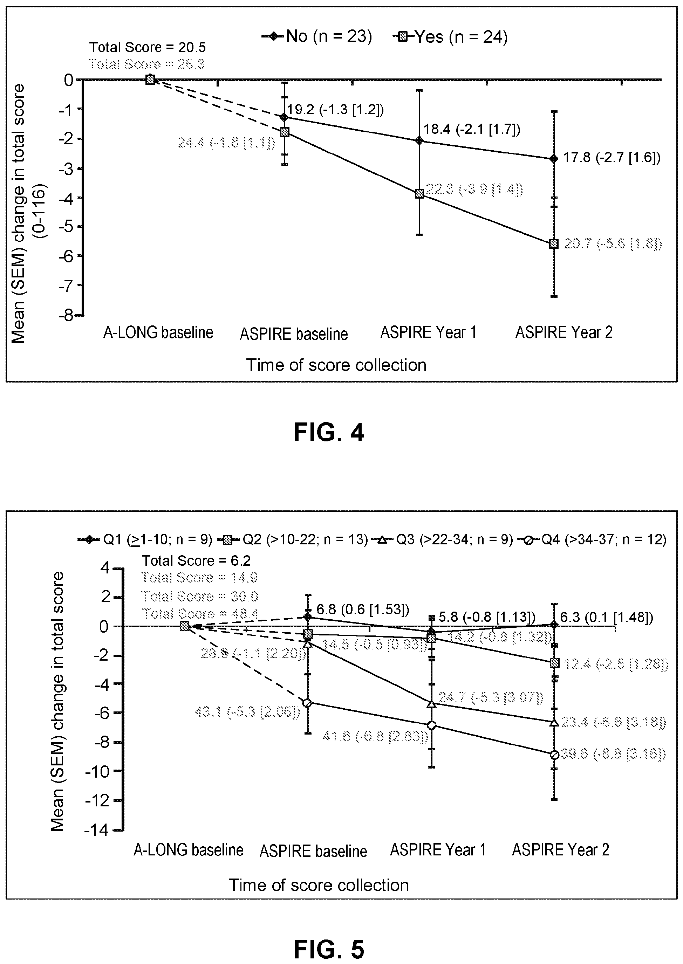

FIG. 4 shows the mean change in total mHJHS (y-axis) from FVIII-Fc study base line through year 2 (x-axis) for subjects having target joints at FVIII-Fc study baseline (squares) and subjects not having target joints at FVIII-Fc study baseline (diamonds).
FIG. 5 shows the mean change in total mHJHS (y-axis) from FVIII-Fc study base line through year 2 (x-axis) for subjects in the lowest quartile of impairment (Q1; ≥1-10) in mHJHS scores at FVIII-Fc study baseline (diamonds), the second lowest quartile of impairment (Q2; ≥10-22) in mHJHS scores at FVIII-Fc study baseline (squares); the second highest quartile of impairment (Q3; ≥22-34) in mHJHS scores at FVIII-Fc study baseline (triangles), and the highest quartile of impairment (Q4; ≥34-37) in mHJHS scores at FVIII-Fc study baseline (diamonds).

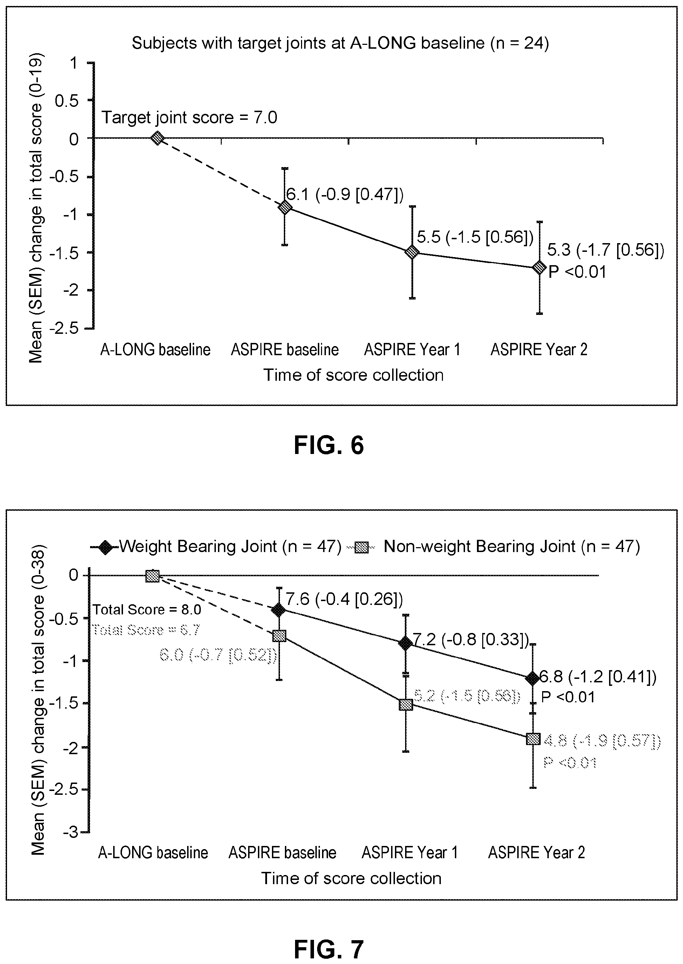

FIG. 6 shows the mean change in total mHJHS (y-axis) from FVIII-Fc study base line through year 2 (x-axis) for subjects having target joints at FVIII-Fc study baseline.
FIG. 7 shows the mean change in total mHJHS (y-axis) from FVIII-Fc study base line through year 2 (x-axis) for weight bearing joints (diamonds) and non-weight bearing target joints (squares).

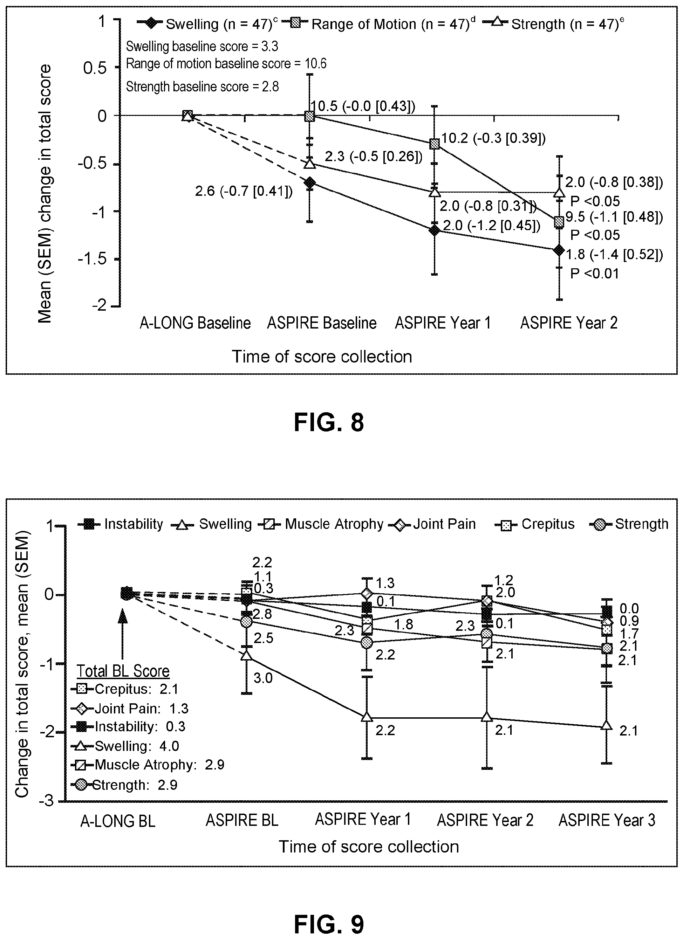

FIG. 8 shows the mean change in mHJHS (y-axis) from FVIII-Fc study base line through year 2 (x-axis) for swelling (diamonds), range of motion (squares), and strength (triangles).
FIG. 9 shows the mean (SEM) change in mHJHS (y-axis) in FVIII-Fc study patients for joint instability (dark grey squares), swelling (black triangles), muscle atrophy (light grey squares), joint pain (light grey diamonds), crepitus (black squares), and strength (light grey circles). Total Baseline (BL) Scores for each mHJHS measurement are shown.

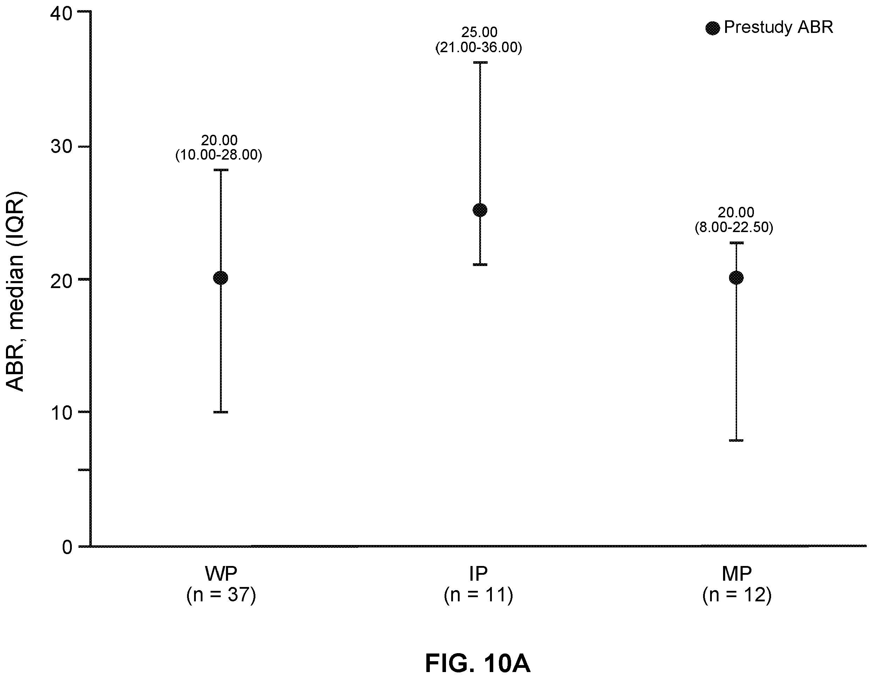

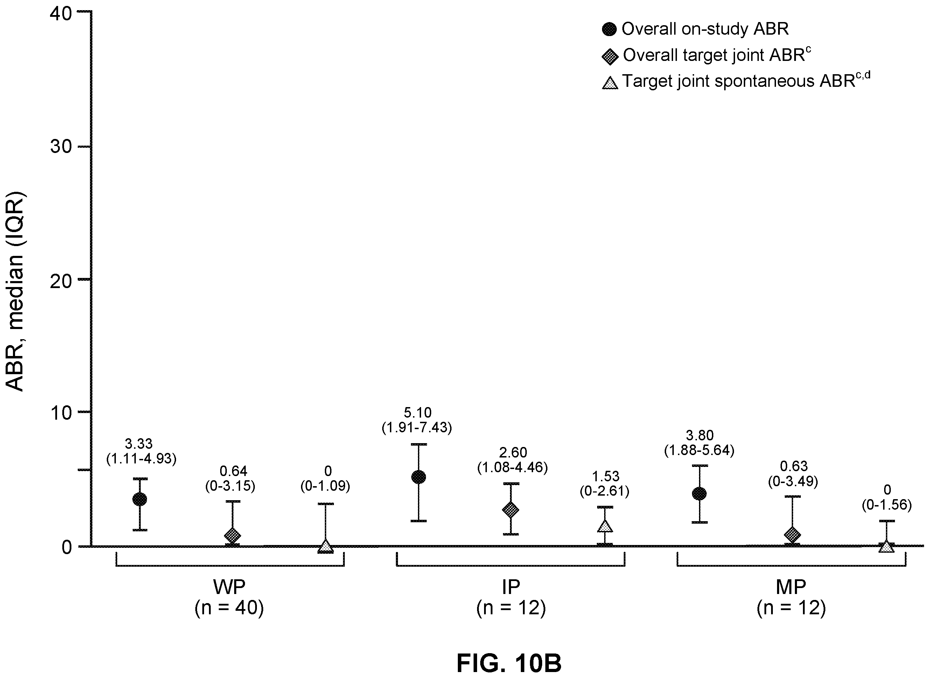

FIGs. 10A-10B show prestudy (FIG. 10A) and on-study median (FIG. 10B) (IQR) annualized bleeding rates (ABRs) for subjects from a rFIXFc study. FIG. 10B further shows overall on-study ABR (dark circles), overall target joint ABR (grey diamonds), and target joint spontaneous ABR (grey triangles). WP = weekly prophylaxis; IP = individualized interval prophylaxis; and MP = modified prophylaxis (FIGs. 10A-10B).

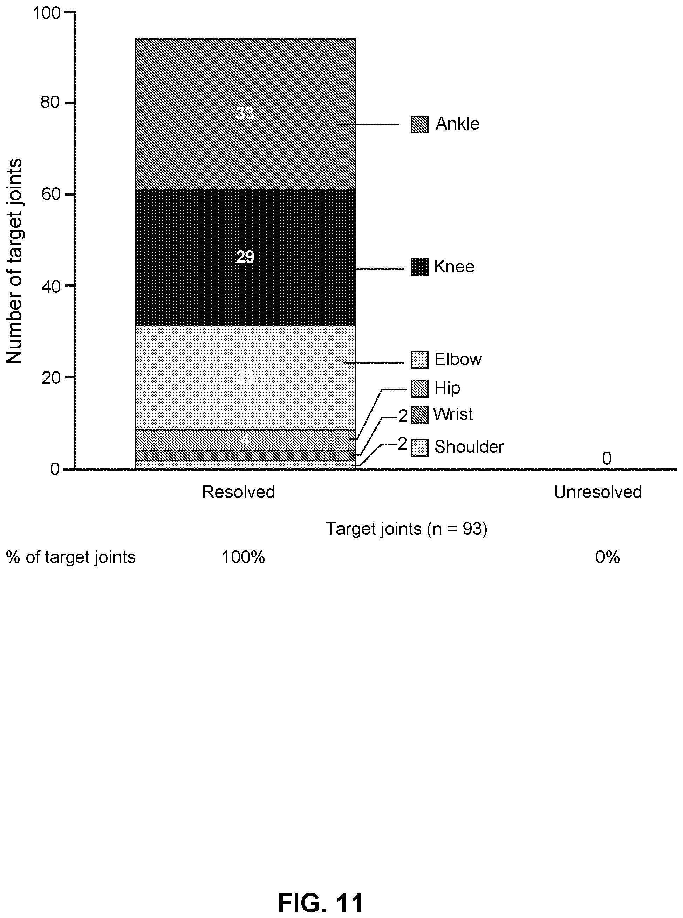

FIG. 11 is a graphical representation showing the number of evaluable target joints (ankle, knee, elbow, hip, wrist, and shoulder) resolved and unresolved in those subjects (n = 37) with at least twelve months of consecutive follow-up time who had not undergone joint surgery within twelve months since the start of follow-up. The number (n) of each target joint is superimposed over the relevant data, with a total of 93 target joints evaluated. The percent of target joints resolved (100%) and unresolved (0%) is shown below the x-axis.

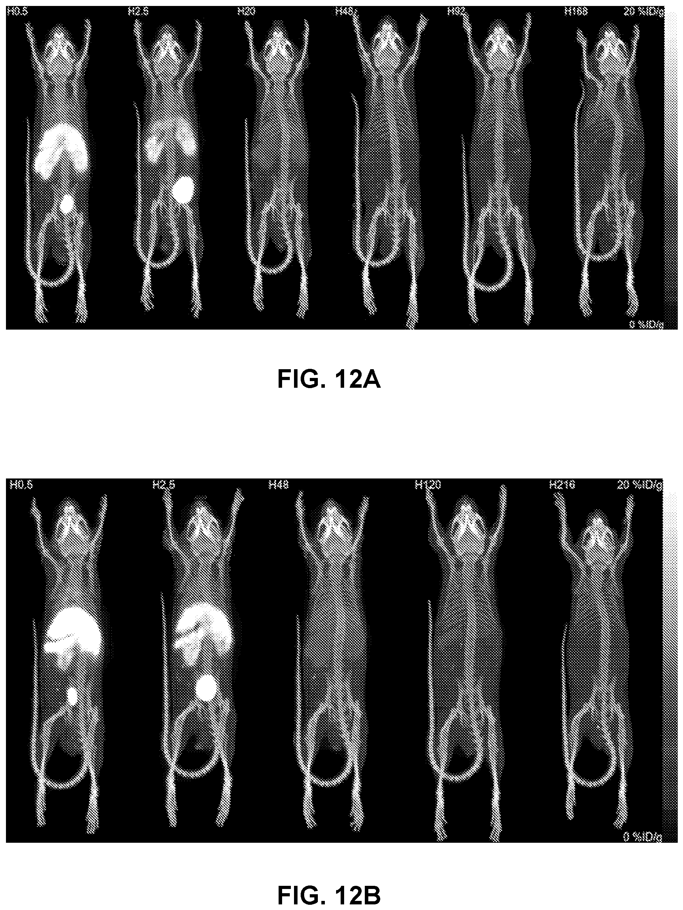

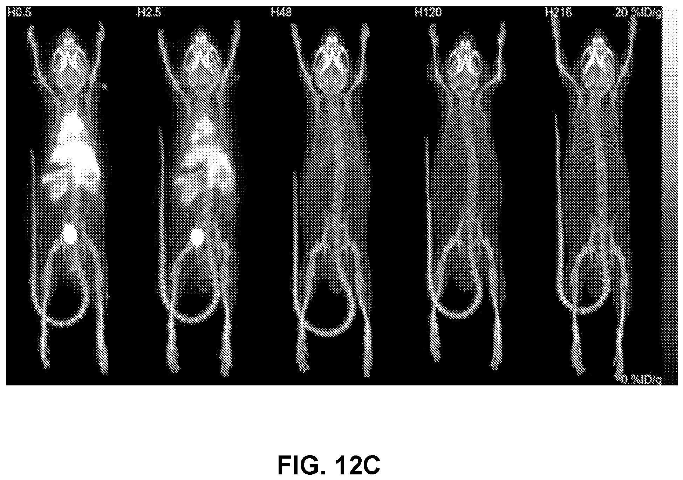

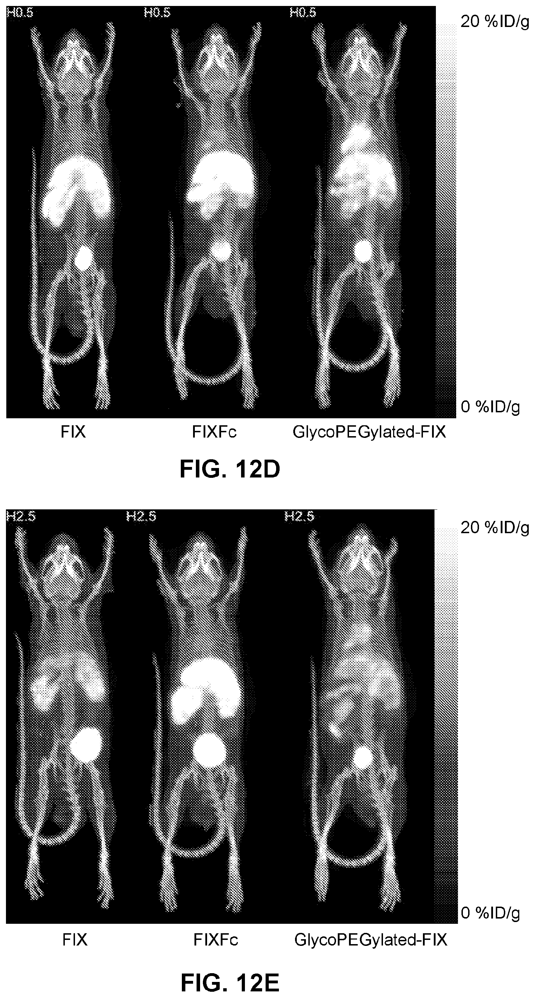

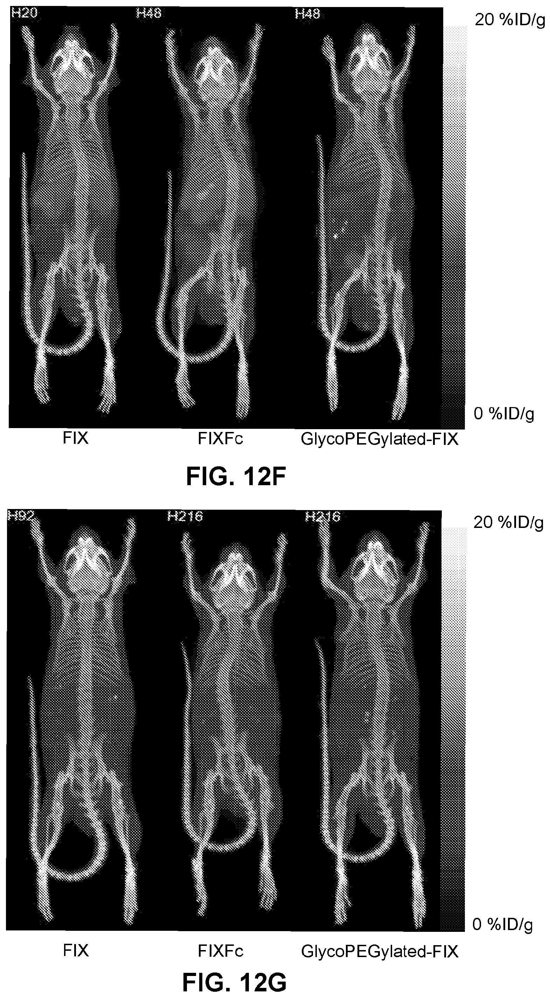

FIGs. 12A-12C are Single Photon Emission Tomography (SPECT) images of mice administered ¹²⁵I-SIB-labeled FIX (FIG. 12A), ¹²⁵I-SIB-labeled FIXFc (FIG. 12B), or ¹²⁵I-SIB-labeled GlycoPEGylated-FIX (FIG. 12C). FIGs. 12D-12G show direct comparisons of mice administered ¹²⁵I-SIB-labeled FIX, ¹²⁵I-SIB-labeled FIXFc, or ¹²⁵I-SIB-labeled GlycoPEGylated-FIX at various time points. Heat maps indicate the relative concentration (%ID/g) of ¹²⁵I-SIB label in each mouse (FIGs. 12A-12G).

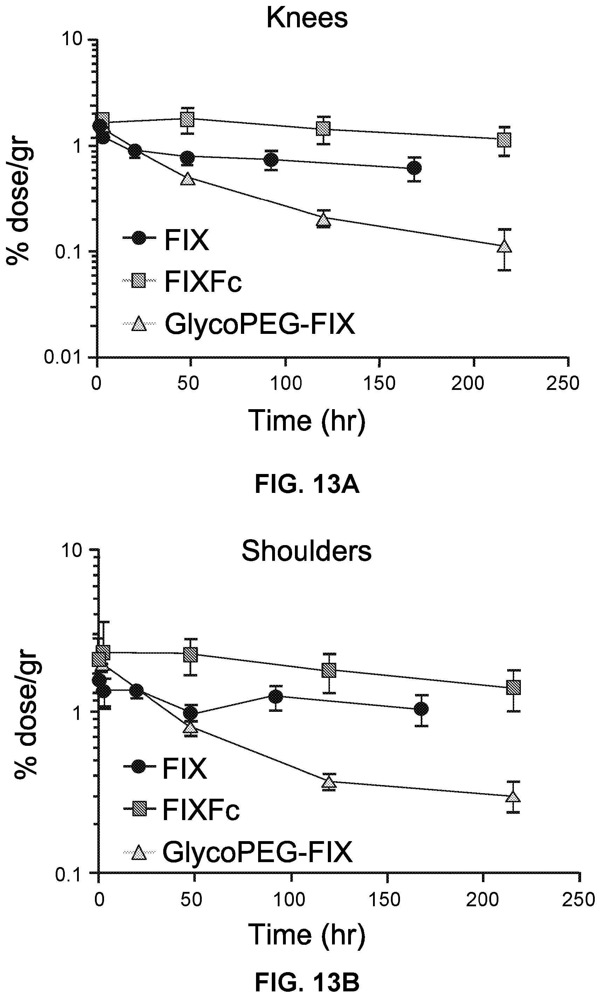

FIGs. 13A-13B are graphs illustrating the intensity of ¹²⁵I-SIB label localization in the mice shown in FIGs. 12A-12G, following administration of ¹²⁵I-SIB-labeled FIX, ¹²⁵I-SIB-labeled FIXFc, or ¹²⁵I-SIB-labeled GlycoPEGylated-FIX over time in the knees (FIG. 13A) and shoulders (FIG. 13B). Data was collected for both the right and left knees and the right and left shoulders, which were combined to generate the data shown (FIGs. 13A and 13B, respectively).

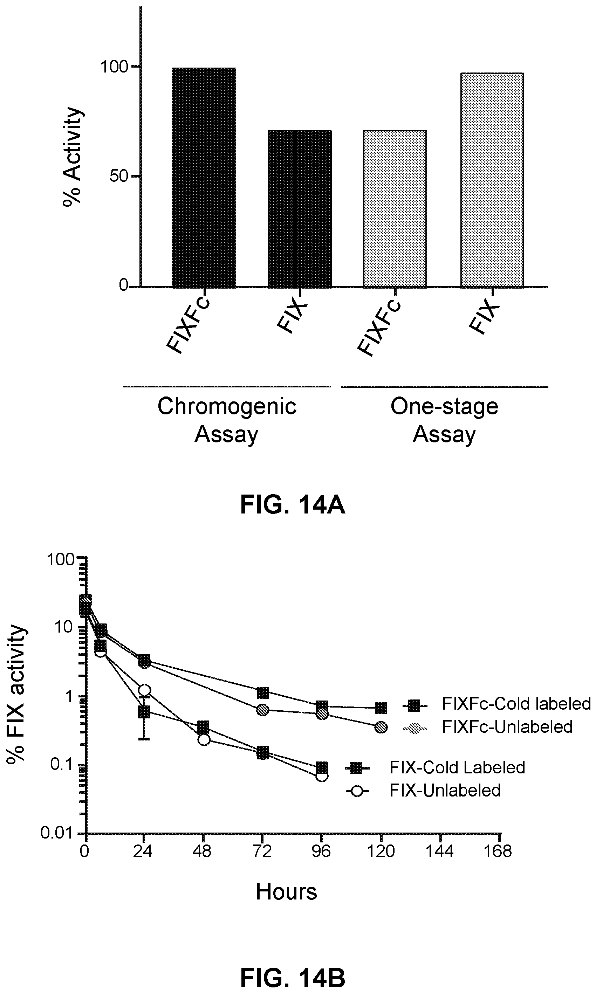

FIG. 14A is a graph illustrating the relative activity of labeled FIX and labeled FIXFc, as compared to unlabeled FIX and FIXFc, and as measured by chromogenic assay or one-stage assay. FIG. 14B is a graph illustrating the pharmacokinetics of labeled and unlabeled FIX molecules in HemB mice.

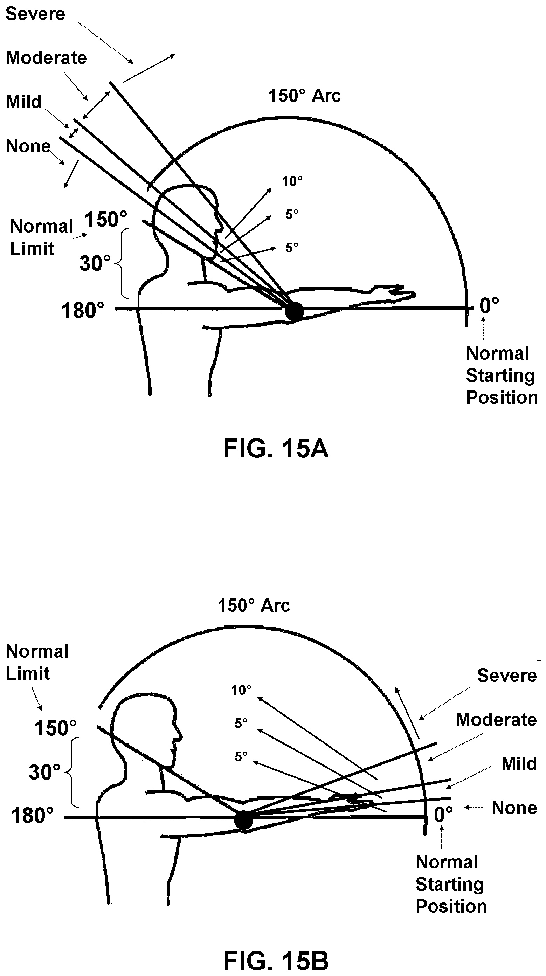

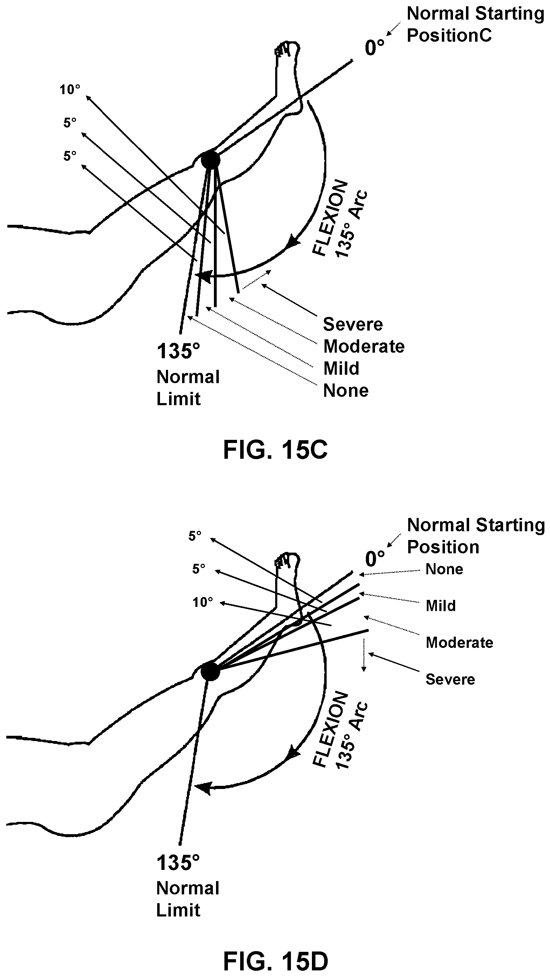

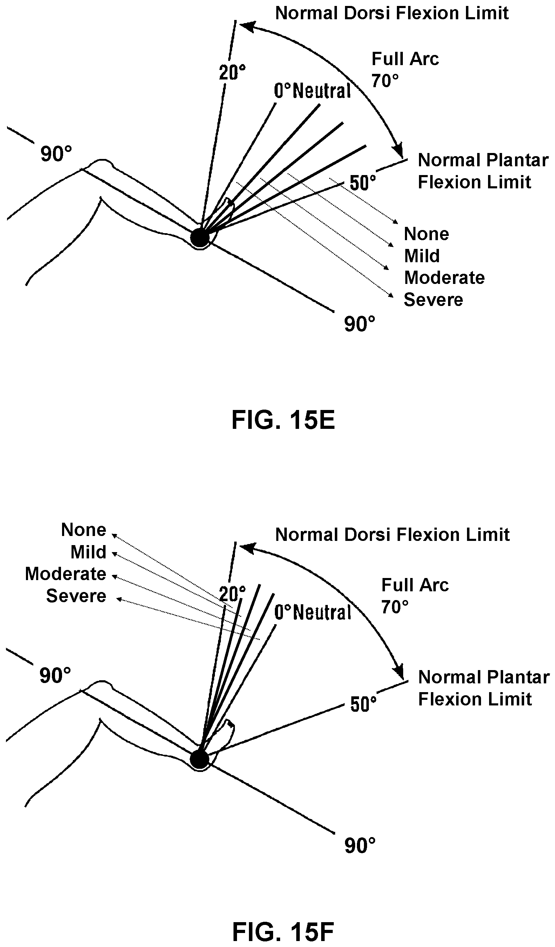

FIGs. 15A-15F are drawings illustrating the range of motions for the elbow (flexion: FIG. 15A; and extension: FIG. 15B), knee (flexion: FIG. 15C; and extension: FIG. 15D), and ankle (plantar flexion: FIG. 15E; and dorsi flexion: FIG. 15F) joints, with the modified hemophilia joint health scores (HJHS) and degrees of flexion/extension overlaid.

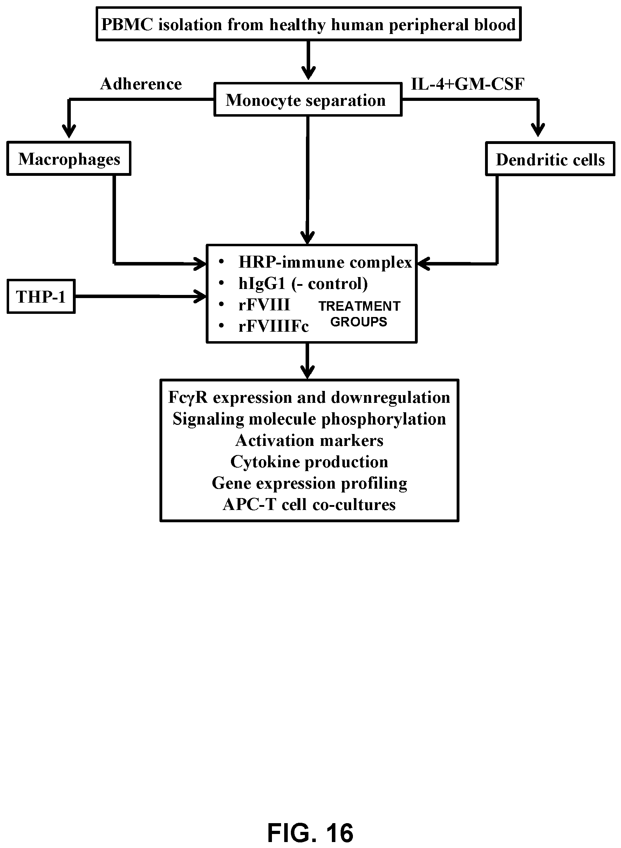

FIG. 16 is a flow diagram, outlining the methods used to investigate the effects of rFVIIIFc on FcγR binding, internalization, signaling and cytokine production, and gene expression changes, as well as subsequent interactions and effects on T cells *in vitro.*

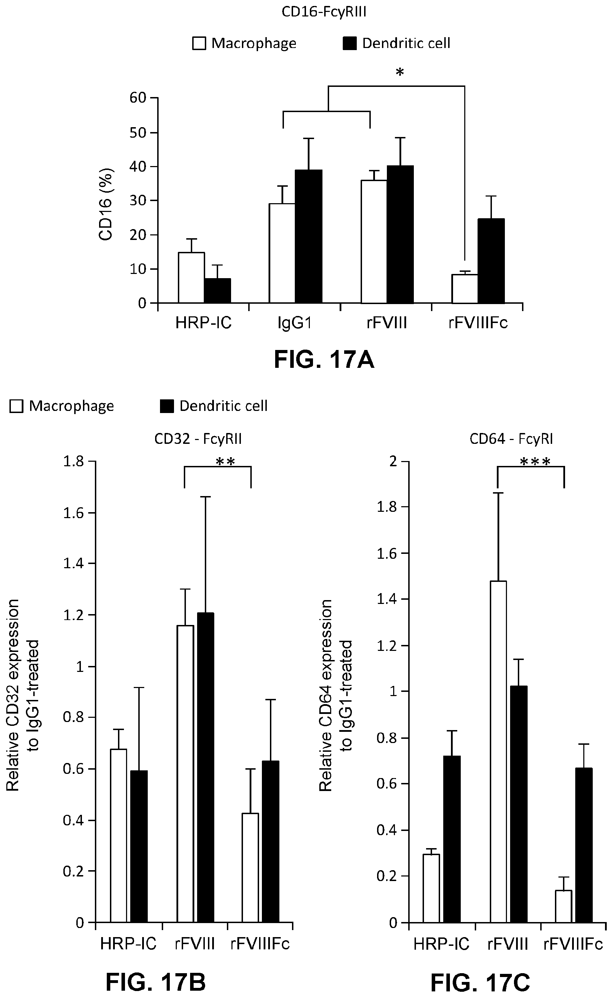

FIGs. 17A-17C are graphical representations of the relative macrophage and dendritic cell surface expression levels of the Fcγ receptors CD16 (FIG. 17A), CD32 (FIG. 17B), and CD64 (FIG. 17C) following treatment with horseradish peroxidase immune complexes (HRP-IC; positive control), IgG1, recombinant FVIII (rFVIII), or a rFVIII Fc fusion protein (rFVIIIFc). Asterisks (*) indicate degree of significance (n=3; * = P≤0.05, ** = P≤0.01, *** = P≤0.005, significance for HRP-IC as compared with the other treatments is not shown).

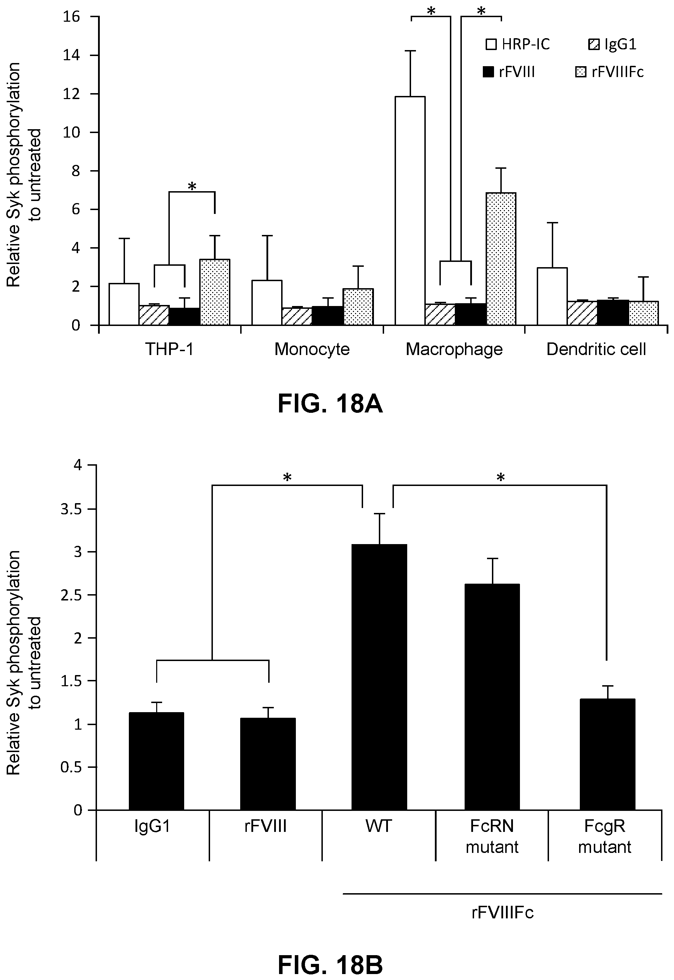

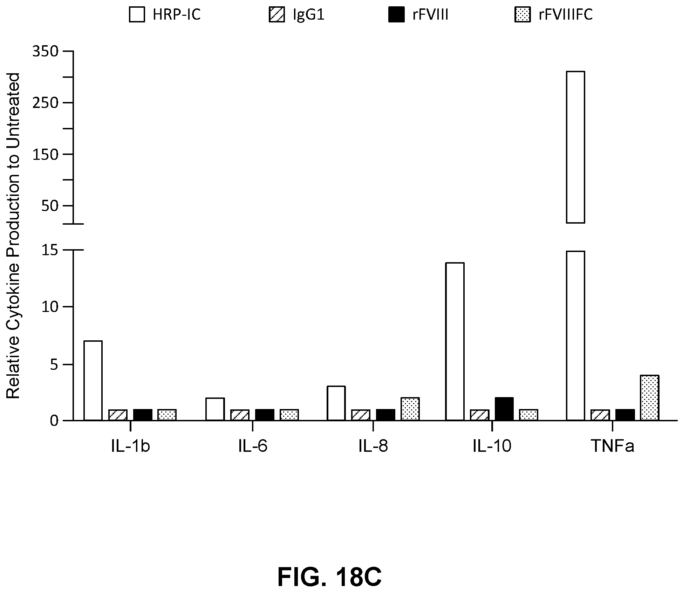

FIGs. 18A-18C are graphical representations illustrating relative signaling following treatment with rFVIII or rFVIIIFc. FIG. 18A shows signaling, as measured by Syk phosphorylation, in THP-1 monocytic cell line ("THP-1"), monocytes, peripheral blood monocyte-derived macrophages ("macrophage"), and peripheral blood monocyte-derived dendritic cells treated with HRP-IC, IgG1, rFVIII or rFVIIIFc for 15 minutes. FIG. 18B shows relative Syk phosphorylation in macrophages treated with rFVIIIFc ("WT"), mutant rFVIIIFc that is unable to bind to neonatal Fc receptor ("FcRn mutant"), or with mutant rFVIIIFc that is unable to bind to FcγR ("FcgR mutant"). FIG. 18C shows the relative production of the proinflammatory cytokines interleukin 1b (IL-1b), IL-6, IL-8, IL-10, and tumor necrosis factor alpha (TNFa) in macrophages twenty-four hours following treatment with HRP-IC, IgG1, rFVIII or rFVIIIFc.

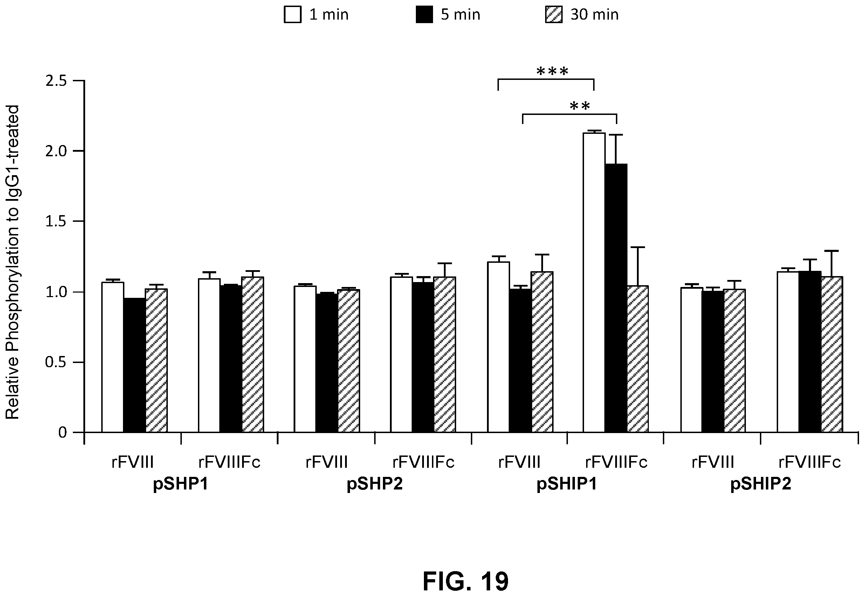

FIG. 19 shows the relative phosphorylation status of Src homology region 2 domaincontaining phosphatase-1 (SHP1), pSHP2, phosphatidylinositol-3,4,5-trisphosphate 5-phosphatase 1 (SHIP1), and pSHIP2 one minute, five minutes, and thirty minutes after treatment with rFVIII or rFVIIIFc. Asterisks (*) indicate degree of significance (n=3; **P≤0.01, ***P≤0.005).

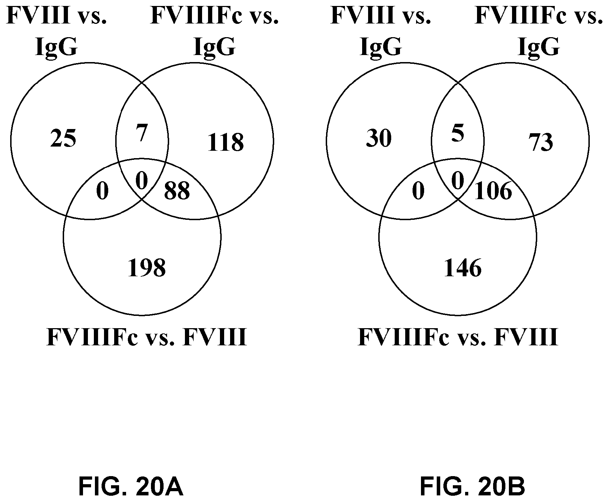

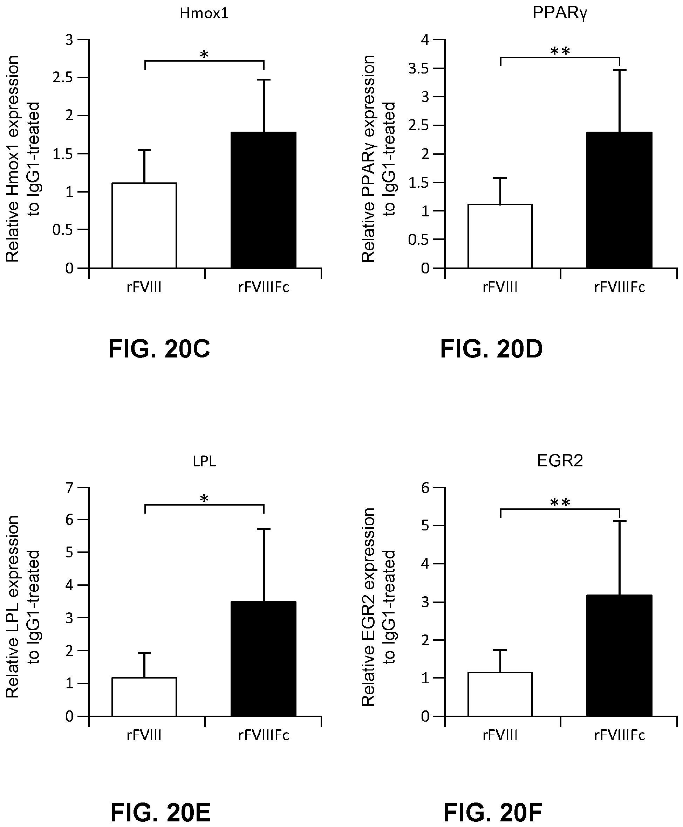

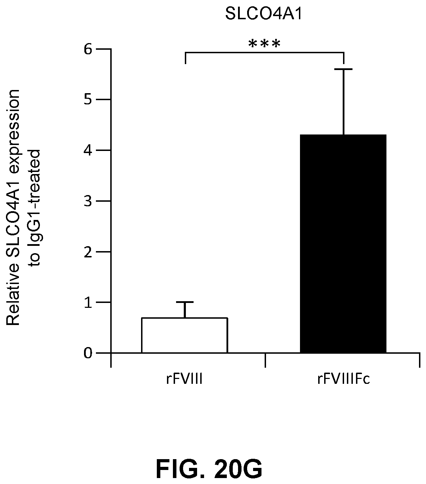

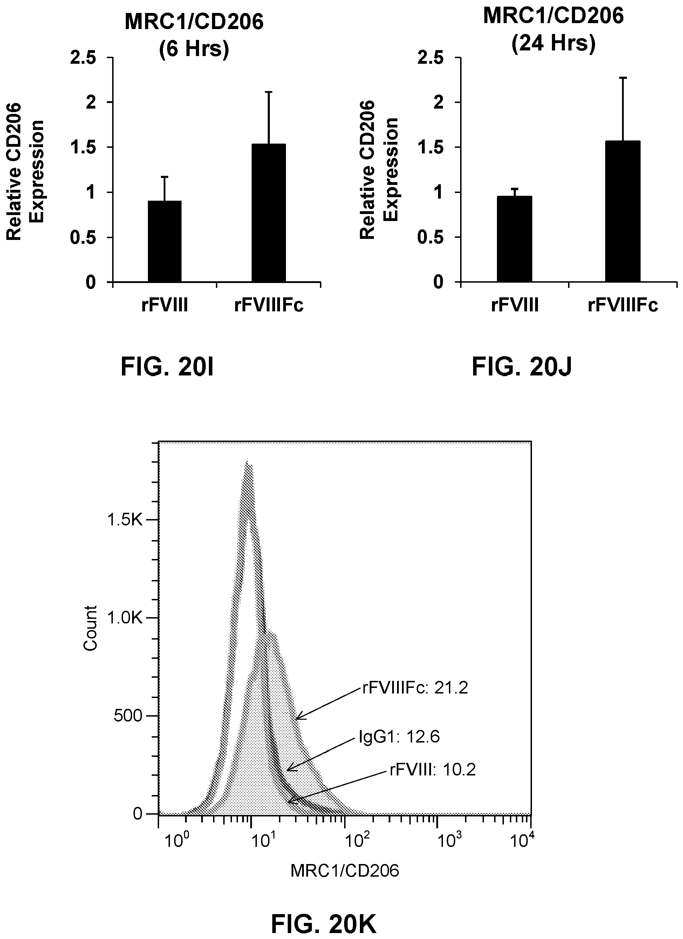

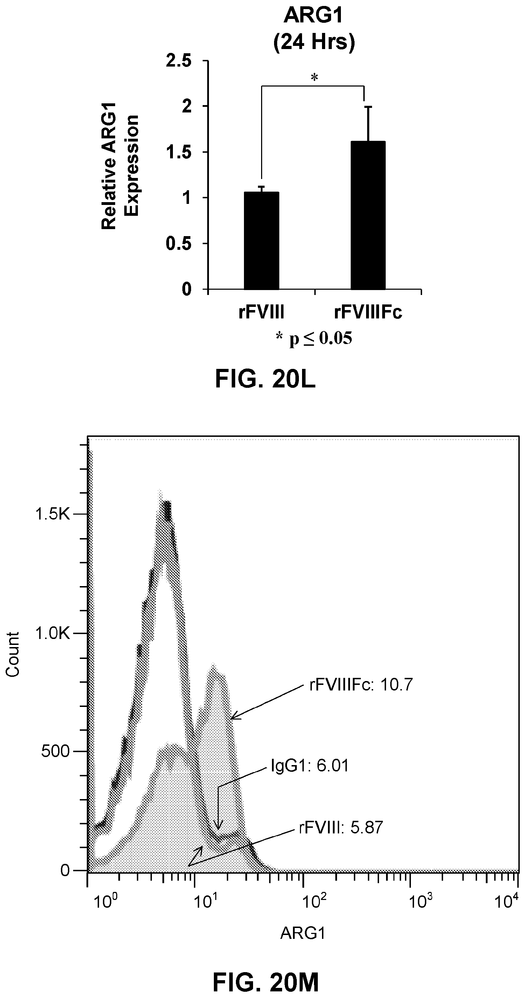

FIGs. 20A-20M are graphical representations of gene expression patterns of tolerogenic macrophages following treatment with rFVIII or rFVIIIFc. FIGs. 20A-20B are Venn diagrams, illustrating the distribution of genes that were significantly downregulated (FIG. 20A) and the distribution of genes that were significantly upregulated (FIG. 20B) in monocyte-derived macrophages treated with IgG1, rFVIII, or rFVIIIFc for six hours (n=3). FIGs. 20C-20G are graphs showing the relative expressions of various NRF2 and lipid metabolism pathway genes, such as heme oxygenase 1 (Hmox1; FIG. 20C), peroxisome proliferator-activated receptor gamma (PPARγ; FIG. 20D), lipoprotein lipase (LPL; FIG. 20E), early growth response 2 (EGR2; FIG. 20F), and solute carrier organic anion transporter family member 4A1 (SLCO4A1; FIG. 20G); CD206 at 6 hours (FIG. 20I) and 12 hours (FIG. 20J) post treatment; and arginase 1 (ARG1; FIG. 20L) as measured by quantitative PCR, following treatment with rFVIII or rFVIIIFc. Asterisks (*) indicate degree of significance (n=8; *P≤0.05, **P≤0.01, ***P≤0.005; FIGs. 20C-20G). FIGs. 20K and 20M are graphs showing the number of cells collected by flow cytometry expressing CD206. In addition, rFVIIIFc-educated macrophages were found to exhibit a characteristic M2-like phenotype (FIGs. 20I-20M). In particular, macrophages treated with rFVIIIFc had higher relative CD206 (also known as mannose receptor C-type 1; MRC1) expression than cells treated with rFVIII after 6 hours (FIG. 20I) and after 24 hours (FIG. 20J), and macrophages treated with rFVIIIFc had higher relative ARG1 expression than cells treated with rFVIII after 24 hours (FIG. 20M).

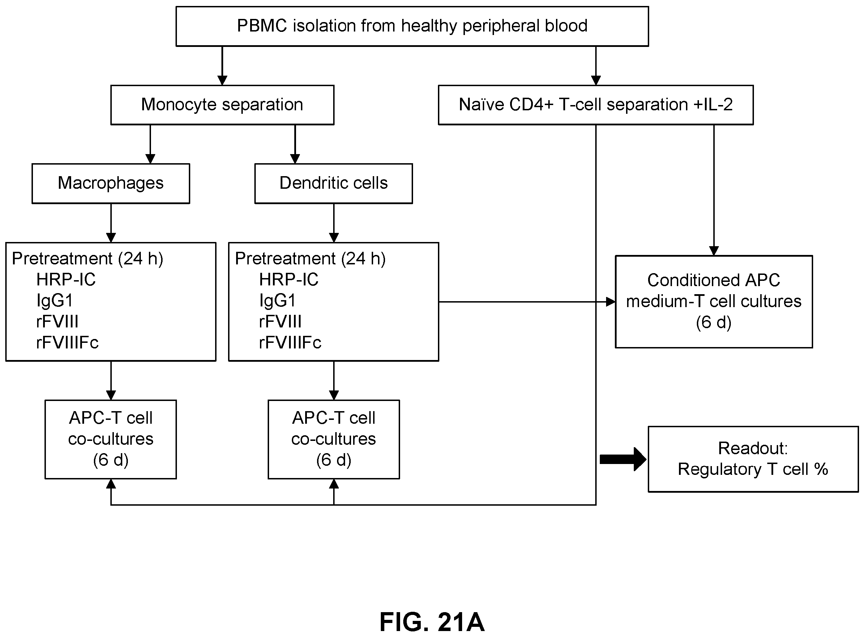

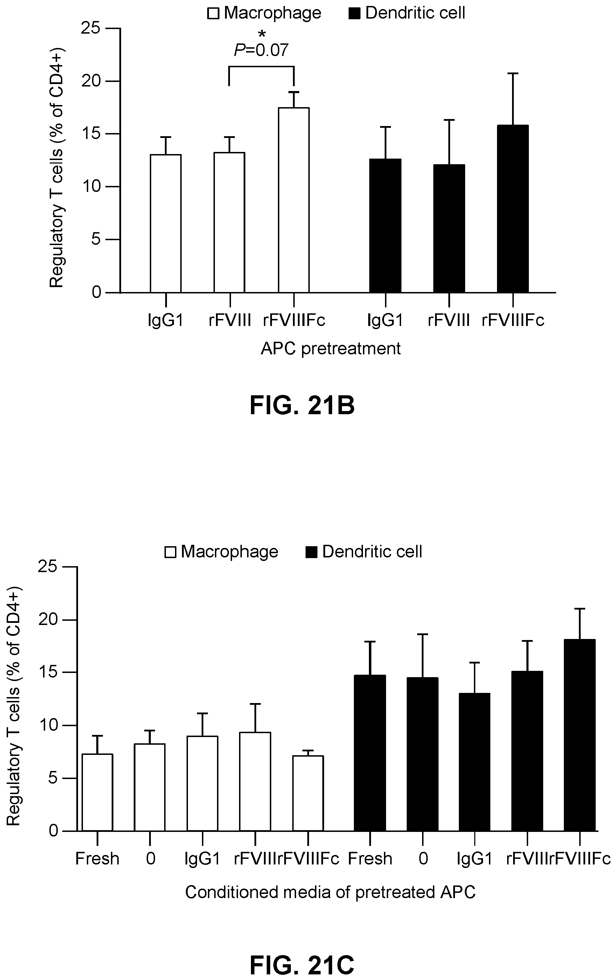

FIG. 21A is a flow chart diagraming the methods used to determine the effects of rFVIIIFc treatment on T-cell differentiation. FIG. 21B is a graphical representation of the percent of regulatory T cells six days after macrophages or dendritic cells were treated with IgG1 (control), rFVIII, or rFVIIIFc for 24 hours, and then placed into co-culture with naive CD4 positive T cells. FIG. 21C is a graphical representation of the percent of regulatory T cells following culture of naïve CD4 positive T cells in the conditioned media of macrophages or dendritic cells pretreated with IgG1, rFVIII, or rFVIIIFc.

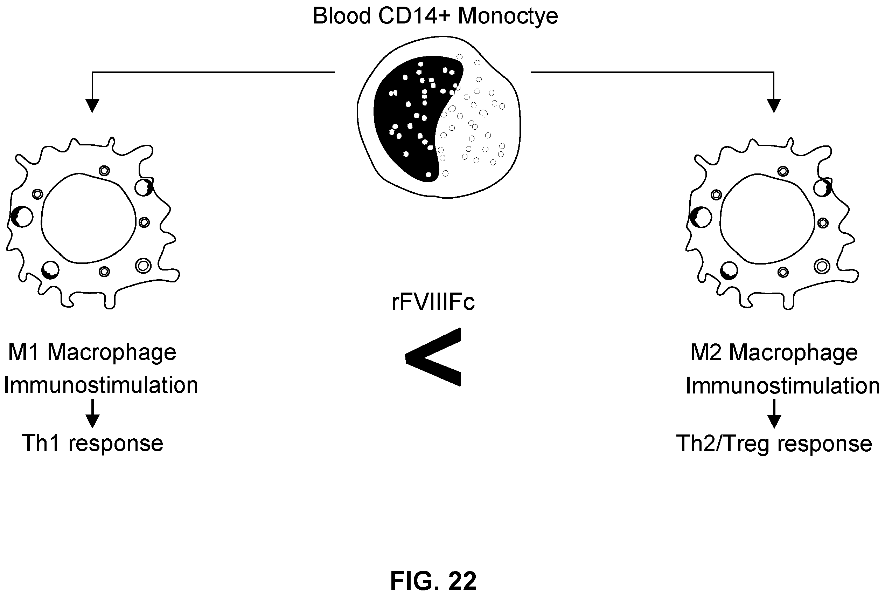

FIG. 22 is an illustration of the proposed mechanism of rFVIIIFc regulatory T-cell differentiation.

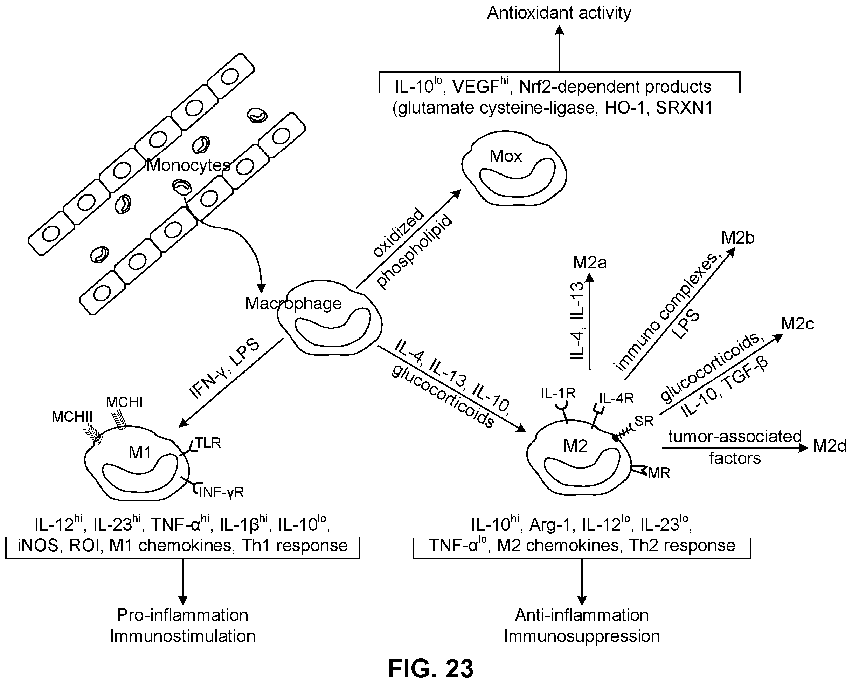

FIG. 23 is an illustration of the proposed effects of rFIXFc on macrophages.

### DETAILED DESCRIPTION

The present disclosure provides a chimeric protein comprising a factor VIII (FVIII) and a first Fc region derived from human IgG1, for use in reversing hemophilic arthropathy of a target joint in a human subject having hemophilia A, wherein the hemophilic arthropathy comprises synovitis, wherein the chimeric protein is administered at a dose from 20 IU/kg to 300 IU/kg, wherein the hemophilic arthropathy is reversed in the subject without surgical intervention, and wherein the administration improves a joint health score in the human subject. The chimeric protein disclosed herein can also be used to treat synovitis, a microbleed, inflammation of one or more joints, vascular remodeling, or any combination thereof in a joint of a human having hemophilia. The chimeric protein for use may also improve the surrounding soft tissue of a joint of a human having hemophilia.

### I. DEFINITIONS

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. Thus, "about 10-20" means "about 10 to about 20." In general, the term "about" can modify a numerical value above and below the stated value by a variance of, e.g., 10 percent, up or down (higher or lower).

"Administering," as used herein, means to give a pharmaceutically acceptable composition, *e.g*., a chimeric protein, disclosed herein to a subject via a pharmaceutically acceptable route. Routes of administration can be intravenous, *e.g*., intravenous injection and intravenous infusion. Additional routes of administration include, *e.g*., subcutaneous, intramuscular, oral, nasal, and pulmonary administration. Chimeric protein and hybrid proteins can be administered as part of a pharmaceutical composition comprising at least one excipient. In some embodiments, the composition, *e.g*., the chimeric protein is administered to a human through a gene therapy, *e.g*., wherein one or more polynucleotides encoding the clotting factor and/or the Fc region are administered to the human, and the clotting factor and/or the Fc region are expressed in the human.

"Treat," "treatment," or "treating," as used herein refers to, *e.g.,* the reduction in severity of a disease or condition; the reduction in the duration of a disease course; the amelioration or elimination of one or more symptoms associated with a disease or condition; the provision of beneficial effects to a subject with a disease or condition, without necessarily curing the disease or condition, but does not include prophylaxis or prevention of hemophilic arthropathy or its symptoms thereof. In one embodiment, the term "treating" or "treatment" means improving a Hemophilia Joint Health Score (HJHS) or a modified HJHS (mHJHS) for a subject. In some embodiments, the overall HJHS or mHJHS is improved. In some embodiments, the individual score for one or more target joints is improved. In some embodiments, the term "treating" or "treatment" means improving a quality of life (QoL) score for a subject. In certain embodiments, a QoL score analyzes the subject's disposition related to sports and leisure, physical health, dealing with hemophilia, family planning, feeling (toward hemophilia) future, partnership and sexuality, treatment, view (of yourself), work and school, or any combination thereof. In other embodiments, the term "treating" or "treatment" means reducing the effects and/or severity of one or more microbleed. In another embodiment, the term "treating" or "treatment" means reducing swelling and/or inflammation and/or pain in one or more target joint. In another embodiment, the term "treating" or "treatment" means reducing vascular remodeling in one or more target joint.

"Prevent" or "preventing," as used herein, refers to decreasing or reducing the occurrence or severity of a particular outcome. In some embodiments, preventing an outcome is achieved through prophylactic treatment.

The term "comparable" as used herein means a compared rate or level resulted from using, *e.g.,* the chimeric polypeptide is equal to, substantially equal to, or similar to the reference rate or level. The term "similar" as used herein means a compared rate or level has a difference of no more than 10% or no more than 15% from the reference rate or level (*e.g.,* FXa generation rate by a chimeric polypeptide consisting essentially of or consisting of two Fc portions and processed FVIII, wherein said processed FVIII is fused to one Fc of the two Fc portions). The term "substantially equal" means a compared rate or level has a difference of no more than 0.01%, 0.5% or 1% from the reference rate or level.

Hemostatic disorder, as used herein, means a genetically inherited or acquired condition characterized by a tendency to hemorrhage, either spontaneously or as a result of trauma, due to an impaired ability or inability to form a fibrin clot. Examples of such disorders include the hemophilias. The three main forms are hemophilia A (Factor VIII deficiency), hemophilia B (Factor IX deficiency or "Christmas disease") and hemophilia C (Factor XI deficiency, mild bleeding tendency). Other hemostatic disorders include, *e.g.,* Von Willebrand disease, Factor XI deficiency (PTA deficiency), Factor XII deficiency, deficiencies or structural abnormalities in fibrinogen, prothrombin, Factor V, Factor VII, Factor X or Factor XIII, Bernard-Soulier syndrome, which is a defect or deficiency in GPIb. GPIb, the receptor for VWF, can be defective and lead to lack of primary clot formation (primary hemostasis) and increased bleeding tendency, and thrombasthenia of Glanzman and Naegeli (Glanzmann thrombasthenia). In liver failure (acute and chronic forms), there is insufficient production of coagulation factors by the liver; this may increase bleeding risk.

"Area under the plasma concentration versus time curve (AUC)," as used herein, is the same as the term of art in pharmacology, and is based upon the rate and extent of absorption of FVIII following administration. AUC is determined over a specified time period, such as 12, 18, 24, 36, 48, or 72 hours, or for infinity using extrapolation based on the slope of the curve. Unless otherwise specified herein, AUC is determined for infinity. The determination of AUC can be carried out in a single subject, or in a population of subjects for which the average is calculated.

The term "procoagulant activity" is meant the ability of the coagulation factor, *e.g.,* a FVIII or a FIX protein, of the invention to participate in the clotting cascade in blood, substituting for the native coagulation factor, *e.g*., native FVIII or FIX. For example, a recombinant FIX protein of the invention has procoagulant activity when it can convert Factor X (FX) to activated Factor X (FXa) in the presence of Factor VIII (FVIII), as tested, *e.g.,* in a chromogenic assay. The FIX activity can be an ability to generate a tenase complex. In other cases, the FIX activity is an ability to generate thrombin (or a clot).

References made to amino acid numbering of immunoglobulins or immunoglobulin fragments, or regions, are all based on Kabat et al. 1991, Sequences of Proteins of Immunological Interest, U. S. Department of Public Health, Bethesda; MD. (The FcRn receptor has been isolated from several mammalian species including humans. The sequences of the human FcRn, rat FcRn, and mouse FcRn are known (Story et al., J. Exp. Med. 180: 2377 (1994).) An Fc can comprise the CH2 and CH3 domains of an immunoglobulin with or without the hinge region of the immunoglobulin. Exemplary Fc variants are provided in WO 2004/101740 and WO 2006/074199.

"Hybrid" polypeptides and proteins, as used herein, means a combination of a chimeric polypeptide with a second polypeptide. The chimeric polypeptide and the second polypeptide in a hybrid can be associated with each other via protein-protein interactions, such as charge-charge or hydrophobic interactions. The chimeric polypeptide and the second polypeptide in a hybrid can be associated with each other via disulfide or other covalent bond(s). Hybrids are described in WO 2004/101740 and WO 2006/074199. See also U.S. Patent Nos. 7,404,956 and 7,348,004. The second polypeptide can be a second copy of the same chimeric polypeptide or it can be a non-identical chimeric polypeptide.

As used herein, an "amino acid corresponding to," "site corresponding to," or "equivalent amino acid" in a protein sequence is identified by alignment to maximize the identity or similarity between a first protein sequence, *e.g.,* a FVIII or a FIX sequence, and a second protein sequence, *e.g.,* a second FVIII or a second FIX sequence. The number used to identify an equivalent amino acid in a second protein sequence is based on the number used to identify the corresponding amino acid in the first protein sequence.

As used herein, the term "insertion site" refers to an amino acid residue number in a polypeptide (typically a mature polypeptide; *e.g.,* a mature FVIII or a mature FIX polypeptide), or fragment, variant, or derivative thereof, which is immediately upstream of the position at which a heterologous moiety can be inserted. An "insertion site" is specified as a number, the number being the number of the amino acid specified protein sequence to which the insertion site corresponds, which is immediately N-terminal to the position of the insertion. For example, the phrase "the EGF2 domain comprises a heterologous moiety at an insertion site which corresponds to amino acid 105 of" a given sequence indicates that the heterologous moiety is located between two amino acids corresponding to amino acid 105 and amino acid 106 of the sequence. However, one of skill in the art would readily be able to identify a corresponding position in any variant of the indicated protein, and the present disclosure is not limited to insertions made solely in the variants specifically disclosed herein. Rather, the insertions disclosed herein can be made in any related variants or fragments thereof having activity at a position corresponding to a position of the variants disclosed herein.

The phrase "immediately downstream of an amino acid" as used herein refers to position right next to the terminal carboxyl group of the amino acid. Similarly, the phrase "immediately upstream of an amino acid" refers to the position right next to the terminal amine group of the amino acid. Therefore, the phrase "between two amino acids of an insertion site" as used herein refers to a position in which a heterologous moiety (*e.g.,* a half-life extending moiety) is inserted between two adjacent amino acids.

The terms "inserted," "is inserted," "inserted into" or grammatically related terms, as used herein refers to the position of a heterologous moiety (*e.g.,* a half-life extending moiety) in a fusion polypeptide relative to the analogous position in specified protein (*e.g.,* a FVIII or a FIX protein). Those of skill in the field will understand how to identify corresponding insertion positions with respect to other polypeptide sequences, *e.g*., other FVIII and FIX variants. As used herein the terms refer to the characteristics of the recombinant polypeptide disclosed herein, and do not indicate, imply or infer any methods or process by which the fusion polypeptide was made. For example, in reference to a fusion polypeptide provided herein, the phrase "a heterologous moiety is inserted into the EGF2 domain immediately downstream of residue 105 of the FIX polypeptide" means that the fusion polypeptide comprises a heterologous moiety immediately downstream of an amino acid which corresponds to amino acid 105 in a particular FIX variant, *e.g*., bounded by amino acids corresponding to amino acids 105 and 106 of the FIX variant.

A "fusion" or "chimeric" protein comprises a first amino acid sequence linked to a second amino acid sequence with which it is not naturally linked in nature. The amino acid sequences which normally exist in separate proteins can be brought together in the fusion polypeptide, or the amino acid sequences which normally exist in the same protein can be placed in a new arrangement in the fusion polypeptide, *e.g*., fusion of a FVIII domain or a FIX domain of the invention with an Ig Fc domain. A fusion protein is created, for example, by chemical synthesis, or by creating and translating a polynucleotide in which the peptide regions are encoded in the desired relationship. A fusion protein can further comprise a second amino acid sequence associated with the first amino acid sequence by a covalent, non-peptide bond or a non-covalent bond.

The terms "heterologous" and "heterologous moiety" mean that a polynucleotide, polypeptide, or other moiety is derived from a distinct entity from that of the entity to which it is being compared. For instance, a heterologous polypeptide can be synthetic, or derived from a different species, different cell type of an individual, or the same or different type of cell of distinct individuals. In one aspect, a heterologous moiety is a polypeptide fused to another polypeptide to produce a fusion polypeptide or protein. In another aspect, a heterologous moiety is a non-polypeptide such as PEG conjugated to a polypeptide or protein.

The terms "linked" and "fused" as used herein refers to a first amino acid sequence or nucleotide sequence covalently or non-covalently joined to a second amino acid sequence or nucleotide sequence, respectively. The first amino acid or nucleotide sequence can be directly joined or juxtaposed to the second amino acid or nucleotide sequence or alternatively an intervening sequence can covalently join the first sequence to the second sequence. The term "linked" means not only a fusion of a first amino acid sequence to a second amino acid sequence at the C-terminus or the N-terminus, but also includes insertion of the whole first amino acid sequence (or the second amino acid sequence) into any two amino acids in the second amino acid sequence (or the first amino acid sequence, respectively). In one embodiment, the first amino acid sequence is linked to a second amino acid sequence by a peptide bond or a linker. The first nucleotide sequence can be linked to a second nucleotide sequence by a phosphodiester bond or a linker. The linker can be a peptide or a polypeptide (for polypeptide chains) or a nucleotide or a nucleotide chain (for nucleotide chains) or any chemical moiety (for both polypeptide and polynucleotide chains). The term "linked" is also indicated by a hyphen (-).

As used herein the term "associated with" refers to a covalent or non-covalent bond formed between a first amino acid chain and a second amino acid chain. In one embodiment, the term "associated with" means a covalent, non-peptide bond or a non-covalent bond. This association can be indicated by a colon, *i.e.,* (:). In another embodiment, it means a covalent bond except a peptide bond. For example, the amino acid cysteine comprises a thiol group that can form a disulfide bond or bridge with a thiol group on a second cysteine residue. In most naturally occurring IgG molecules, the CH1 and CL regions are associated by a disulfide bond and the two heavy chains are associated by two disulfide bonds at positions corresponding to 239 and 242 using the Kabat numbering system (position 226 or 229, EU numbering system). Examples of covalent bonds include, but are not limited to, a peptide bond, a metal bond, a hydrogen bond, a disulfide bond, a sigma bond, a pi bond, a delta bond, a glycosidic bond, an agnostic bond, a bent bond, a dipolar bond, a Pi backbond, a double bond, a triple bond, a quadruple bond, a quintuple bond, a sextuple bond, conjugation, hyperconjugation, aromaticity, hapticity, or antibonding. Non-limiting examples of non-covalent bond include an ionic bond (*e.g.,* cation-pi bond or salt bond), a metal bond, an hydrogen bond (*e.g.,* dihydrogen bond, dihydrogen complex, low-barrier hydrogen bond, or symmetric hydrogen bond), van der Walls force, London dispersion force, a mechanical bond, a halogen bond, aurophilicity, intercalation, stacking, entropic force, or chemical polarity.

As used herein, the term "cleavage site" or "enzymatic cleavage site" refers to a site recognized by an enzyme. Certain enzymatic cleavage sites comprise an intracellular processing site. In one embodiment, a polypeptide has an enzymatic cleavage site cleaved by an enzyme that is activated during the clotting cascade, such that cleavage of such sites occurs at the site of clot formation. Exemplary such sites include, *e.g.,* those recognized by thrombin, Factor XIa or Factor Xa. Other enzymatic cleavage sites are known in the art.

As used herein, the term "processing site" or "intracellular processing site" refers to a type of enzymatic cleavage site in a polypeptide which is a target for enzymes that function after translation of the polypeptide. In one embodiment, such enzymes function during transport from the Golgi lumen to the trans-Golgi compartment. Intracellular processing enzymes cleave polypeptides prior to secretion of the protein from the cell. Examples of such processing sites include, *e.g.,* those targeted by the PACE/furin (where PACE is an acronym for Paired basic Amino acid Cleaving Enzyme) family of endopeptidases. These enzymes are localized to the Golgi membrane and cleave proteins on the carboxyterminal side of the sequence motif Arg-[any residue]-(Lys or Arg)-Arg. As used herein the "furin" family of enzymes includes, *e.g*., PCSK1 (also known as PC1/Pc3), PCSK2 (also known as PC2), PCSK3 (also known as furin or PACE), PCSK4 (also known as PC4), PCSK5 (also known as PC5 or PC6), PCSK6 (also known as PACE4), or PCSK7 (also known as PC7/LPC, PC8, or SPC7). Other processing sites are known in the art.

In constructs that include more than one processing or cleavage site, it will be understood that such sites may be the same or different.

A "processable linker" as used herein refers to a linker comprising at least one intracellular processing site, which is described elsewhere herein.

"Baseline," as used herein, is the lowest measured plasma level of a given analyte, *e.g.,* a clotting factor (*e.g.,* FVIII or FIX), in a subject prior to administering a dose. The plasma levels can be measured at two time points prior to dosing: at a screening visit and immediately prior to dosing. Alternatively, (a) the baseline in subjects whose pretreatment clotting factor activity is <1%, who have no detectable clotting factor antigen, and have nonsense genotypes can be defined as 0%, (b) the baseline for subjects with pretreatment clotting factor activity <1% and who have detectable clotting factor antigen can be set at 0.5%, (c) the baseline for subjects whose pretreatment clotting factor activity is between 1 - 2% is Cₘᵢₙ (the lowest activity throughout the PK study), and (d) the baseline for subjects whose pretreatment clotting factor activity is ≥2% can be set at 2%.

"Equivalent dose," as used herein, means the same dose of clotting factor activity, *e.g.,* FVIII activity or FIX activity, as expressed in International Units, which is independent of molecular weight of the polypeptide in question. For example, one International Unit (IU) of FVIII activity corresponds approximately to the quantity of FVIII in one milliliter of normal human plasma. Several assays are available for measuring clotting factor activity, including the European Pharmacopoeia chromogenic substrate assay and a one stage clotting assay.

"Dosing interval," as used herein, means the amount of time that elapses between multiple doses being administered to a subject. The comparison of dosing interval can be carried out in a single subject or in a population of subjects and then the average obtained in the population can be calculated.

"Subject," as used herein means a human individual. Subject can be a patient who is currently suffering from a bleeding disorder or is expected to be in need of such a treatment. In some embodiments, the subject has never been previously treated with the clotting factor (*i.e.,* the subject is a previously untreated subject or previously untreated patient). In some embodiments, the subject is a fetus and the methods comprise administering the composition, *e.g.*, the chimeric polypeptide, to the mother of the fetus and the administration to the subject occurs from the mother across the placenta. In some embodiments, the subject is a child or an adult. In some embodiments, the subject is a child less than one-year-old, less than two-year-old, less than three-year-old, less than four-year-old, less than five-year-old, less than six-year-old, less than seven-year-old, less than eight-year-old, less than nine-year-old, less than ten-year-old, less than eleven-year-old, or less than twelve-year-old. In some embodiments, the child is less than one-year old. In certain embodiments, the subject is less than 6 years old. In other embodiments, the subject is between 6 and less than 12 years old. In other embodiments, the subject is 12 years old or older. In some embodiments, the child or adult subject develops a bleeding disorder, wherein the onset of the symptoms of the bleeding disorder is after the one-year-old age. In some embodiments, the administration of the composition, *e.g*., the chimeric polypeptide, to the subject is sufficient to prevent, inhibit, or reduce development of an immune response selected from a humoral immune response, a cell-mediated immune response, or both a humoral immune response and a cell-mediated immune response against the clotting factor.

A "therapeutic dose," "dose," "effective dose," or "dosing amount" as used (interchangeably) herein, means a dose that achieves a therapeutic goal, as described herein. In some embodiments, a "therapeutic dose" means a dose that improves an HJHS, mHJHS, or a QoL score, as compared to the HJHS, mHJHS, or a QoL score prior to the treatment. In some embodiments, a "therapeutic dose" means a dose that reduces swelling, inflammation, and/or pain in one or more joints in a subject as compared to the level of swelling, inflammation, and/or pain in the joint prior to the treatment. In some embodiments, a "therapeutic dose" means a dose that reduces the effects and/or severity of one or more microbleed, as compared to the effects and/or severity of the microbleed prior to the treatment. In another embodiment, a "therapeutic dose" means a dose that reduces vascular remodeling in one or more target joint, as compared to the vascular remodeling prior to the treatment.

Also disclosed are fragments or variants of polypeptides, and any combination thereof. The term "fragment" or "variant" when referring to polypeptides used in the methods of the present disclosure include any polypeptides which retain at least some of the properties (*e.g.,* Fc variant or coagulation activity for a FVIII or a FIX variant) of the reference polypeptide. Fragments of polypeptides include proteolytic fragments, as well as deletion fragments, in addition to specific antibody fragments discussed elsewhere herein, but do not include the naturally occurring full-length polypeptide (or mature polypeptide). Variants of polypeptide binding domains or binding molecules used in the methods of the present disclosure include fragments as described above, and also polypeptides with altered amino acid sequences due to amino acid substitutions, deletions, or insertions. Variants can be naturally or non-naturally occurring. Non-naturally occurring variants can be produced using art-known mutagenesis techniques. Variant polypeptides can comprise conservative or non-conservative amino acid substitutions, deletions or additions. One particular FIX variant disclosed herein is the R338L FIX (Padua) variant. *See, e.g.,* Simioni, P., et al., "X-Linked Thrombophilia with a Mutant Factor IX (Factor IX Padua)," NEJM 361:1671-75 (October 2009).

A "conservative amino acid substitution" is one in which the amino acid residue is replaced with an amino acid residue having a similar side chain. Families of amino acid residues having similar side chains have been defined in the art, including basic side chains (*e.g.,* lysine, arginine, histidine), acidic side chains (*e.g.,* aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Thus, if an amino acid in a polypeptide is replaced with another amino acid from the same side chain family, the substitution is considered to be conservative. In another embodiment, a string of amino acids can be conservatively replaced with a structurally similar string that differs in order and/or composition of side chain family members.

The term "percent sequence identity" between two polynucleotide or polypeptide sequences refers to the number of identical matched positions shared by the sequences over a comparison window, taking into account additions or deletions *(i.e.,* gaps) that must be introduced for optimal alignment of the two sequences. A matched position is any position where an identical nucleotide or amino acid is presented in both the target and reference sequence. Gaps presented in the target sequence are not counted since gaps are not nucleotides or amino acids. Likewise, gaps presented in the reference sequence are not counted since target sequence nucleotides or amino acids are counted, not nucleotides or amino acids from the reference sequence.

The percentage of sequence identity is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. The comparison of sequences and determination of percent sequence identity between two sequences may be accomplished using readily available software both for online use and for download. Suitable software programs are available from various sources, and for alignment of both protein and nucleotide sequences. One suitable program to determine percent sequence identity is bl2seq, part of the BLAST suite of programs available from the U.S. government's National Center for Biotechnology Information BLAST web site (blast.ncbi.nlm.nih.gov). Bl2seq performs a comparison between two sequences using either the BLASTN or BLASTP algorithm. BLASTN is used to compare nucleic acid sequences, while BLASTP is used to compare amino acid sequences. Other suitable programs are, *e.g.,* Needle, Stretcher, Water, or Matcher, part of the EMBOSS suite of bioinformatics programs and also available from the European Bioinformatics Institute (EBI) at www.ebi.ac.uk/Tools/psa.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

One skilled in the art will appreciate that the generation of a sequence alignment for the calculation of a percent sequence identity is not limited to binary sequence-sequence comparisons exclusively driven by primary sequence data. Sequence alignments can be derived from multiple sequence alignments. One suitable program to generate multiple sequence alignments is ClustalW2, available from www.clustal.org. Another suitable program is MUSCLE, available from www.drive5.com/muscle/. ClustalW2 and MUSCLE are alternatively available, *e.g*., from the EBI.

It will also be appreciated that sequence alignments can be generated by integrating sequence data with data from heterogeneous sources such as structural data (*e.g.,* crystallographic protein structures), functional data (*e.g.,* location of mutations), or phylogenetic data. A suitable program that integrates heterogeneous data to generate a multiple sequence alignment is T-Coffee, available at www.tcoffee.org, and alternatively available, *e.g.*, from the EBI. It will also be appreciated that the final alignment used to calculate percent sequence identity may be curated either automatically or manually.

The polynucleotide variants can contain alterations in the coding regions, non-coding regions, or both. Polynucleotide variants may contain alterations which produce silent substitutions, additions, or deletions, but do not alter the properties or activities of the encoded polypeptide. Nucleotide variants can be produced by silent substitutions due to the degeneracy of the genetic code. Variants can be produced in which 5-10, 1-5, or 1-2 amino acids are substituted, deleted, or added in any combination. Polynucleotide variants can be produced for a variety of reasons, *e.g.,* to optimize codon expression for a particular host (change codons in the human mRNA to others, *e.g.,* a bacterial host such as *E. coli*)*.*

Naturally occurring variants are called "allelic variants," and refer to one of several alternate forms of a gene occupying a given locus on a chromosome of an organism (Genes II, Lewin, B., ed., John Wiley & Sons, New York (1985)). These allelic variants can vary at either the polynucleotide and/or polypeptide level and are included in the present disclosure. Alternatively, non-naturally occurring variants can be produced by mutagenesis techniques or by direct synthesis.

Using known methods of protein engineering and recombinant DNA technology, variants can be generated to improve or alter the characteristics of the polypeptides. For instance, one or more amino acids can be deleted from the N-terminus or C-terminus of the secreted protein without substantial loss of biological function. Ron et al., J. Biol. Chem. 268: 2984-2988 (1993), reported variant KGF proteins having heparin binding activity even after deleting 3, 8, or 27 amino-terminal amino acid residues. Similarly, interferon gamma exhibited up to ten times higher activity after deleting 8-10 amino acid residues from the carboxy terminus of this protein. (Dobeli et al., J. Biotechnology 7:199-216 (1988).)
Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See Abstract.) In fact, only 23 unique amino acid sequences, out of more than 3,500 nucleotide sequences examined, produced a protein that significantly differed in activity from wild-type.

As stated above, polypeptide variants include, *e.g*., modified polypeptides. Modifications include, *e.g.*, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation (Mei et al., Blood 116:270-79 (2010)), proteolytic processing, phosphorylation, prenylation, racemization, selenoylation, sulfation, transfer-RNA mediated addition of amino acids to proteins such as arginylation, and ubiquitination. In some embodiments, FVIII is modified, *e.g.*, pegylated, at any convenient location. In some embodiments, FVIII is pegylated at a surface exposed amino acid of FVIII, *e.g.,* a surface exposed cysteine, which can be an engineered cysteine. Id. In some embodiments, modified FVIII, *e.g.*, pegylated FVIII, is a chimeric or fusion FVIII.

The term "downstream" refers to a nucleotide sequence that is located 3' to a reference nucleotide sequence. "Downstream" can also refer to a peptide sequence that is located C-terminal to a reference peptide sequence.

The term "upstream" refers to a nucleotide sequence that is located 5' to a reference nucleotide sequence. "Upstream" can also refer to a peptide sequence that is located N-terminal to a reference peptide sequence.

As used herein, the term "regulatory region" refers to nucleotide sequences located upstream (5' non-coding sequences), within, or downstream (3' non-coding sequences) of a coding region, and which influence the transcription, RNA processing, stability, or translation of the associated coding region. Regulatory regions may include promoters, translation leader sequences, introns, polyadenylation recognition sequences, RNA processing sites, effector binding sites and stem-loop structures. If a coding region is intended for expression in a eukaryotic cell, a polyadenylation signal and transcription termination sequence will usually be located 3' to the coding sequence.

A polynucleotide, which encodes a gene product, *e.g.,* a polypeptide, can include a promoter and/or other transcription or translation control elements operably associated with one or more coding regions. Other transcription control elements, besides a promoter, for example enhancers, operators, repressors, and transcription termination signals, can also be operably associated with a coding region to direct gene product expression.

A variety of transcription control regions are known to those skilled in the art. These include, without limitation, transcription control regions, which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as lymphokine-inducible promoters (e.g., promoters inducible by interferons or interleukins).

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

The term "expression" as used herein refers to a process by which a polynucleotide produces a gene product, for example, an RNA or a polypeptide.

A "vector" refers to any vehicle for the cloning of and/or transfer of a nucleic acid into a host cell. A vector may be a replicon to which another nucleic acid segment may be attached so as to bring about the replication of the attached segment. A "replicon" refers to any genetic element (*e.g.,* plasmid, phage, cosmid, chromosome, virus) that functions as an autonomous unit of replication *in vivo, i.e.,* capable of replication under its own control. The term "vector" includes both viral and nonviral vehicles for introducing the nucleic acid into a cell *in vitro, ex vivo* or *in vivo.* A large number of vectors are known and used in the art including, for example, plasmids, modified eukaryotic viruses, or modified bacterial viruses. Insertion of a polynucleotide into a suitable vector can be accomplished by ligating the appropriate polynucleotide fragments into a chosen vector that has complementary cohesive termini.

The term "plasmid" refers to an extra-chromosomal element often carrying a gene that is not part of the central metabolism of the cell, and usually in the form of circular double-stranded DNA molecules. Such elements may be autonomously replicating sequences, genome integrating sequences, phage or nucleotide sequences, linear, circular, or supercoiled, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell.

Eukaryotic viral vectors that can be used include, but are not limited to, adenovirus vectors, retrovirus vectors, adeno-associated virus vectors, and poxvirus, *e.g*., vaccinia virus vectors, baculovirus vectors, or herpesvirus vectors. Non-viral vectors include plasmids, liposomes, electrically charged lipids (cytofectins), DNA-protein complexes, and biopolymers.

A "cloning vector" refers to a "replicon," which is a unit length of a nucleic acid that replicates sequentially and which comprises an origin of replication, such as a plasmid, phage or cosmid, to which another nucleic acid segment may be attached so as to bring about the replication of the attached segment. Certain cloning vectors are capable of replication in one cell type, *e.g.,* bacteria and expression in another, *e.g.,* eukaryotic cells. Cloning vectors typically comprise one or more sequences that can be used for selection of cells comprising the vector and/or one or more multiple cloning sites for insertion of nucleic acid sequences of interest.

The term "expression vector" refers to a vehicle designed to enable the expression of an inserted nucleic acid sequence following insertion into a host cell. The inserted nucleic acid sequence is placed in operable association with regulatory regions as described above.

Vectors are introduced into host cells by methods well known in the art, *e.g.,* transfection, electroporation, microinjection, transduction, cell fusion, DEAE dextran, calcium phosphate precipitation, lipofection (lysosome fusion), use of a gene gun, or a DNA vector transporter.

An "isolated" polypeptide or a fragment, variant, or derivative thereof refers to a polypeptide that is not in its natural milieu. No particular level of purification is required. For example, an isolated polypeptide can simply be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

As used herein, the term "host cell" refers to a cell or a population of cells harboring or capable of harboring a recombinant nucleic acid. Host cells can be prokaryotic cells (*e.g., E. coli*), or alternatively, the host cells can be eukaryotic, for example, fungal cells (*e.g.,* yeast cells such as *Saccharomyces cerevisiae, Pichia pastoris,* or *Schizosaccharomyces pombe*), and various animal cells, such as insect cells (*e.g.,* Sf-9) or mammalian cells (*e.g.,* HEK293F, CHO, COS- 7, NIH-3T3).

"Volume of distribution at steady state (Vss)," as used herein, has the same meaning as the term used in pharmacology, which is the apparent space (volume) into which a drug distributes. Vss = the amount of drug in the body divided by the plasma concentration at steady state.

### II. THE CHIMERIC PROTEIN FOR USE OF THE INVENTION

The present disclosure is based on the discovery that a clotting factor fused to an Fc region can be used to reverse hemophilic arthropathy. Hemophilic arthropathy was previously known to be irreversible once it is developed, as soft tissue changes could only be visualized by MRI. The currently known options for an individual suffering from hemophilic arthropathy include a surgery or a sclerosing agent to remove hypertrophied synovium. A sclerosing agent, either radioactive or chemical material, can impede further cartilaginous and bony deterioration; however, it is not capable of reversing hemophilic arthropathy. Arthroplasty of the knee and hip have been successful in reducing pain and loss of motion when other efforts to control synovial hypertrophy fail. Hilgartner M., Current Opinion in Pediatrics: February 2002, V14, Issue 1, pp. 46-49.

The present disclosure therefore provides a chimeric protein comprising a factor VIII (FVIII) and a first Fc region derived from human IgG1, for use in reversing hemophilic arthropathy of a target joint in a human having hemophilia A, wherein the hemophilic arthropathy comprises synovitis, wherein the chimeric protein is administered at a dose from 20 IU/kg to 300 IU/kg, wherein the hemophilic arthropathy is reversed in the subject without surgical intervention, and wherein the administration improves a joint health score in the human subject. The treatment of hemophilic arthropathy can reverse, partially or completely, the hemophilic arthropathy (*e.g.,* one or more symptoms of hemophilic arthropathy).

In general, hemophilic arthropathy refers to joint disease that occurs in a human subject suffering from hemophilia as a long-term consequence or repeated bleeding into the subject's joints. Spontaneous bleeding into one or more joints is common in hemophilia patients. Joints with several consecutive bleeds within a 6 month period are often referred to as "target joints," and these joints often progress to hemophilic arthropathy.

Hemophilic arthropathy can present with various symptoms, including, but not limited to, synovial hyperplasia, chronic inflammation (including synovitis), fibrosis, haemosiderosis, subarticular cyst formation, pain, reduced range of motion, muscle atrophy, ankylosis, osteoporosis, cartilage degeneration with collapse of the joint space, and any combination thereof. Hemophilic arthropathy includes various stages: (i) Stage I including soft-tissue swelling, but no skeletal abnormalities; (ii) Stage II including overgrowth and osteoporosis of the epiphysis, joint integrity is maintained. There are no bone cysts and no narrowing of the articular cartilage space. The radiologic Stage II parallels the clinical stage of subacute hemophilic arthropathy; (iii) Stage III including minimal to moderate joint space narrowing with subchondral cysts. Widening of the intercondylar notch of the knee and the trochlear notch of the ulna. In the knee there may be squaring of the patella. Articular cartilage is still preserved and hemophilic arthropathy is still reversible; (iv) Stage IV including destruction of articular cartilage with severe narrowing of the joint space. The other osseous changes found in stage III are more pronounced; and (v) Stage V including total loss of joint space with fibrous ankylosis of the joint. There is marked incongruity of the articular structures with severe irregular hypertrophy of the epiphysis.

The present disclosure provides that treatment with the chimeric protein, comprising the clotting factor and the Fc region is capable of reducing and/or ameliorating the symptoms of reversible hemophilic arthropathy. In some embodiments, the chimeric protein can treat one more stages of hemophilic arthropathy, e.g., Stages I, II, and/or III. As used herein, a "reversible" hemophilic arthropathy is a manifestation of hemophilic arthropathy that can partially or completely revert to its original, healthy state following treatment. In contrast, an "irreversible" hemophilic arthropathy is a manifestation of hemophilic arthropathy that is permanent and that will not improve following treatment. Therefore, the present methods can improve, reduce, or ameliorate (or reverse either partially or completely) one or more symptoms of hemophilic arthropathy, e.g., synovial hyperplasia, chronic inflammation (including synovitis), haemosiderosis, subarticular cyst formation, pain, reduced range of motion, swelling, vascular remodeling, or any combination thereof.

The hemophilic arthropathy (reversible) treated using the present disclosure can affect any joint in the body. In some embodiments, the joint is a load bearing joint, *e.g.,* a joint selected from the group consisting of one or both knees, one or both ankles, one or both hips, one or more joints of the foot, and any combination thereof. In another embodiment, the joint is a non-load bearing joint, *e.g.,* a joint selected from the group consisting of one or both elbows, one or both shoulders, one or both wrists, one or more joints of the hand, or any combination thereof. In another embodiment, the joint is a knee.

Synovitis refers to inflammation of the synovial membrane, which surrounds the joint. In some embodiments, the synovitis is of any joint in the body. In some embodiments, the synovitis presents as swelling of the joint, and the methods of the present disclosure reduce the swelling of the joint. In some embodiments, the synovitis presents as pain in the joint, and the methods of the present disclosure reduce the pain in the joint. In other embodiments, the synovitis presents as decreased range of motion of the joint, and the methods of the present invention increase the range of motion of the joint.

In other embodiments, the reversible hemophilic arthropathy comprises a microbleed in a joint. In some embodiments, the reversible hemophilic arthropathy is the result of a microbleed. A microbleed refers to a very small bleed in one or more joints, which can lead to hemophilic arthropathy following repeated occurrences. In some embodiments, the reversible hemophilic arthropathy comprises an acute joint bleed. In some embodiments, the reversible hemophilic arthropathy is the result of an acute joint bleed. An acute joint bleed refers to a more substantial bleeding episode in one or more joints.

In certain embodiments, the reversible hemophilic arthropathy comprises inflammation of one or more joints. In some embodiments, the inflammation is in any joint in the body. In some embodiments, the inflammation presents as swelling of the joint, and the methods of the present disclosure reduce the swelling of the joint. In some embodiments, the inflammation presents as pain in the joint, and the methods of the present disclosure reduce the pain in the joint. In other embodiments, the inflammation presents as decreased range of motion of the joint, and the methods of the present invention increase the range of motion of the joint. In certain embodiments, the method further provides measuring inflammation in one or more joints prior to administration of the effective amount of a composition or a chimeric protein comprising a clotting factor and an Fc region. In some embodiments, the method further provides measuring inflammation in one or more joints after administration of the effective amount of a composition or a chimeric protein comprising a clotting factor and an Fc region.

In other embodiments, the hemophilic arthropathy is evidenced by the expression of one or more biomarkers associated with joint inflammation and/or joint damage. In some embodiments, one or more biomarkers are upregulated in the human indicating increased joint inflammation and/or joint damage. In some embodiments, one or more biomarkers are downregulated in the human indicating increased joint inflammation and/or joint damage. In some embodiments, a human in need of treatment is identified based on the expression of one or more biomarkers associated with increased responsiveness to treatment using the methods of the present disclosure.

In certain embodiments, the chimeric protein comprising the clotting factor and the Fc region localizes to a target joint following administration more so than the clotting factor alone. In certain embodiments, the chimeric protein comprising the clotting factor and the Fc region remains localized to the one or more target joints for a longer period of time than the clotting factor alone following administration.

In yet other embodiments, a subject is identified who exhibits one or more markers for reversible hemophilic arthropathy, and then the chimeric protein comprising the clotting factor and the Fc region is administered.

In some embodiments, the chimeric protein for use of the present invention prevents or reduces the occurrence of vascular remodeling in a joint of a human having hemophilia. A common element associated with hemophilic arthropathy is remodeling of the vasculature surrounding a joint, in particular a target joint. Vascular remodeling can be characterized by increased angiogenesis and increased occurrence of microbleeds into the joint. In certain embodiments, the vascular remodeling is in a target joint. In other embodiments, the vascular remodeling is in a joint other than a target joint. In certain embodiments, the vascular remodeling is in a load bearing joint, *e.g.,* a joint selected from the group consisting of one or both knees, one or both ankles, one or both hips, one or more joints of the foot, and any combination thereof. In another embodiment, the vascular remodeling is in a non-load bearing joint, *e.g.,* a joint selected from the group consisting of one or both elbows, one or both shoulders, one or both wrists, one or more joints of the hand, or any combination thereof. In another embodiment, the vascular remodeling is in a knee. In some embodiments, the vascular remodeling is in a muscle. In some embodiments, the vascular remodeling is in the spleen and/or the liver.

In certain embodiments, the chimeric protein for use of the present invention improves the surrounding soft tissue of a joint of a human having hemophilia. Another common pathology of hemophilic arthropathy is hyperproliferation of the soft tissue of the joint. In some embodiments, the soft tissue improved by the chimeric protein for use of the present disclosure is in any joint in the body. In certain embodiments, the soft tissue improved by the chimeric protein for use of the present disclosure is in a target joint. In other embodiments, the soft tissue improved by the chimeric protein for use of the present disclosure is in a joint other than a target joint.

In some embodiments, the chimeric protein for use of the present invention reduces the severity of one or more symptom associated with hyperproliferation of the soft tissue of a joint. In some embodiments, the hyperproliferation of the soft tissue of a joint presents as swelling of the joint, and the methods of the present disclosure reduce the swelling of the joint. In some embodiments, the hyperproliferation of the soft tissue of a joint presents as pain in the joint, and the methods of the present disclosure reduce the pain in the joint. In other embodiments, the hyperproliferation of the soft tissue of a joint presents as decreased range of motion of the joint, and the methods of the present invention increase the range of motion of the joint.

The chimeric protein for use disclosed herein can be used on a subject who has been treated and has shown decreased hemophilic arthropathy to prevent further developments of hemophilic arthropathy of one or more joints. In some embodiments, the chimeric protein for use of the present disclosure is applied to a subject to treat an existing hemophilic arthropathy of one or more joints and to prevent the development of further hemophilic arthropathy on the same or a different joint.

In some embodiments, the chimeric protein for use of the present disclosure allows the clotting factor to distribute to tissues outside of the plasma compartment as well as in the plasma compartment.

The chimeric protein for use of the present disclosure improves the joint health of one or more joint in a human having hemophilia. Joint health can be measured using any metrics known in the art. In some embodiments, joint health is measured using the hemophilia joint health score (HJHS) system (*see* Feldman et al., "Hemophilia Joint Health Score (HJHS) 2.1," available at http://www.wfh.org/en/page.aspx?pid=885 (last accessed November 18, 2016)). The HJHS measures joint health, in the domain of body structure and function (i.e. impairment), of the joints most commonly affected by bleeding in hemophilia: the knees, ankles, and elbows. Though it was primarily designed for children with hemophilia aged 4-18 years with mild joint impairment (*e.g.,* treated with prophylaxis), it can be applied to any population. In some embodiments, the HJHS measures swelling, duration (of swelling), muscle atrophy, crepitus on motion, flexion loss, extension loss, joint pain, and strength for each of the left and right elbows, left and right knees, and left and right ankles of a human having hemophilia. In some embodiments, each parameter is assigned a numerical score. In one particular embodiment, standard HJHS, version 2.1 is used to measure joint health. In some embodiments, swelling is scored from 0 to 3, with 0 being no swelling, and 3 being severe swelling. In some embodiments, duration of swelling is scored from 0 to 1, with 0 being no swelling or swelling for less than 6 months, and 1 being swelling for greater than or equal to 6 months. In some embodiments, muscle atrophy is scored from 0 to 2, 0 being no atrophy, and 2 being severe atrophy. In some embodiments, crepitus in motion is scored from 0 to 2, 0 being no crepitus in motion, and 2 being severe crepitus in motion. In some embodiments, flexion loss is scored from 0 to 3, 0 being less than 5° flexion loss, and 3 being greater than 20° flexion loss. In some embodiments, extension loss is scored from 0 to 3, 0 being less than 5° extension loss, and 3 being greater than 20° extension loss. In some embodiments, joint pain is scored from 0 to 2, with 0 being no pain through active range of motion, and 2 being pain through active range of motion. In some embodiments, global gait is scored, with 0 reflecting that all skills are within normal limits; 1, 2, and 3 reflecting that 1, 2, and 3 skills, respectively, are not within normal limits; and 4 reflecting that no skills are within normal limits. In certain embodiments, the global gait score is measured based on walking, climbing stairs, running, and/or hopping on one leg. In some embodiments, the scores are combined to generate a total score. In other embodiments, individual scores for one or more joints are evaluated as an indication of the health of the one or more joints.

In other embodiments, a modified HJHS system is used to measure joint health. In some embodiments, the mHJHS differs from the standard HJHS, version 2.1, in that the response options for joint pain and gait were condensed into fewer categories, an assessment for instability was added, and the total score is lower (range, 0-116; 0 indicates normal joint function, 116 indicates severe disease) compared with the standard HJHS (range, 0-124). Scores preceded by a bleed within 2 weeks were excluded. Scores for joints that underwent surgical interventional were imputed using last observation carry forward. To evaluate year-by-year change, rFVIIIFc extension study subjects who had mHJHS data at 4 time points (rFVIIIFc pivotal phase 3 trial baseline, rFVIIIFc extension study baseline, rFVIIIFc extension study Year 1, and rFVIIIFc extension study Year 2) were included in this post hoc analysis. Change in mHJHS score from rFVIIIFc pivotal phase 3 trial baseline to rFVIIIFc extension study Year 2 (negative value indicates improvement) was summarized using descriptive statistics. Change in mHJHS score from rFVIIIFc pivotal phase 3 trial baseline to follow-up visits was summarized for: (1) total score (range, 0-116; by pre-study regimen (prophylactic versus episodic); by severity of functional impairment based on initial mHJHS; and by presence of target joints at baseline; (2) target joints (range, 0-19: sum of all questions for a single target joint); (3) Weight bearing (e.g., ankle and knee) and non-weight bearing (e.g., elbow) joints (range, 0-38: sum of right and left joints of a single location); and (4) individual components (range of motion (range, 0-36: combination of questions "Extension loss [dorsiflexion of ankles]" and "Flexion loss [plantarflexion of ankles]" of all joints); swelling (range, 0-24: combination of questions "Swelling" and "Duration of swelling" of all joints); and strength (range, 0-6: sum of all joints)).

In some embodiments, joint scoring is done separately for the 6 joints (left ankle-LA, right ankle-RA, left elbow-LE, right elbow-RE, left knee-LK, right knee-RK). In some embodiments, swelling is scored according to the following: 0 = none; 1 = mild; 2 = moderate; 3 = severe. In some embodiments, duration of swelling is scored according to the following: 0 = no swelling or ≤ 6 months; 1 = >6 months. In some embodiments, muscle atrophy is scored according to the following: 0 = none; 1 = mild; 2 = severe. In some embodiments, crepitus on motion is scored according to the following: 0 = absent; 1 = present. In some embodiments, flexion loss, including loss of plantarflexion of ankles, is scored according to the following: 0 = none; 1 = mild; 2 = moderate; 3 = severe. In some embodiments, extension loss, including loss of dorsiflexion of ankles, is scored according to the following: 0 = none; 1 = mild; 2 = moderate; 3 = severe. In some embodiments, instability is scored according to the following: 0 = none; 1 = significant pathologic joint laxity. In some embodiments, joint pain is scored according to the following: 0 = no pain, either through range or at end range of motion; 1 = present. In some embodiments, strength is scored according to the following: 0 = normal (holds position against gravity and maximum resistance); 1 = minimal decrease (holds position against gravity and moderate resistance, but not maximum resistance); 2 = mild decrease (holds position against gravity or minimal resistance); 3 = moderate decrease (able to move joint if gravity eliminated); 4 = severe decrease (trace or no muscle contraction). In certain embodiments, for scoring flexion loss and extension loss at knees and elbows, the following applies: none = approximately 0-5 degrees; mild = approximately 5-10 degrees; moderate = approximately 11-20 degrees; and severe = approximately >20 degrees.

In some embodiments, gait is scored once (range is 0-2), wherein 0 = no difficulty with walking or climbing up/down stairs; 1 = no difficulty with walking, but difficulty with stairs; and 2 = difficulty with walking and with stairs.

In certain embodiments, joint scoring is done separately for the 6 joints (left ankle-LA, right ankle-RA, left elbow-LE, right elbow-RE, left knee-LK, right knee-RK) according to the following categories and scales (range is 0-19 for each joint and 0-114 for all six joints): swelling (0 = none; 1 = mild; 2 = moderate; 3 = severe); duration of swelling (0 = no swelling or ≤ 6 months; 1 = >6 months); muscle atrophy (0 = none; 1 = mild; 2 = severe); crepitus on motion (0 = absent; 1 = present); flexion loss, including loss of plantarflexion of ankles (0 = none; 1 = mild; 2 = moderate; 3 = severe); extension loss, including loss of dorsiflexion of ankles (0 = none; 1 = mild; 2 = moderate; 3 = severe); instability (0 = none; 1 = significant pathologic joint laxity); joint pain (0 = no pain, either through range or at end range of motion; 1 = present); strength (0 = normal (holds position against gravity and maximum resistance); 1 = minimal decrease (holds position against gravity and moderate resistance, but not maximum resistance); 2 = mild decrease (holds position against gravity or minimal resistance); 3 = moderate decrease (able to move joint if gravity eliminated); 4 = severe decrease (trace or no muscle contraction); wherein, for scoring flexion loss and extension loss at knees and elbows, the following applies: none = approximately 0-5 degrees; mild = approximately 5-10 degrees; moderate = approximately 11-20 degrees; and severe = approximately >20 degrees.

In some embodiments, the administration improves a HJHS score of the human relative to the HJHS score prior to the administration. In some embodiments, the HJHS score is a total HJHS score, which includes the sum of all joint scores measured plus a global gate score. In some embodiments, the HJHS score is a total HJHS score, which includes the sum of all joint scores measured and not a global gate score. In other embodiments, the HJHS score is for one or both elbows, one or both knees, one or both ankles, or any combination thereof. In one particular embodiment, the HJHS score is for an elbow. In another embodiment, the HJHS score is for a knee. In another embodiment, the HJHS score is for an ankle. In another embodiment, the HJHS score reflects global gait.

In some embodiments, the administration reduces joint pain in the human. In some embodiments, the joint pain is reduced relative to the joint pain prior to the administration. In certain embodiments, the method further provides measuring joint pain in one or more joints prior to administration of the effective amount of a composition or a chimeric protein comprising a clotting factor and an Fc region. In some embodiments, the chimeric protein for use further provides measuring joint pain in one or more joints after administration of the effective amount of a composition or a chimeric protein comprising a clotting factor and an Fc region.

In certain embodiments, the effects of the present chimeric protein for use are observed in one or more joints of the human. In some embodiments, the effects of the present chimeric protein for use are observed in at least one joint. In some embodiments, the effects of the present chimeric protein for use are observed in at least two, at least three, at least four, at least five, at least six, at least seven, at least eight, at least nine, or at least ten joints.

In some embodiments, the chimeric protein for use of the present disclosure further comprises identifying a human in need of treatment, *e.g*., identifying a subject having hemophilic arthropathy. Hemophilic arthropathy can be detected and/or monitored using any methods known in the art. In some embodiments, an imaging system is used to detect and/or monitor a hemophilic arthropathy in a subject. In some embodiments, the imaging system comprises any imaging system known in the art that is used to characterize joints. For example, in certain embodiments, the imaging system comprises a radiography, a magnetic resonance imaging, an ultrasonography, power Doppler sonography, or any combination thereof.

In some embodiments, the subject has been previously treated with a clotting factor protein not fused to an Fc portion. This clotting factor can be a full length or mature clotting factor. In some embodiments, such clotting factor can be ADVATE^{®}, RECOMBINATE^{®}, KOGENATE FS^{®}, HELIXATE FS^{®}, XYNTHA^{®}/REFACTO AB^{®}, HEMOFIL-M^{®}, MONARC-M^{®}, MONOCLATE-P^{®}, HUMATE-P^{®}, ALPHANATE^{®}, KOATE-DVI^{®}, AFSTYLA^{®}, AND HYATE:C^{®}, IDELVION^{®}.

### II.A. Chimeric Proteins

The chimeric protein for use of the present invention comprises a factor VIII (FVIII) and a first Fc region derived from human IgG1.

Not being bound by any theory, it is believed that an Fc region fused to a clotting factor is useful in treating reversible hemophilic arthropathy. Inflammation in hemophilia occurs during bleeding into joints. It has been shown that TNF-α, an inflammatory cytokine involved in hemophilic arthropathy, induces NF-κB signaling and thereby upregulates FcRn expression in human monocytes (Liu et al., J Immunol, 2007. 179(5): p. 2999-3011). FcRn can thus be upregulated at sites of inflammation, allowing Fc-containing proteins to localize to sites of inflammation through binding to Fc receptors as part of the regulation of the inflammatory processes. The Fc region fused to a clotting factor can also interact with inhibitory Fc receptors that can cause immune modulation and down regulation of inflammatory pathways. For example, rFVIIIFc could block the Fc neonatal receptor (FcRn) and activate Fcγ receptors (FcγR), leading to altered levels of pro- and anti-inflammatory molecules. Thus, in some embodiments, the Fc region of the chimeric protein facilitates localization of the chimeric protein to the joint, *e.g.,* the site of inflammation and/or injury and/or damage.

### II.A.1. Factor VIII

"Factor VIII," abbreviated throughout the instant application as "FVIII," as used herein, means functional FVIII polypeptide in its normal role in coagulation, unless otherwise specified. Thus, the term FVIII includes variant polypeptides that are functional. "A FVIII protein" is used interchangeably with FVIII polypeptide (or protein) or FVIII. Examples of the FVIII functions include, but are not limited to, an ability to activate coagulation, an ability to act as a cofactor for factor IX, or an ability to form a tenase complex with factor IX in the presence of Ca²⁺ and phospholipids, which then converts Factor X to the activated form Xa. The FVIII protein can be the human, porcine, canine, rat, or murine FVIII protein. In addition, comparisons between FVIII from humans and other species have identified conserved residues that are likely to be required for function (Cameron et al., Thromb. Haemost. 79:317-22 (1998); US 6,251,632). The full length polypeptide and polynucleotide sequences are known, as are many functional fragments, mutants and modified versions. Various FVIII amino acid and nucleotide sequences are disclosed in, *e.g.,* US Publication Nos. 2015/0158929 A1, 2014/0308280 A1, and 2014/0370035 A1 and International Publication No. WO 2015/106052 A1. FVIII polypeptides include, *e.g*., full-length FVIII, full-length FVIII minus Met at the N-terminus, mature FVIII (minus the signal sequence), mature FVIII with an additional Met at the N-terminus, and/or FVIII with a full or partial deletion of the B domain. FVIII variants include B domain deletions, whether partial or full deletions.

In some embodiments, the FVIII of the chimeric protein or composition of the present disclosure comprises a B domain deleted FVIII. A "B domain" of FVIII, as used herein, is the same as the B domain known in the art that is defined by internal amino acid sequence identity and sites of proteolytic cleavage by thrombin, *e.g*., residues Ser741-Arg1648 of mature human FVIII. The other human FVIII domains are defined by the following amino acid residues, relative to mature human FVIII: A1, residues Ala1-Arg372; A2, residues Ser373-Arg740; A3, residues Ser1690-Ile2032; C1, residues Arg2033-Asn2172; C2, residues Ser2173-Tyr2332 of mature FVIII. The sequence residue numbers used herein without referring to any SEQ ID Numbers correspond to the FVIII sequence without the signal peptide sequence (19 amino acids) unless otherwise indicated. The A3-C1-C2 sequence, also known as the FVIII heavy chain, includes residues Ser1690-Tyr2332. The remaining sequence, residues Glu1649-Arg1689, is usually referred to as the FVIII light chain activation peptide. The locations of the boundaries for all of the domains, including the B domains, for porcine, mouse and canine FVIII are also known in the art. In one embodiment, the B domain of FVIII is deleted ("B-domain-deleted FVIII" or "BDD FVIII"). An example of a BDD FVIII is REFACTO^{®} (recombinant BDD FVIII). In one particular embodiment the B domain deleted FVIII variant comprises a deletion of amino acid residues 746 to 1648 of mature FVIII.

A "B-domain-deleted FVIII" may have the full or partial deletions disclosed in U.S. Pat. Nos. 6,316,226, 6,346,513, 7,041,635, 5,789,203, 6,060,447, 5,595,886, 6,228,620, 5,972,885, 6,048,720, 5,543,502, 5,610,278, 5,171,844, 5,112,950, 4,868,112, and 6,458,563 and Int'l Publ. No. WO 2015106052 A1 (PCT/US2015/010738). In some embodiments, a B-domain-deleted FVIII sequence used in the methods of the present disclosure comprises any one of the deletions disclosed at col. 4, line 4 to col. 5, line 28 and Examples 1-5 of U.S. Pat. No. 6,316,226 (also in US 6,346,513). In another embodiment, a B-domain deleted Factor VIII is the S743/Q1638 B-domain deleted Factor VIII (SQ BDD FVIII) (*e.g.,* Factor VIII having a deletion from amino acid 744 to amino acid 1637, *e.g.,* Factor VIII having amino acids 1-743 and amino acids 1638-2332 of mature FVIII). In some embodiments, a B-domain-deleted FVIII used in the methods of the present disclosure has a deletion disclosed at col. 2, lines 26-51 and examples 5-8 of U.S. Patent No. 5,789,203 (also US 6,060,447, US 5,595,886, and US 6,228,620). In some embodiments, a B-domain-deleted Factor VIII has a deletion described in col. 1, lines 25 to col. 2, line 40 of US Patent No. 5,972,885; col. 6, lines 1-22 and example 1 of U.S. Patent no. 6,048,720; col. 2, lines 17-46 of U.S. Patent No. 5,543,502; col. 4, line 22 to col. 5, line 36 of U.S. Patent no. 5,171,844; col. 2, lines 55-68, figure 2, and example 1 of U.S. Patent No. 5,112,950; col. 2, line 2 to col. 19, line 21 and table 2 of U.S. Patent No. 4,868,112; col. 2, line 1 to col. 3, line 19, col. 3, line 40 to col. 4, line 67, col. 7, line 43 to col. 8, line 26, and col. 11, line 5 to col. 13, line 39 of U.S. Patent no. 7,041,635; or col. 4, lines 25-53, of U.S. Patent No. 6,458,563. In some embodiments, a B-domain-deleted FVIII has a deletion of most of the B domain, but still contains amino-terminal sequences of the B domain that are essential for *in vivo* proteolytic processing of the primary translation product into two polypeptide chains, as disclosed in WO 91/09122. In some embodiments, a B-domain-deleted FVIII is constructed with a deletion of amino acids 747-1638, *i.e.,* virtually a complete deletion of the B domain. Hoeben R.C., et al. J. Biol. Chem. 265 (13): 7318-7323 (1990). A B-domain-deleted Factor VIII may also contain a deletion of amino acids 771-1666 or amino acids 868-1562 of FVIII. Meulien P., et al. Protein Eng. 2(4): 301-6 (1988). Additional B domain deletions that are part of the invention include: deletion of amino acids 982 through 1562 or 760 through 1639 (Toole et al., Proc. Natl. Acad. Sci. U.S.A. (1986) 83, 5939-5942)), 797 through 1562 (Eaton, et al. Biochemistry (1986) 25:8343-8347)), 741 through 1646 (Kaufman (PCT published application No. WO 87/04187)), 747-1560 (Sarver, et al., DNA (1987) 6:553-564)), 741 through 1648 (Pasek (PCT application No.88/00831)), or 816 through 1598 or 741 through 1648 (Lagner (Behring Inst. Mitt. (1988) No 82:16-25, EP 295597)). In one particular embodiment, the B-domain-deleted FVIII comprises a deletion of amino acid residues 746 to 1648 of mature FVIII. In another embodiment, the B-domain-deleted FVIII comprises a deletion of amino acid residues 745 to 1648 of mature FVIII.

In other embodiments, BDD FVIII includes a FVIII polypeptide containing fragments of the B-domain that retain one or more N-linked glycosylation sites, *e.g.*, residues 757, 784, 828, 900, 963, or optionally 943, which correspond to the amino acid sequence of the full-length FVIII sequence. Examples of the B-domain fragments include 226 amino acids or 163 amino acids of the B-domain as disclosed in Miao, H.Z., et al., Blood 103(a): 3412-3419 (2004), Kasuda, A, et al., J. Thromb. Haemost. 6: 1352-1359 (2008), and Pipe, S.W., et al., J. Thromb. Haemost. 9: 2235-2242 (2011) *(i.e.,* the first 226 amino acids or 163 amino acids of the B domain are retained). In still other embodiments, BDD FVIII further comprises a point mutation at residue 309 (from Phe to Ser) to improve expression of the BDD FVIII protein. *See* Miao, H.Z., et al., Blood 103(a): 3412-3419 (2004). In still other embodiments, the BDD FVIII includes a FVIII polypeptide containing a portion of the B-domain, but not containing one or more furin cleavage sites *(e.g.,* Arg1313 and Arg 1648). *See* Pipe, S.W., et al., J. Thromb. Haemost. 9: 2235-2242 (2011). In some embodiments, the BDD FVIII comprises a single chain FVIII that contains a deletion in amino acids 765 to 1652 corresponding to the mature full length FVIII (also known as rVIII-SingleChain and AFSTYLA^{®}). *See* US Patent No. 7,041,635. Each of the foregoing deletions may be made in any FVIII sequence.

A great many functional FVIII variants are known, as is discussed above and below. In addition, hundreds of nonfunctional mutations in FVIII have been identified in hemophilia patients, and it has been determined that the effect of these mutations on FVIII function is due more to where they lie within the 3-dimensional structure of FVIII than on the nature of the substitution (Cutler et al., Hum. Mutat. 19:274-8 (2002)). In addition, comparisons between FVIII from humans and other species have identified conserved residues that are likely to be required for function (Cameron et al., Thromb. Haemost. 79:317-22 (1998); US 6,251,632).

In some embodiments, an effective amount of the chimeric protein comprising the FVIII and the Fc region is equivalent to an effective amount of the FVIII without the Fc region. The effective amount is from 20 IU/kg to 300 IU/kg. In some embodiments, the effective amount is about 20 IU/kg to about 250 IU/kg, about 20 IU/kg to about 200 IU/kg, about 20 IU/kg to about 190 IU/kg, about 20 IU/kg to about 180 IU/kg, about 20 IU/kg to about 170 IU/kg, about 20 IU/kg to about 160 IU/kg, about 20 IU/kg to about 150 IU/kg, about 20 IU/kg to about 140 IU/kg, about 20 IU/kg to about 130 IU/kg, from about 20 IU/kg to about 120 IU/kg, from about 20 IU/kg to about 110 IU/kg, from about 20 IU/kg to about 100 IU/kg, from about 20 IU/kg to about 90 IU/kg, from about 20 IU/kg to about 80 IU/kg, from about 20 IU/kg to about 70 IU/kg, from about 20 IU/kg to about 60 IU/kg, from about 25 IU/kg to about 100 IU/kg, from about 25 IU/kg to about 90 IU/kg, from about 25 IU/kg to about 80 IU/kg, from about 25 IU/kg to about 70 IU/kg, from about 25 IU/kg to about 65 IU/kg. In one particular embodiment, the effective amount is from about 20 IU/kg to about 100 IU/kg. In another embodiment, the effective amount is from about 25 IU/kg to about 65 IU/kg. In other embodiments, the effective amount is from about 20 IU/kg to about 100 IU/kg, from about 30 IU/kg to about 100 IU/kg, from about 40 IU/kg to about 100 IU/kg, from about 50 IU/kg to about 100 IU/kg, from about 60 IU/kg to about 100 IU/kg, from about 70 IU/kg to about 100 IU/kg, from about 80 IU/kg to about 100 IU/kg, from about 90 IU/kg to about 100 IU/kg, from about 20 IU/kg to about 90 IU/kg, from about 20 IU/kg to about 80 IU/kg, from about 20 IU/kg to about 70 IU/kg, from about 20 IU/kg to about 60 IU/kg, from about 20 IU/kg to about 50 IU/kg, from about 20 IU/kg to about 40 IU/kg, or from about 20 IU/kg to about 30 IU/kg.

In some embodiments, the effective amount is 20 IU/kg, about 25 IU/kg, about 30 IU/kg, about 35 IU/kg, about 40 IU/kg, about 45 IU/kg, about 50 IU/kg, about 55 IU/kg, about 60 IU/kg, about 65 IU/kg, about 70 IU/kg, about 75 IU/kg, about 80 IU/kg, about 85 IU/kg, about 90 IU/kg, about 95 IU/kg, about 100 IU/kg, about 105 IU/kg, about 110 IU/kg, about 115 IU/kg, about 120 IU/kg, about 125 IU/kg, about 130 IU/kg, about 135 IU/kg, about 140 IU/kg, about 145 IU/kg, about 150 IU/kg, about 155 IU/kg, about 160 IU/kg, about 165 IU/kg, about 170 IU/kg, about 175 IU/kg, about 180 IU/kg, about 185 IU/kg, about 190 IU/kg, about 195 IU/kg, about 200 IU/kg, about 225 IU/kg, about 250 IU/kg, about 275 IU/kg, or 300 IU/kg. In one particular embodiment, the effective amount is about 50 IU/kg. In another embodiment, the effective amount is about 100 IU/kg. In another embodiment, the effective amount is about 200 IU/kg.

The dosing interval when administering the chimeric protein comprising FVIII and an Fc region or a fragment thereof can be at least about one and one-half times longer than the dosing interval required for an equivalent dose of said clotting factor without the Fc domain. The dosing interval can be at least about one and one-half to six times longer, one and one-half to five times longer, one and one-half to four times longer, one and one-half to three times longer, or one and one-half to two times longer, than the dosing interval required for an equivalent dose of said FVIII without the Fc domain.

In some embodiments, the effective dose of the chimeric protein comprising the FVIII and the Fc region is administered to the human at a dosing interval of about two days, about three days, about four days, about five days, about six days, about seven days, about eight days, about nine days, about ten days, about 11 days, about 12 days, about 13 days, about 14 days, about 15 days, about 16 days, about 17 days, about 18 days, about 19 days, about 20 days, about 21 days, about 22 days, about 23 days, or about 24 days. In some embodiments, the effective dose of the composition or chimeric protein comprising a FVIII and an Fc region is administered to the human at a dosing interval of about 25 days, about 26 days, about 27 days, about 28 days, about 29 days, about 30 days, about 45 days, or about 60 days.

In some embodiments, the chimeric protein comprising the FVIII and the Fc region is administered at a dosing interval of about 1 to about 14 days, about 1 to about 13 days, about 1 to about 12 days, about 1 to about 11 days, about 1 to about 10 days, about 1 to about 9 days, about 1 to about 8 days, about 1 to about 7 days, about 1 to about 6 days, about 1 to about 5 days, about 1 to about 4 days, about 1 to about 3 days, about 1 to about 2 days, about 2 to about 14 days, about 3 to about 14 days, about 4 to about 14 days, about 5 to about 14 days, about 6 to about 14 days, about 7 to about 14 days, about 8 to about 14 days, about 9 to about 14 days, about 10 to about 14 days, about 11 to about 14 days, about 12 to about 14 days, about 13 to about 14 days, or about 5 to about 10 days. In other embodiments, the chimeric protein comprising the FVIII and the Fc region is administered at a dosing interval of about 1 to about 21 days, about 1 to about 20 days, about 1 to about 19 days, about 1 to about 18 days, about 1 to about 17 days, about 1 to about 16 days, about 1 to about 15 days, about 1 to about 14 days, about 1 to about 13 days, about 1 to about 12 days, about 1 to about 11 days, about 1 to about 10 days, about 1 to about 9 days, about 1 to about 8 days, about 1 to about 7 days, about 1 to about 6 days, about 1 to about 5 days, about 1 to about 4 days, about 1 to about 3 days, about 1 to about 2 days, about 2 to about 21 days, about 3 to about 21 days, about 4 to about 21 days, about 5 to about 21 days, about 6 to about 21 days, about 7 to about 21 days, about 8 to about 21 days, about 9 to about 21 days, about 10 to about 21 days, about 11 to about 21 days, about 12 to about 21 days, about 13 to about 21 days, about 14 to about 21 days, about 15 to about 21 days, about 16 to about 21 days, about 17 to about 21 days, about 18 to about 21 days, about 19 to about 21 days, about 20 to about 21 days, about 5 to about 10 days, about 10 to about 15 days, about 15 to about 20 days. In certain embodiments, the chimeric protein comprising the FVIII and the Fc region is administered at a dosing interval of about 2 to about 6 days. In another embodiment, the chimeric protein comprising the FVIII and the Fc region is administered at a dosing interval of about 3 to about 5 days.

In one embodiment, the effective dose is 25-65 IU/kg (25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, or 65 IU/kg) and the dosing interval is once every 3-5, 3-6, 3-7, 3, 4, 5, 6, 7, or 8 or more days, or three times per week, or no more than three times per week. In another embodiment, the effective dose is 65 IU/kg and the dosing interval is once weekly, or once every 6-7 days. The doses can be administered repeatedly as long as they are necessary (e.g., at least 10, 20, 28, 30, 40, 50, 52, or 57 weeks, at least 1, 2, 3, 4, 5, 6, 7, 8, 9, or 10 years). In one particular embodiment, the effective dose is about 25-65 IU/kg and the dosing interval is once every 3-5 days.

The chimeric protein comprising the FVIII and the Fc region can be formulated for any appropriate manner of administration, including, for example, topical (e.g., transdermal or ocular), oral, buccal, nasal, vaginal, rectal or parenteral administration.

The term parenteral as used herein includes subcutaneous, intradermal, intravascular (e.g., intravenous), intramuscular, spinal, intracranial, intrathecal, intraocular, periocular, intraorbital, intrasynovial and intraperitoneal injection, as well as any similar injection or infusion technique. The composition can be also for example a suspension, emulsion, sustained release formulation, cream, gel or powder. The composition can be formulated as a suppository, with traditional binders and carriers such as triglycerides.

In one example, the pharmaceutical formulation is a liquid formulation, *e.g.,* a buffered, isotonic, aqueous solution. In another example, the pharmaceutical composition has a pH that is physiologic, or close to physiologic. In other examples, the aqueous formulation has a physiologic or close to physiologic osmolarity and salinity. It can contain sodium chloride and/or sodium acetate.

In some embodiments, the chimeric protein comprising the FVIII and the Fc region used in accordance with the present invention is formulated in a pharmaceutical composition comprising: (a) the chimeric polypeptide; (b) one or more stabilizing agents selected from sucrose, trehalose, raffinose, arginine, or mixture thereof; (c) sodium chloride (NaCl); (d) L-histidine; (e) calcium chloride; and (f) polysorbate 20 or polysorbate 80. In certain embodiments, the pharmaceutical composition comprises: (a) 50 IU/ml to 2500 IU/ml of the chimeric polypeptide; (b) 10 mg/ml to 25 mg/ml of sucrose; (c) 8.8 mg/ml to 14.6 mg/ml sodium chloride (NaCl); (d) 0.75 mg/ml to 2.25 mg/ml L-histidine; (e) 0.75 mg/ml to 1.5 mg/ml calcium chloride dihydrate; and (f) 0.08 mg/ml to 0.25 mg/ml polysorbate 20 or polysorbate 80. In some examples, the pharmaceutical composition is lyophilized.

In some embodiments, the pharmaceutical composition does not comprise an immune cell. In some embodiments, the pharmaceutical composition does not comprise a cell.

### II.A.2. Factor IX (not according to the invention)

Human Factor IX (FIX) is a serine protease that is an important component of the intrinsic pathway of the blood coagulation cascade. "Factor IX" or "FIX," as used herein, refers to a coagulation factor protein and species and sequence variants thereof, and includes, but is not limited to, the 461 single-chain amino acid sequence of human FIX precursor polypeptide ("prepro"), the 415 single-chain amino acid sequence of mature human FIX, and the R338L FIX (Padua) variant. FIX includes any form of FIX molecule with the typical characteristics of blood coagulation FIX. As used herein "Factor IX" and "FIX" are intended to encompass polypeptides that comprise the domains Gla (region containing γ-carboxyglutamic acid residues), EGF1 and EGF2 (regions containing sequences homologous to human epidermal growth factor), activation peptide ("AP," formed by residues R136-R180 of the mature FIX), and the C-terminal protease domain ("Pro"), or synonyms of these domains known in the art, or can be a truncated fragment or a sequence variant that retains at least a portion of the biological activity of the native protein.

FIX or sequence variants have been cloned, as described in U.S. Patent Nos. 4,770,999 and 7,700,734, and cDNA coding for human Factor IX has been isolated, characterized, and cloned into expression vectors (*see, e.g.,* Choo et al., Nature 299:178-180 (1982); Fair et al., Blood 64:194-204 (1984); and Kurachi et al., Proc. Natl. Acad. Sci., U.S.A. 79:6461-6464 (1982)). One particular variant of FIX, the R338L FIX (Padua) variant, characterized by Simioni et al, 2009, comprises a gain-of-function mutation, which correlates with a nearly 8-fold increase in the activity of the Padua variant relative to native FIX. FIX variants can also include any FIX polypeptide having one or more conservative amino acid substitutions, which do not affect the FIX activity of the FIX polypeptide.

The FIX may comprise coagulation factor IX (recombinant), albumin fusion protein (also known as rIX-FP and IDELVION^{®}).

The FIX polypeptide is 55 kDa, synthesized as a prepropolypeptide chain composed of three regions: a signal peptide of 28 amino acids (amino acids 1 to 28), a propeptide of 18 amino acids (amino acids 29 to 46), which is required for gamma-carboxylation of glutamic acid residues, and a mature Factor IX of 415 amino acids. The propeptide is an 18-amino acid residue sequence N-terminal to the gamma-carboxyglutamate domain. The propeptide binds vitamin K-dependent gamma carboxylase and then is cleaved from the precursor polypeptide of FIX by an endogenous protease, most likely PACE (paired basic amino acid cleaving enzyme), also known as furin or PCSK3. Without the gamma carboxylation, the Gla domain is unable to bind calcium to assume the correct conformation necessary to anchor the protein to negatively charged phospholipid surfaces, thereby rendering Factor IX nonfunctional. Even if it is carboxylated, the Gla domain also depends on cleavage of the propeptide for proper function, since retained propeptide interferes with conformational changes of the Gla domain necessary for optimal binding to calcium and phospholipid. In humans, the resulting mature Factor IX is secreted by liver cells into the blood stream as an inactive zymogen, a single chain protein of 415 amino acid residues that contains approximately 17% carbohydrate by weight (Schmidt, A. E., et al. (2003) Trends Cardiovasc Med, 13: 39).

The mature FIX is composed of several domains that in an N- to C-terminus configuration are: a GLA domain, an EGF1 domain, an EGF2 domain, an activation peptide (AP) domain, and a protease (or catalytic) domain. A short linker connects the EGF2 domain with the AP domain. FIX contains two activation peptides formed by R145-A146 and R180-V181, respectively. Following activation, the single-chain FIX becomes a 2-chain molecule, in which the two chains are linked by a disulfide bond. Clotting factors can be engineered by replacing their activation peptides resulting in altered activation specificity. In mammals, mature FIX must be activated by activated Factor XI to yield Factor IXa. The protease domain provides, upon activation of FIX to FIXa, the catalytic activity of FIX. Activated Factor VIII (FVIIIa) is the specific cofactor for the full expression of FIXa activity.

In other cases, a FIX polypeptide comprises an Thr148 allelic form of plasma derived Factor IX and has structural and functional characteristics similar to endogenous Factor IX.

A great many functional FIX variants are known. International publication number WO 02/040544 A3 discloses mutants that exhibit increased resistance to inhibition by heparin at page 4, lines 9-30 and page 15, lines 6-31. International publication number WO 03/020764 A2 discloses FIX mutants with reduced T cell immunogenicity in Tables 2 and 3 (on pages 14-24), and at page 12, lines 1-27. International publication number WO 2007/149406 A2 discloses functional mutant FIX molecules that exhibit increased protein stability, increased *in vivo* and *in vitro* half-life, and increased resistance to proteases at page 4, line 1 to page 19, line 11. WO 2007/149406 A2 also discloses chimeric and other variant FIX molecules at page 19, line 12 to page 20, line 9. International publication number WO 08/118507 A2 discloses FIX mutants that exhibit increased clotting activity at page 5, line 14 to page 6, line 5. International publication number WO 09/051717 A2 discloses FIX mutants having an increased number of N-linked and/or O-linked glycosylation sites, which results in an increased half-life and/or recovery at page 9, line 11 to page 20, line 2. International publication number WO 09/137254 A2 also discloses Factor IX mutants with increased numbers of glycosylation sites at page 2, paragraph [006] to page 5, paragraph [011] and page 16, paragraph [044] to page 24, paragraph [057]. International publication number WO 09/130198 A2 discloses functional mutant FIX molecules that have an increased number of glycosylation sites, which result in an increased half-life, at page 4, line 26 to page 12, line 6. International publication number WO 09/140015 A2 discloses functional FIX mutants that have an increased number of Cys residues, which can be used for polymer (*e.g.,* PEG) conjugation, at page 11, paragraph [0043] to page 13, paragraph [0053]. The FIX polypeptides are also described in International Application No. PCT/US2011/043569 filed July 11, 2011 and published as WO 2012/006624 on January 12, 2012.

In addition, hundreds of non-functional mutations in FIX have been identified in hemophilia subjects, many of which are disclosed in Table 5, at pages 11-14 of International publication number WO 09/137254 A2. Such non-functional mutations are not included in the invention, but provide additional guidance for which mutations are more or less likely to result in a functional FIX polypeptide.

Factor IX coagulant activity is expressed as International Unit(s) (IU). One IU of FIX activity corresponds approximately to the quantity of FIX in one milliliter of normal human plasma. Several assays are available for measuring Factor IX activity, including the one stage clotting assay (activated partial thromboplastin time; aPTT), thrombin generation time (TGA) and rotational thromboelastometry (ROTEM^{®}). The invention contemplates sequences that have homology to FIX sequences, sequence fragments that are natural, such as from humans, nonhuman primates, mammals (including domestic animals), and non-natural sequence variants which retain at least a portion of the biologic activity or biological function of FIX and/or that are useful for preventing, treating, mediating, or ameliorating a coagulation factor-related disease, deficiency, disorder or condition (e.g., bleeding episodes related to trauma, surgery, of deficiency of a coagulation factor). Sequences with homology to human FIX can be found by standard homology searching techniques, such as NCBI BLAST.

In some cases, an effective amount of the composition or chimeric protein comprising a FIX and an Fc region is equivalent to an effective amount of the FIX without the Fc region. In certain cases, the effective amount is from about 0.1 IU/kg to about 500 IU/kg. In some cases, the effective amount is from about 10 IU/kg to about 400 IU/kg. In some cases, the effective amount is from about 20 IU/kg to about 300 IU/kg. In some cases, the effective amount is about 20 IU/kg to about 275 IU/kg, about 20 IU/kg to about 250 IU/kg, about 20 IU/kg to about 225 IU/kg, about 20 IU/kg to about 200 IU/kg, about 20 IU/kg to about 175 IU/kg, about 20 IU/kg to about 150 IU/kg, about 20 IU/kg to about 100 IU/kg, from about 20 IU/kg to about 90 IU/kg, from about 20 IU/kg to about 80 IU/kg, from about 20 IU/kg to about 70 IU/kg, from about 20 IU/kg to about 60 IU/kg, from about 20 IU/kg to about 50 IU/kg, from about 20 IU/kg to about 40 IU/kg, from about 20 IU/kg to about 30 IU/kg, from about 30 IU/kg to about 100 IU/kg, from about 40 IU/kg to about 100 IU/kg, from about 50 IU/kg to about 100 IU/kg, from about 60 IU/kg to about 100 IU/kg, from about 70 IU/kg to about 100 IU/kg, from about 80 IU/kg to about 100 IU/kg, from about 90 IU/kg to about 100 IU/kg, from about 100 IU/kg to about 200 IU/kg, from about 150 IU/kg to about 200 IU/kg, or from about 25 IU/kg to about 75 IU/kg. In one particular case, the effective amount is from about 20 IU/kg to about 100 IU/kg.

In some cases, the effective amount is about 10 IU/kg, about 15 IU/kg, about 20 IU/kg, about 25 IU/kg, about 30 IU/kg, about 35 IU/kg, about 40 IU/kg, about 45 IU/kg, about 50 IU/kg, about 55 IU/kg, about 60 IU/kg, about 65 IU/kg, about 70 IU/kg, about 75 IU/kg, about 80 IU/kg, about 85 IU/kg, about 90 IU/kg, about 95 IU/kg, about 100 IU/kg, about 105 IU/kg, about 110 IU/kg, about 115 IU/kg, about 120 IU/kg, about 125 IU/kg, about 130 IU/kg, about 135 IU/kg, about 140 IU/kg, about 145 IU/kg, about 150 IU/kg, about 155 IU/kg, about 160 IU/kg, about 165 IU/kg, about 170 IU/kg, about 175 IU/kg, about 180 IU/kg, about 185 IU/kg, about 190 IU/kg, about 195 IU/kg, or about 200 IU/kg. In one particular case, the effective amount is about 50 IU/kg. In another case, the effective amount is about 100 IU/kg.

The dosing interval when administering the composition or chimeric protein comprising FIX and an Fc region or a fragment thereof can be at least about one and one-half times longer than the dosing interval required for an equivalent dose of said FIX without the Fc domain. The dosing interval can be at least about one and one-half to six times longer, one and one-half to five times longer, one and one-half to four times longer, one and one-half to three times longer, or one and one-half to two times longer, than the dosing interval required for an equivalent dose of said FIX without the Fc domain. In some cases, the dosing interval is at least about one and one-half, two, two and one-half, three, three and one-half, four, four and one-half, five, five and one-half or six times longer than the dosing interval required for an equivalent dose of said FIX without the Fc domain. The dosing interval can be about every three, four, five, six, seven, eight, nine, ten, eleven, twelve, thirteen, or fourteen days or longer. The dosing interval can be at least about one and one-half to 5, one and one-half, 2, 3, 4, or 5 days or longer. For on-demand treatment, the dosing interval of said chimeric polypeptide or hybrid is about once every 24-36, 24-48, 24-72, 24-96, 24-120, 24-144, 24-168, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72 hours or longer.

In some cases, the effective dose of the composition or chimeric protein comprising a FIX and an Fc region is administered to the human at a dosing interval of about two days, about three days, about four days, about five days, about six days, about seven days, about eight days, about nine days, about ten days, about 11 days, about 12 days, about 13 days, about 14 days, about 15 days, about 16 days, about 17 days, about 18 days, about 19 days, about 20 days, about 21 days, about 22 days, about 23 days, or about 24 days. In some cases, the effective dose of the composition or chimeric protein comprising a FVIII and an Fc region is administered to the human at a dosing interval of about 25 days, about 26 days, about 27 days, about 28 days, about 29 days, about 30 days, about 45 days, or about 60 days. In certain cases, the effective dose of the composition or chimeric protein comprising a FVIII and an Fc region is administered to the human at a dosing interval of about 7 days, about 8 days, about 9 days, about 10 days, about 11 days, about 12 days, about 13 days, or about 14 days. In one particular case, the effective dose of the composition or chimeric protein comprising a FVIII and an Fc region is administered to the human at a dosing interval of about 2 days (*e.g*., about 48 hours). In another case, the effective dose of the composition or chimeric protein comprising a FVIII and an Fc region is administered to the human at a dosing interval of about 7 days. In another case, the effective dose of the composition or chimeric protein comprising a FVIII and an Fc region is administered to the human at a dosing interval of about 10 days. In some cases, an effective dose of the composition or chimeric protein comprising a FVIII and an Fc region is administered to the human at a dosing interval of every 6-10 hours. In certain cases, the effective dose of the composition or chimeric protein comprising a FVIII and an Fc region is administered to the human at a dosing interval of every day.

In some cases, the composition or chimeric protein comprising a FVIII and an Fc region is administered at a dosing interval of about 1 to about 21 days, about 1 to about 20 days, about 1 to about 19 days, about 1 to about 18 days, about 1 to about 17 days, about 1 to about 16 days, about 1 to about 15 days, about 1 to about 14 days, about 1 to about 13 days, about 1 to about 12 days, about 1 to about 11 days, about 1 to about 10 days, about 1 to about 9 days, about 1 to about 8 days, about 1 to about 7 days, about 1 to about 6 days, about 1 to about 5 days, about 1 to about 4 days, about 1 to about 3 days, about 1 to about 2 days, about 2 to about 21 days, about 3 to about 21 days, about 4 to about 21 days, about 5 to about 21 days, about 6 to about 21 days, about 7 to about 21 days, about 8 to about 21 days, about 9 to about 21 days, about 10 to about 21 days, about 11 to about 21 days, about 12 to about 21 days, about 13 to about 21 days, about 14 to about 21 days, about 15 to about 21 days, about 16 to about 21 days, about 17 to about 21 days, about 18 to about 21 days, about 19 to about 21 days, about 20 to about 21 days, about 5 to about 10 days, about 10 to about 15 days, about 15 to about 20 days.

In one case, the effective dose is a dose that can maintain 1-50 IU/dL of circulating FIX (*e.g.,* at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 IU/dL). In another case, the effective dose is 50 IU/kg and the dosing interval is once weekly. In another case, the effective dose is 100 IU/kg, and the dosing interval is once every 10 days. The doses can be administered repeatedly as long as they are necessary (e.g., at least 10, 20, 28, 30, 40, 50, 52, or 57 weeks, at least 1, 2, 3, 4, 5, 6, 7, 8, 9, or 10 years).

The composition or chimeric protein comprising a FIX and an Fc region can be formulated for any appropriate manner of administration, including, for example, topical (*e.g.,* transdermal or ocular), oral, buccal, nasal, vaginal, rectal or parenteral administration.

The term parenteral as used herein includes subcutaneous, intradermal, intravascular (*e.g.*, intravenous), intramuscular, spinal, intracranial, intrathecal, intraocular, periocular, intraorbital, intrasynovial and intraperitoneal injection, as well as any similar injection or infusion technique. The composition can be also for example a suspension, emulsion, sustained release formulation, cream, gel or powder. The composition can be formulated as a suppository, with traditional binders and carriers such as triglycerides.

In one example, the pharmaceutical formulation is a liquid formulation, *e.g.,* a buffered, isotonic, aqueous solution. In another example, the pharmaceutical composition has a pH that is physiologic, or close to physiologic. In other examples, the aqueous formulation has a physiologic or close to physiologic osmolarity and salinity. It can contain sodium chloride and/or sodium acetate.

In some cases, the chimeric protein comprising a FIX and an Fc region used in the methods of the present invention is formulated in a pharmaceutical composition comprising: (a) the chimeric polypeptide; (b) a carbohydrate mixture comprising sucrose and mannitol; (c) sodium chloride (NaCl); (d) L-histidine; and (e) polysorbate 20 or polysorbate 80. In certain cases, the pharmaceutical composition comprises: (a) between about 25 IU/ml and about 700 IU/ml of the Factor IX polypeptide; (b) between about 10 mg/ml and about 20 mg/ml of sucrose; (c) between about 20 mg/ml and about 40 mg/ml of mannitol; (d) between about 3 mg/ml and about 4 mg/ml NaCl; (e) between about 3 mg/ml and about 6 mg/ml L-histidine; (f) between about 0.08 mg/ml and about 0.2 mg/ml of polysorbate 20 or polysorbate 80; or (g) any combination thereof. In some examples, the pharmaceutical composition used in the methods of the present disclosure is lyophilized.

In some cases, the pharmaceutical composition does not comprise an immune cell. In some cases, the pharmaceutical composition does not comprise a cell.

### H.A.3 Fc

The chimeric protein of the disclosure includes an Fc domain or a portion thereof that binds to FcRn, FcyRIIB and/or DC-SIGN. The Fc domain is fused to the clotting factor as part of the chimeric protein comprising the clotting factor and the Fc region. The Fc domain or a portion thereof can improve pharmacokinetic or pharmacodynamic properties of the chimeric protein. In certain embodiments, the Fc domain or a portion thereof extends a half-life of a molecule fused to the Fc domain or a portion thereof. In some embodiments, the Fc region of the chimeric protein facilitates localization of the chimeric protein to the joint.

As used herein, the term "Fc domain" or "Fc region" as used herein, means the functional portion of a polypeptide which corresponds to the Fc domain of native Ig, *i.e.,* as formed by the dimeric association of the respective Fc domains of its two heavy chains. A native Fc domain forms a homodimer with another Fc domain. In contrast, the term "genetically-fused Fc region" or "single-chain Fc region" (scFc region), as used herein, refers to a synthetic dimeric Fc region comprised of Fc domains genetically linked within a single polypeptide chain (*i.e.,* encoded in a single contiguous genetic sequence).

In one embodiment, the "Fc region" refers to the portion of a single Ig heavy chain beginning in the hinge region just upstream of the papain cleavage site (*i.e.* residue 216 in IgG, taking the first residue of heavy chain constant region to be 114) and ending at the C-terminus of the antibody. Accordingly, a complete Fc domain comprises at least a hinge domain, a CH2 domain, and a CH3 domain.

The Fc region of an Ig constant region, depending on the Ig isotype can include the CH2, CH3, and CH4 domains, as well as the hinge region. Chimeric proteins comprising an Fc region of an Ig bestow several desirable properties on a chimeric protein including increased stability, increased serum half-life (see Capon et al., 1989, Nature 337:525) as well as binding to Fc receptors such as the neonatal Fc receptor (FcRn) (U.S. Pat. Nos. 6,086,875, 6,485,726, 6,030,613; WO 03/077834; US2003-0235536A1).

In some embodiments, the Fc region specifically binds to FcRn. The FcRn receptor has been isolated from several mammalian species including humans. The sequences of the human FcRn, monkey FcRn, rat FcRn, and mouse FcRn are known (Story et al. 1994, J. Exp. Med. 180:2377). The FcRn receptor binds IgG (but not other Ig classes such as IgA, IgM, IgD, and IgE) at relatively low pH, actively transports the IgG transcellularly in a luminal to serosal direction, and then releases the IgG at relatively higher pH found in the interstitial fluids. It is expressed in adult epithelial tissue (U.S. Pat. Nos. 6,485,726, 6,030,613, 6,086,875; WO 03/077834; US2003-0235536A1) including lung and intestinal epithelium (Israel et al. 1997, Immunology 92:69) renal proximal tubular epithelium (Kobayashi et al. 2002, Am. J. Physiol. Renal Physiol. 282:F358) as well as nasal epithelium, vaginal surfaces, and biliary tree surfaces.

Fc regions useful in the present invention encompass molecules that can specifically bind FcRn, FcyRIIB, and/or DC-SIGN, including whole IgG, the Fc fragment of IgG, and other fragments that include the complete binding region of the FcRn receptor. The region of the Fc portion of IgG that binds to FcRn, FcyRIIB, and/or DC-SIGN has been described.

In one particular embodiment, the Fc region specifically binds to a low affinity immunoglobulin gamma Fc region receptor II-b (FcyRIIB). FcyRIIB is an inhibitory Fc receptor, which controls aspects of inflammatory response. In particular, activation of FcyRIIB inhibits activating signals that lead to inflammation. Thus, in effect, activation of FcyRIIB inhibits inflammation.

In another embodiment, the Fc region specifically binds to dendritic cell-specific intercellular adhesion molecule-3-grabbing non-integrin (DC-SIGN). DC-SIGN, also known as CD209, is a C-type lectin receptor expressed by most myeloid derived cells, including certain monocytes, dendritic cells, and macrophages. Studies in mice have revealed that activation of DC-SIGN may inhibit inflammation. *See* Nimerjahn, Chapter 5, Molecular and Cellular Pathways Involved in the Anti-inflammatory Activity of IgG, Molecular Mechanisms of Antibody Activity, New York, NY 2013: 113-138.

Specifically bound refers to two molecules forming a complex that is relatively stable under physiologic conditions. Specific binding is characterized by a high affinity and a low to moderate capacity as distinguished from nonspecific binding which usually has a low affinity with a moderate to high capacity. Typically, binding is considered specific when the affinity constant KA is higher than 10⁶ M⁻¹, or higher than 10⁸ M⁻¹. If necessary, non-specific binding can be reduced without substantially affecting specific binding by varying the binding conditions. The appropriate binding conditions such as concentration of the molecules, ionic strength of the solution, temperature, time allowed for binding, concentration of a blocking agent (e.g. serum albumin, milk casein), etc., may be optimized by a skilled artisan using routine techniques.

In certain embodiments, a chimeric protein of the invention comprises one or more truncated Fc regions that are nonetheless sufficient to confer FcRn, FcyRIIB, and/or DC-SIGN binding properties to the Fc region. For example, the portion of an Fc region that binds to FcRn, FcyRIIB, and/or DC-SIGN (*i.e.,* the FcRn, FcyRIIB, and/or DC-SIGN binding portion) comprises from about amino acids 282-438 of IgG1, EU numbering (with the primary contact sites being amino acids 248, 250-257, 272, 285, 288, 290-291, 308-311, and 314 of the CH2 domain and amino acid residues 385-387, 428, and 433-436 of the CH3 domain. Thus, an Fc region of the invention may comprise or consist of an FcRn, FcyRIIB, and/or DC-SIGN binding portion. According to the invention, an FcRn, FcyRIIB, and/or DC-SIGN binding portion from an antibody of the human isotype IgG1 is used.

In another embodiment, the "Fc region" includes an amino acid sequence of an Fc domain or derived from an Fc domain. In certain embodiments, an Fc region comprises at least one of: a hinge (*e.g.,* upper, middle, and/or lower hinge region) domain (about amino acids 216-230 of an antibody Fc region according to EU numbering), a CH2 domain (about amino acids 231-340 of an antibody Fc region according to EU numbering), a CH3 domain (about amino acids 341-438 of an antibody Fc region according to EU numbering), a CH4 domain, or a variant, portion, or fragment thereof. In other embodiments, an Fc region comprises a complete Fc domain (*i.e.,* a hinge domain, a CH2 domain, and a CH3 domain). In some embodiments, an Fc region comprises, consists essentially of, or consists of a hinge domain (or a portion thereof) fused to a CH3 domain (or a portion thereof), a hinge domain (or a portion thereof) fused to a CH2 domain (or a portion thereof), a CH2 domain (or a portion thereof) fused to a CH3 domain (or a portion thereof), a CH2 domain (or a portion thereof) fused to both a hinge domain (or a portion thereof) and a CH3 domain (or a portion thereof). In still other embodiments, an Fc region lacks at least a portion of a CH2 domain (*e.g.,* all or part of a CH2 domain). In a particular embodiment, an Fc region comprises or consists of amino acids corresponding to EU numbers 221 to 447.

In certain embodiments, the Fc variant confers a change in at least one effector function imparted by an Fc region comprising the wild-type Fc domain (*e.g*., an improvement or reduction in the ability of the Fc region to bind to Fc receptors (*e.g.,* reduction in binding to FcγRI or FcγRIII or improvement in binding to FcRn or FcγRII), complement proteins (*e.g.* C1q), or other Fc binding partners (*e.g.,* improvement in binding to DC-SIGN), or to trigger antibody-dependent cytotoxicity (ADCC), phagocytosis, or complement-dependent cytotoxicity (CDCC)). In other embodiments, the Fc variant provides an engineered cysteine residue.

The Fc regions used in the context of the invention may employ art-recognized Fc variants which are known to impart a change (*e.g.,* an enhancement or reduction) in effector function and/or FcR binding. Specifically, a binding molecule of the invention may include, for example, a change (*e.g.,* a substitution) at one or more of the amino acid positions disclosed in International PCT Publications WO88/07089A1, WO96/14339A1, WO98/05787A1, WO98/23289A1, WO99/51642A1, WO99/58572A1, WO00/09560A2, WO00/32767A1, WO00/42072A2, WO02/44215A2, WO02/060919A2, WO03/074569A2, WO04/016750A2, WO04/029207A2, WO04/035752A2, WO04/063351A2, WO04/074455A2, WO04/099249A2, WO05/040217A2, WO04/044859, WO05/070963A1, WO05/077981A2, WO05/092925A2, WO05/123780A2, WO06/019447A1, WO06/047350A2, and WO06/085967A2; US Patent Publication Nos. US2007/0231329, US2007/0231329, US2007/0237765, US2007/0237766, US2007/0237767, US2007/0243188, US20070248603, US20070286859, US20080057056 ; or US Patents 5,648,260; 5,739,277; 5,834,250; 5,869,046; 6,096,871; 6,121,022; 6,194,551; 6,242,195; 6,277,375; 6,528,624; 6,538,124; 6,737,056; 6,821,505; 6,998,253; 7,083,784; 7,404,956, and 7,317,091. In one embodiment, the specific change (*e.g.,* the specific substitution of one or more amino acids disclosed in the art) may be made at one or more of the disclosed amino acid positions. In another embodiment, a different change at one or more of the disclosed amino acid positions (*e.g.*, the different substitution of one or more amino acid position disclosed in the art) may be made.

The Fc region can be modified according to well recognized procedures such as site directed mutagenesis and the like to yield modified Fc fragments or portions thereof that will be bound by FcRn, FcyRIIB, and/or DC-SIGN. Such modifications include modifications remote from the FcRn, FcyRIIB, and/or DC-SIGN contact sites as well as modifications within the contact sites that preserve or even enhance binding to the FcRn, FcyRIIB, and/or DC-SIGN. For example, the following single amino acid residues in human IgG1 Fc (Fc γ1) can be substituted without significant loss of Fc binding affinity for FcRn, FcyRIIB, and/or DC-SIGN: P238A, S239A, K246A, K248A, D249A, M252A, T256A, E258A, T260A, D265A, S267A, H268A, E269A, D270A, E272A, L274A, N276A, Y278A, D280A, V282A, E283A, H285A, N286A, T289A, K290A, R292A, E293A, E294A, Q295A, Y296F, N297A, S298A, Y300F, R301A, V303A, V305A, T307A, L309A, Q311A, D312A, N315A, K317A, E318A, K320A, K322A, S324A, K326A, A327Q, P329A, A330Q, P331A, E333A, K334A, T335A, S337A, K338A, K340A, Q342A, R344A, E345A, Q347A, R355A, E356A, M358A, T359A, K360A, N361A, Q362A, Y373A, S375A, D376A, A378Q, E380A, E382A, S383A, N384A, Q386A, E388A, N389A, N390A, Y391F, K392A, L398A, S400A, D401A, D413A, K414A, R416A, Q418A, Q419A, N421A, V422A, S424A, E430A, N434A, T437A, Q438A, K439A, S440A, S444A, and K447A, where for example P238A represents wild type proline substituted by alanine at position number 238.

As an example, a specific embodiment incorporates the N297A mutation, removing a highly conserved N-glycosylation site. In addition to alanine other amino acids may be substituted for the wild type amino acids at the positions specified above. Mutations may be introduced singly into Fc giving rise to more than one hundred Fc regions distinct from the native Fc. Additionally, combinations of two, three, or more of these individual mutations may be introduced together, giving rise to hundreds more Fc regions. Moreover, one of the Fc region of a construct of the invention may be mutated and the other Fc region of the construct not mutated at all, or they both may be mutated but with different mutations.

In one embodiment, the Fc domain or a portion thereof is a polypeptide including SEQ ID NO: 3 of U.S. Pat. No. 5,739,277 and optionally further including a sequence selected from SEQ ID NOs: 11, 1, 2, and 31 of U.S. Pat. No. 5,739,277.

In certain embodiments, the Fc domain or a portion thereof is hemi-glycosylated. For example, the chimeric protein comprising two Fc regions may contain a first, glycosylated, Fc region (*e.g.,* a glycosylated CH2 region) and a second, aglycosylated, Fc region (*e.g.,* an aglycosylated CH2 region). In one embodiment, a linker may be interposed between the glycosylated and aglycosylated Fc regions. In another embodiment, the Fc region is fully glycosylated, *i.e.,* all of the Fc regions are glycosylated. In other embodiments, the Fc region may be aglycosylated, *i.e.,* none of the Fc moieties are glycosylated.

In certain embodiments, the chimeric protein for use of the invention comprises an amino acid substitution to an Fc domain or a portion thereof (*e.g.,* Fc variants), which alters the antigen-independent effector functions of Fc domain, in particular the circulating half-life of the protein.

An Fc region used in the invention may also comprise an art recognized amino acid substitution which alters the glycosylation of the chimeric protein. For example, the Fc region of the chimeric protein linked to a FVIII protein or a FIX protein may comprise an Fc region having a mutation leading to reduced glycosylation (*e.g*., N- or O-linked glycosylation) or may comprise an altered glycoform of the wild-type Fc moiety (*e.g.,* a low fucose or fucose-free glycan).

In one embodiment, an unprocessed chimeric protein for use of the invention may comprise a genetically fused Fc region (*i.e.,* scFc region) having two or more of its constituent Ig constant region or a portion thereof independently selected from the Ig constant region or a portion thereof described herein. In one embodiment, the Fc regions of a dimeric Fc region are the same. In another embodiment, at least two of the Fc regions are different. For example, the Fc regions of the proteins of the invention comprise the same number of amino acid residues or they may differ in length by one or more amino acid residues (*e.g*., by about 5 amino acid residues (*e.g*., 1, 2, 3, 4, or 5 amino acid residues), about 10 residues, about 15 residues, about 20 residues, about 30 residues, about 40 residues, or about 50 residues). In yet other embodiments, the Fc regions of the protein of the invention may differ in sequence at one or more amino acid positions. For example, at least two of the Fc regions may differ at about 5 amino acid positions (*e.g.,* 1, 2, 3, 4, or 5 amino acid positions), about 10 positions, about 15 positions, about 20 positions, about 30 positions, about 40 positions, or about 50 positions).

In some embodiments, a chimeric protein used in the context of the present disclosure comprises more than one polypeptide chain. In some embodiments, the chimeric protein comprises two polypeptide chains. In certain embodiments, the first polypeptide chain comprises a clotting factor and a first Fc region, and the second polypeptide chain comprises a second Fc region. In certain embodiments, the first Fc region and the second Fc region are associated by a covalent bond. In one embodiment, the first Fc region and the second Fc region are associated by a peptide bond. In another embodiment, the first Fc region and the second Fc region are associated by a disulfide bond.

In one particular embodiment, the chimeric protein comprises a factor VIII portion and a von Willebrand factor (VWF) portion, wherein the FVIII portion comprises a FVIII polypeptide or a fragment thereof, wherein the VWF portion comprises a VWF polypeptide or a fragment thereof, wherein the FVIII portion is linked to a first Fc region, wherein the VWF portion is linked to a second Fc region, and wherein the first Fc region and the second Fc region are associated with each other. In certain embodiments, the VWF portion comprises the D' and D3 domains of VWF. In one embodiment, the first polypeptide, the second polypeptide, or both the first polypeptide and the second polypeptide further comprise one or more half-life extending moieties.

The Fc region or a portion thereof is derived from a human IgG1.

A variety of the Fc region gene sequences (*e.g*., human Fc gene sequences) are available in the form of publicly accessible deposits. Fc sequences can be selected having a particular effector function (or lacking a particular effector function) or with a particular modification to reduce immunogenicity. Many sequences of antibodies and antibody-encoding genes have been published and suitable Fc region sequences can be derived from these sequences using art recognized techniques. The genetic material obtained using any of the foregoing methods may then be altered or synthesized to obtain chimeric proteins used in the methods of the present disclosure. It will further be appreciated that the scope of this invention encompasses alleles, variants and mutations of constant region DNA sequences.

The sequences of the Fc or a portion thereof can be cloned, *e.g.,* using the polymerase chain reaction and primers which are selected to amplify the domain of interest. To clone a sequence of the Fc region or a portion thereof from an antibody, mRNA can be isolated from hybridoma, spleen, or lymph cells, reverse transcribed into DNA, and antibody genes amplified by PCR. PCR amplification methods are described in detail in U.S. Pat. Nos. 4,683,195; 4,683,202; 4,800,159; 4,965,188; and in, *e.g.,* "PCR Protocols: A Guide to Methods and Applications" Innis et al. eds., Academic Press, San Diego, CA (1990); Ho et al. 1989. Gene 77:51; Horton et al. 1993. Methods Enzymol. 217:270). PCR may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as mouse constant region probes. Numerous primer sets suitable for amplification of antibody genes are known in the art (*e.g*., 5' primers based on the N-terminal sequence of purified antibodies (Benhar and Pastan. 1994. Protein Engineering 7:1509); rapid amplification of cDNA ends (Ruberti, F. et al. 1994. J. Immunol. Methods 173:33); antibody leader sequences (Larrick et al. 1989 Biochem. Biophys. Res. Commun. 160:1250). The cloning of antibody sequences is further described in Newman et al., U.S. Pat. No. 5,658,570, filed January 25, 1995.

### II.B. Half-Life Extending Moieties

In some embodiments, a chimeric protein used in the context of the present disclosure further comprises one or more half-life extending moieties. Half-life of a clotting factor can be determined by any method known to those of skill in the art, *e.g.,* FVIII activity assays (chromogenic assay or one stage clotting aPTT assay) to detect plasma FVIII activity levels or FVIII/FIX ELISA to detect plasma FVIII/FIX antigen level. In a particular embodiment, half-life of the clotting activity of a clotting factor is determined by one stage clotting assay. In a more particular embodiment, half-life of the clotting activity of a clotting factor is determined in mice, either HemA mice or FVIII and von Willebrand Factor double knockout (DKO) mice.

In certain aspects, a heterologous moiety which increases half-life of the clotting factor of the invention comprises, without limitation, a heterologous polypeptide such as albumin, an immunoglobulin Fc region, an XTEN sequence, the C-terminal peptide (CTP) of the β subunit of human chorionic gonadotropin, a PAS sequence, a HAP sequence, a transferrin, albumin-binding moieties, or any fragments, derivatives, variants, or combinations of these polypeptides. In other related aspects a half-life extending moiety can include an attachment site for a non-polypeptide moiety such as polyethylene glycol (PEG), hydroxyethyl starch (HES), polysialic acid, or any derivatives, variants, or combinations of these moieties. In certain embodiments, the half-life extending moiety comprises albumin or a fragment thereof, an albumin binding moiety, a PAS sequence, a HAP sequence, transferrin or a fragment thereof, polyethylene glycol (PEG), polysialic acid, hydroxyethyl starch (HES), a derivative thereof, or any combination thereof. In some embodiments, the half-life extending moiety does not comprise an XTEN. In other embodiments, the half-life extending moiety comprises an XTEN.

In other embodiments, a chimeric protein for use of the invention is conjugated to one or more polymers. The polymer can be water-soluble or non-water-soluble. The polymer can be covalently or non-covalently attached to the clotting factor, the Fc, or to other moieties conjugated to either the clotting factor or the Fc. Non-limiting examples of the polymer can be poly(alkylene oxide), poly(vinyl pyrrolidone), poly(vinyl alcohol), polyoxazoline, or poly(acryloylmorpholine). Additional types of, *e.g*., polymer-conjugated FVIII are disclosed in U.S. Patent No. 7,199,223.

In certain aspects, a chimeric protein of the invention can comprise one, two, three or more half-life extending moieties, which can each be the same or different molecules.

In some embodiments, the half-life extending moiety is fused to the N-terminus or the C-terminus of the chimeric polypeptide. In some embodiments, the half-life extending moiety is fused to the N-terminus or the C-terminus of the clotting factor. In some embodiments, the half-life extending moiety is fused to the N-terminus or the C-terminus of the Fc. In certain embodiments, the half-life extending moiety is inserted within the clotting factor of the chimeric protein.

In some embodiments, the chimeric protein comprises FVIII or a portion thereof, and the half-life extending moiety is inserted within the FVIII at one or more positions disclosed in U.S. Patent Publ. No. 2015-0158929 A1 and/or Int'l Publication No. WO 2015106052 A1. In one particular embodiment, the half-life extending moiety is inserted with in the B domain (or a fragment thereof) of the FVIII. In one particular embodiment, the half-life extending moiety is inserted within the FVIII immediately downstream of amino acid residue 745 of mature FVIII.

Also disclosed but not forming part of the invention are the chimeric protein comprising FIX or a portion thereof, and in which the half-life extending moiety is inserted within the FIX at one or more positions disclosed in Int'l Appl. No. PCT/US16/045401. In one particular case, the half-life extending moiety is inserted within the FIX at an insertion site immediately downstream of an amino acid residue selected from the group consisting of amino acid 103, amino acid 105, amino acid 142, amino acid 149, amino acid 162, amino acid 166, amino acid 174, amino acid 224, amino acid 226, amino acid 228, amino acid 413 of mature Padua FIX. In one case, the chimeric protein comprises a FIX and an Fc region, wherein the FIX comprises a half-life extending moiety inserted within the activation peptide (AP) domain of the FIX. In one particular case, the chimeric protein comprises a FIX and an Fc region, wherein the FIX comprises a half-life extending moiety inserted within the FIX immediately downstream of amino acid residue 166 of mature Padua FIX.

### II.B.1. Albumins

In certain aspects, a chimeric protein used in the methods of the present disclosure comprises at least one albumin polypeptide or fragment, variant, or derivative thereof. Human serum albumin (HSA, or HA), a protein of 609 amino acids in its full-length form, is responsible for a significant proportion of the osmotic pressure of serum and also functions as a carrier of endogenous and exogenous ligands. The term "albumin" as used herein includes full-length albumin or a functional fragment, variant, derivative, or analog thereof. Examples of albumin or the fragments or variants thereof are disclosed in US Pat. Publ. Nos. 2008/0194481A1, 2008/0004206 A1, 2008/0161243 A1, 2008/0261877 A1, or 2008/0153751 A1 or PCT Appl. Publ. Nos. 2008/033413 A2, 2009/058322 A1, or 2007/021494 A2.

The albumin-binding polypeptides (ABPs) can comprise, without limitation, bacterial albumin-binding domains, albumin-binding peptides, or albumin-binding antibody fragments that can bind to albumin. Domain 3 from streptococcal protein G, as disclosed by Kraulis et al., FEBS Lett. 378:190-194 (1996) and Linhult et al., Protein Sci. 11:206-213 (2002) is an example of a bacterial albumin-binding domain. Examples of albumin-binding peptides are disclosed in Dennis et al., J. Biol. Chem. 2002, 277: 35035-35043 (2002). Examples of albumin-binding antibody fragments are disclosed in Muller and Kontermann, Curr. Opin. Mol. Ther. 9:319-326 (2007); Roovers et al., Cancer Immunol. Immunother. 56:303-317 (2007), and Holt et al., Prot. Eng. Design Sci., 21:283-288 (2008).

In certain aspects, a chimeric protein used in the context of the present disclosure comprises at least one attachment site for a non-polypeptide small molecule, variant, or derivative that can bind to albumin thereof. For example, the chimeric protein can include one or more organic albumin-binding moieties. An example of such albumin-binding moieties is 2-(3-maleimidopropanamido)-6-(4-(4-iodophenyl)butanamido)hexanoate ("Albu" tag) as disclosed by Trussel et al., Bioconjugate Chem. 20:2286-2292 (2009).

### II.B.2. XTENs

In certain aspects, a chimeric protein used in the methods of the present disclosure comprises at least one XTEN polypeptide or fragment, variant, or derivative thereof. As used here "XTEN sequence" refers to extended length polypeptides with non-naturally occurring, substantially non-repetitive sequences that are composed mainly of small hydrophilic amino acids, with the sequence having a low degree or no secondary or tertiary structure under physiologic conditions. As a chimeric protein partner, XTENs can serve as a carrier, conferring certain desirable pharmacokinetic, physicochemical and pharmaceutical properties, *e.g*., when fused with or inserted into the clotting factor of the chimeric protein. Such desirable properties include but are not limited to enhanced pharmacokinetic parameters and solubility characteristics.

An XTEN sequence fused with or inserted into the clotting factor of the chimeric protein used in the methods of the present disclosure can confer to the chimeric protein one or more of the following advantageous properties: conformational flexibility, enhanced aqueous solubility, high degree of protease resistance, low immunogenicity, low binding to mammalian receptors, or increased hydrodynamic (or Stokes) radii. In certain aspects, an XTEN sequence can increase pharmacokinetic properties such as longer half-life (*e.g., in vivo* half-life) or increased area under the curve (AUC), so that the chimeric protein stays *in vivo* and has procoagulant activity for an increased period of time compared to the chimeric protein without the XTEN.

Examples of XTEN sequences that can be inserted into recombinant FVIII proteins of the invention are disclosed, *e.g.,* in U.S. Patent Publication Nos. 2010/0239554 A1, 2010/0323956 A1, 2011/0046060 A1, 2011/0046061 A1, 2011/0077199 A1, or 2011/0172146 A1, or International Patent Publication Nos. WO 2010091122 A1, WO 2010144502 A2, WO 2010144508 A1, WO 2011028228 A1, WO 2011028229 A1, WO 2011028344 A2, or WO 2015106052 A1.

### II.B.3. VWF or a Fragment Thereof

In certain aspects, a chimeric protein used in the methods of the present disclosure comprises at least one VWF polypeptide or fragment, variant, or derivative thereof. VWF (also known as F8VWF) is a large, multimeric glycoprotein present in blood plasma and produced constitutively in endothelium (in the Weibel-Palade bodies), megakaryocytes (α-granules of platelets), and subendothelial connective tissue. The basic VWF monomer is a 2813 amino acid protein. Every monomer contains a number of specific domains with a specific function, the D'/D3 domain (which binds to Factor VIII), the A1 domain (which binds to platelet GPIb-receptor, heparin, and/or possibly collagen), the A3 domain (which binds to collagen), the C1 domain (in which the RGD domain binds to platelet integrin αIIbβ3 when this is activated), and the "cysteine knot" domain at the C-terminal end of the protein (which VWF shares with platelet-derived growth factor (PDGF), transforming growth factor-β (TGFβ) and β-human chorionic gonadotropin (βHCG)).

In one embodiment, the VWF polypeptide is a VWF fragment. The term "a VWF fragment" as used herein includes, but is not limited to, functional VWF fragments comprising a D' domain and a D3 domain, which are capable of inhibiting binding of endogenous VWF to FVIII. In one embodiment, the chimeric protein used in the methods of the present disclosure comprises a clotting factor, an Fc region, and a VWF fragment, wherein the clotting factor comprises FVIII, and wherein the VWF fragment binds to the FVIII protein. In another embodiment, the VWF fragment blocks the VWF binding site on the FVIII protein, thereby inhibiting interaction of the FVIII protein with endogenous VWF. The VWF fragments include derivatives, variants, mutants, or analogues that retain these activities of VWF. In certain embodiments, the VWF fragment comprises the D' domain and D3 domain of VWF.

The 2813 monomer amino acid sequence for human VWF is reported as Accession Number _NP_000543.2__ in Genbank. The nucleotide sequence encoding the human VWF is reported as Accession Number __NM_000552.3_ in Genbank.

In certain embodiments, the VWF protein useful herein can be further modified to improve its interaction with FVIII, *e.g.,* to improve binding affinity to FVIII. In other embodiments, the VWF proteins useful for the invention can have other modifications, *e.g*., the protein can be pegylated, glycosylated, hesylated, or polysialylated. Example VWF sequences useful in the methods of the present disclosure are provided, *e.g.,* in U.S. Publication Nos. US 2015/0023959 A1, US 2015/0266943 A1, and US 2015/0158929. In certain embodiments, the VWF protein or a fragment thereof is fused to or co-administered with an FcRn binding partner. In some embodiments, the VWF protein or a fragment thereof is fused to an Fc or co-administered with an Fc or a polypeptide comprising an Fc. In some embodiments, the VWF protein or a fragment thereof is fused to an albumin or co-administered with an albumin or a polypeptide comprising an albumin.

### II.B.4. CTP

In certain aspects, a chimeric protein used in the methods of the present disclosure comprises at least one C-terminal peptide (CTP) of the β subunit of human chorionic gonadotropin or fragment, variant, or derivative thereof. CTP peptides are known to increase the half-life of that protein. *See, e.g.,* U.S. Patent No. 5,712,122. Non-limiting exemplary CTP peptides are disclosed in U.S. Patent Application Publication No. US 2009/0087411 A1.

### II.B.5. PAS

In certain aspects, a chimeric protein used in the methods of the present disclosure comprises at least one PAS peptide or fragment, variant, or derivative thereof. A PAS peptide or PAS sequence, as used herein, means an amino acid sequence comprising mainly alanine and serine residues or comprising mainly alanine, serine, and proline residues, the amino acid sequence forming random coil conformation under physiological conditions. Accordingly, the PAS sequence is a building block, an amino acid polymer, or a sequence cassette comprising, consisting essentially of, or consisting of alanine, serine, and proline which can be used as a part of the heterologous moiety in the chimeric protein. An amino acid polymer also can form random coil conformation when residues other than alanine, serine, and proline are added as a minor constituent in the PAS sequence. By "minor constituent" is meant that that amino acids other than alanine, serine, and proline can be added in the PAS sequence to a certain degree, *e.g.*, up to about 12%, *i.e.*, about 12 of 100 amino acids of the PAS sequence, up to about 10%, up to about 9%, up to about 8%, about 6%, about 5%, about 4%, about 3%, *i.e.* about 2%, or about 1%, of the amino acids. The amino acids different from alanine, serine and proline can be selected from the group consisting of Arg, Asn, Asp, Cys, Gln, Glu, Gly, His, Ile, Leu, Lys, Met, Phe, Thr, Trp, Tyr, and Val. Under physiological conditions, a PAS peptide forms a random coil conformation and thereby can mediate an increased *in vivo* and/or *in vitro* stability to a recombinant protein of the invention, and has procoagulant activity.

Non-limiting examples of the PAS peptides are disclosed in, *e.g.,* US Pat. Publ. No. 2010/0292130 A1; PCT Appl. Publ. No. WO 2008/155134 A1; and European issued patent EP2173890.

### II.B.6. HAP

In certain aspects, a chimeric protein used in the methods of the present disclosure comprises at least one homo-amino acid polymer (HAP) peptide or fragment, variant, or derivative thereof. A HAP peptide can comprise a repetitive sequence of glycine, which has at least 50 amino acids, at least 100 amino acids, 120 amino acids, 140 amino acids, 160 amino acids, 180 amino acids, 200 amino acids, 250 amino acids, 300 amino acids, 350 amino acids, 400 amino acids, 450 amino acids, or 500 amino acids in length. A HAP sequence is capable of extending half-life of a moiety fused to or linked to the HAP sequence. Non-limiting examples of the HAP sequence includes, but are not limited to (Gly)ₙ, (Gly₄Ser)ₙ or S(Gly₄Ser)ₙ, wherein n is 1, 2, 3, 4, 5, 6, 7, 8, 9, 10, 11, 12, 13, 14, 15, 16, 17, 18, 19, or 20. In one embodiment, n is 20, 21, 22, 23, 24, 25, 26, 27, 28, 29, 30, 31, 32, 33, 34, 35, 36, 37, 38, 39, or 40. In another embodiment, n is 50, 60, 70, 80, 90, 100, 110, 120, 130, 140, 150, 160, 170, 180, 190, or 200. *See, e.g.,* Schlapschy M et al., Protein Eng. Design Selection, 20: 273-284 (2007).

### II.B.7 Transferrin

In certain aspects, a chimeric protein used in the methods of the present disclosure comprises at least one transferrin peptide or fragment, variant, or derivative thereof. Any transferrin can be fused with the chimeric protein used in the methods of the present disclosure. As an example, wild-type human Tf (Tf) is a 679 amino acid protein, of approximately 75 kDa (not accounting for glycosylation), with two main domains, N (about 330 amino acids) and C (about 340 amino acids), which appear to originate from a gene duplication. *See* GenBank accession numbers NM001063, XM002793, M12530, XM039845, XM 039847 and S95936 (www.ncbi.nlm.nih.gov).

Transferrin transports iron through transferrin receptor (TfR)-mediated endocytosis. After the iron is released into an endosomal compartment and Tf-TfR complex is recycled to cell surface, the Tf is released back extracellular space for next cycle of iron transporting. Tf possesses a long half-life that is in excess of 14-17 days (Li et al., Trends Pharmacol. Sci. 23:206-209 (2002)). Transferrin fusion proteins have been studied for half-life extension, targeted deliver for cancer therapies, oral delivery and sustained activation of proinsulin (Brandsma et al., Biotechnol. Adv., 29: 230-238 (2011); Bai et al., Proc. Natl. Acad. Sci. USA 102:7292-7296 (2005); Kim et al., J. Pharmacol. Exp. Ther., 334:682-692 (2010); Wang et al., J. Controlled Release 155:386-392 (2011)).

### II.B.8. PEG

In certain aspects, a chimeric protein used in the methods of the present disclosure comprises at least one attachment site for a non-polypeptide heterologous moiety or fragment, variant, or derivative thereof. For example, a chimeric protein used in the methods of the present disclosure can include one or more polyethylene glycol (PEG) moieties attached to one or more amino acid residues in the clotting factor and/or the Fc region.

PEGylation of a protein can refer to a conjugate formed between the protein and at least one polyethylene glycol (PEG) molecule. PEG is commercially available in a large variety of molecular weights and average molecular weight ranges. Typical examples of PEG average molecular weight ranges include, but are not limited to, about 200, about 300, about 400, about 600, about 1000, about 1300-1600, about 1450, about 2000, about 3000, about 3000-3750, about 3350, about 3000-7000, about 3500-4500, about 5000-7000, about 7000-9000, about 8000, about 10000, about 8500-11500, about 16000-24000, about 35000, about 40000, about 60000, and about 80000 Daltons. These average molecular weights are provided merely as examples and are not meant to be limiting in any way.

A chimeric protein used in the methods of the present disclosure can be PEGylated to include mono- or poly-(*e.g*., 2-4) PEG moieties. PEGylation can be carried out by any of the PEGylation reactions known in the art. Methods for preparing a PEGylated protein product will generally include (i) reacting a polypeptide with polyethylene glycol (such as a reactive ester or aldehyde derivative of PEG) under conditions whereby the peptide of the invention becomes attached to one or more PEG groups; and (ii) obtaining the reaction product(s). In general, the optimal reaction conditions for the reactions will be determined case by case based on known parameters and the desired result.

There are a number of PEG attachment methods available to those skilled in the art, for example Malik F et al., Exp. Hematol. 20:1028-35 (1992); Francis, Focus on Growth Factors 3(2):4-10 (1992); European Pat. Pub. Nos. EP0401384, EP0154316, and EP0401384; and International Pat. Appl. Pub. Nos. WO92/16221 and WO95/34326. As a non-limiting example, FVIII variants can contain cysteine substitutions, and the cysteines can be further conjugated to PEG polymer. See Mei et al., Blood 116:270-279 (2010) and U.S. Patent No. 7,632,921.

### II.B.9. HES

In certain aspects, a chimeric protein used in the methods of the present disclosure comprises at least one hydroxyethyl starch (HES) polymer. HES is a derivative of naturally occurring amylopectin and is degraded by alpha-amylase in the body. HES exhibits advantageous biological properties and is used as a blood volume replacement agent and in hemodilution therapy in the clinics. *See, e.g.,* Sommermeyer et al., Krankenhauspharmazie 8:271-278 (1987); and Weidler et al., Arzneim.-Forschung/Drug Res. 41: 494-498 (1991).

HES is mainly characterized by the molecular weight distribution and the degree of substitution. HES has a mean molecular weight (weight mean) of from 1 to 300 kD, from 2 to 200kD, from 3 to 100 kD, or from 4 to 70kD. Hydroxyethyl starch can further exhibit a molar degree of substitution of from 0.1 to 3, from 0.1 to 2, from 0.1 to 0.9, or from 0.1 to 0.8, and a ratio between C2:C6 substitution in the range of from 2 to 20 with respect to the hydroxyethyl groups. HES with a mean molecular weight of about 130 kD is VOLUVEN^{®} from Fresenius. VOLUVEN^{®} is an artificial colloid, employed, *e.g.,* for volume replacement used in the therapeutic indication for therapy and prophylaxis of hypovolaemia. There are a number of HES attachment methods available to those skilled in the art, *e.g.,* the same PEG attachment methods described above.

### II.B.10. PSA

In certain aspects, a chimeric protein used in the methods of the present disclosure comprises at least one polysialic acid (PSA) polymer. PSAs are naturally occurring unbranched polymers of sialic acid produced by certain bacterial strains and in mammals in certain cells. See, *e.g.,* Roth J. et al. (1993) in Polysialic Acid: From Microbes to Man, eds. Roth J., Rutishauser U., Troy F. A. (BirkhäuserVerlag, Basel, Switzerland), pp. 335-348. PSAs can be produced in various degrees of polymerization from n=about 80 or more sialic acid residues down to n=2 by limited acid hydrolysis or by digestion with neuraminidases, or by fractionation of the natural, bacterially derived forms of the polymer. There are a number of PSA attachment methods available to those skilled in the art, *e.g.,* the same PEG attachment methods described above. In certain aspects, an activated PSA can also be attached to a cysteine amino acid residue within the clotting factor, *e.g.,* on FVIII or FIX, or within the Fc region. *See, e.g.,* U.S. Patent No. 5846951.

### II.B.11. Clearance Receptors

In certain aspects, the half-life of a chimeric protein used in the methods of the present disclosure can be extended where the clotting factor of the chimeric protein comprises FVIII and at least one fragment of a FVIII clearance receptor or FVIII-binding fragment, variant, or derivative thereof. Insertion of soluble forms of clearance receptors, such as the low density lipoprotein-related protein receptor LRP1, or fragments thereof, can block binding of FVIII to clearance receptors and thereby extend its half-life, *e.g., in vivo* half-life. LRP1 is a 600 kDa integral membrane protein that is implicated in the receptor-mediate clearance of a variety of proteins, including FVIII. *See, e.g.,* Lenting et al., Haemophilia 16:6-16 (2010). Other suitable FVIII clearance receptors are, *e.g.,* LDLR (low-density lipoprotein receptor), VLDLR (very low-density lipoprotein receptor), and megalin (LRP-2), or fragments thereof. *See, e.g.,* Bovenschen et al., Blood 106:906-912 (2005); Bovenschen, Blood 116:5439-5440 (2010); Martinelli et al., Blood 116:5688-5697 (2010).

### III. POLYNUCLEOTIDES, VECTORS, AND HOST CELLS

The present disclosure provides a polynucleotide or a set of polynucleotides encoding the chimeric protein comprising the clotting factor and the Fc region. In some embodiments, the polynucleotide or the set of polynucleotides is within an expression vector or a set of expression vectors. In certain embodiments, the expression vector or the set of expression vectors is within one or more host cells.

The polynucleotide encoding the chimeric protein comprising the clotting factor and the Fc region can be a single nucleotide sequence, two nucleotide sequences, three nucleotide sequences, or more. In one embodiment, a single nucleotide sequence encodes a chimeric protein comprising the clotting factor FVIII and the Fc region. In another embodiment, the polynucleotide comprises two nucleotide sequences, the first nucleotide sequence encoding the clotting factor FVIII and the second nucleotide sequence encoding the Fc region. In another embodiment, the polynucleotide comprises two nucleotide sequences, the first nucleotide sequence encoding the clotting factor FVIII and the Fc region and the second nucleotide sequence encoding a second Fc region. In certain embodiments, the encoded Fc domains form a covalent bond after expression.

In some embodiments, the polynucleotide is codon-optimized.

As used herein, an expression vector refers to any nucleic acid construct which contains the necessary elements for the transcription and translation of an inserted coding sequence, or in the case of an RNA viral vector, the necessary elements for replication and translation, when introduced into an appropriate host cell. Expression vectors can include plasmids, phagemids, viruses, and derivatives thereof.

A gene expression control sequence as used herein is any regulatory nucleotide sequence, such as a promoter sequence or promoter-enhancer combination, which facilitates the efficient transcription and translation of the coding nucleic acid to which it is operably linked. The gene expression control sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, beta-actin promoter, and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus, and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

For the purposes of this invention, numerous expression vector systems can be employed. These expression vectors are typically replicable in the host organisms either as episomes or as an integral part of the host chromosomal DNA. Expression vectors can include expression control sequences including, but not limited to, promoters (*e.g*., naturally-associated or heterologous promoters), enhancers, signal sequences, splice signals, enhancer elements, and transcription termination sequences. Preferably, the expression control sequences are eukaryotic promoter systems in vectors capable of transforming or transfecting eukaryotic host cells. Expression vectors can also utilize DNA elements which are derived from animal viruses such as bovine papilloma virus, polyoma virus, adenovirus, vaccinia virus, baculovirus, retroviruses (RSV, MMTV or MOMLV), cytomegalovirus (CMV), or SV40 virus. Others involve the use of polycistronic systems with internal ribosome binding sites.

Commonly, expression vectors contain selection markers (*e.g*., ampicillin-resistance, hygromycin-resistance, tetracycline resistance or neomycin resistance) to permit detection of those cells transformed with the desired DNA sequences (*see*, *e.g.*, Itakura et al., US Patent No. 4,704,362). Cells which have integrated the DNA into their chromosomes can be selected by introducing one or more markers which allow selection of transfected host cells. The marker can provide for prototrophy to an auxotrophic host, biocide resistance (*e.g*., antibiotics) or resistance to heavy metals such as copper. The selectable marker gene can either be directly linked to the DNA sequences to be expressed, or introduced into the same cell by cotransformation.

An example of a vector useful for optimized expression of the chimeric proteins used in the methods of the present disclosure is NEOSPLA (U.S. Patent No. 6,159,730). This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene and leader sequence. This vector has been found to result in very high level expression of antibodies upon incorporation of variable and constant region genes, transfection in cells, followed by selection in G418 containing medium and methotrexate amplification. Vector systems are also taught in US Patent Nos. 5,736,137 and 5,658,570. This system provides for high expression levels, *e.g.,* > 30 pg/cell/day. Other exemplary vector systems are disclosed *e.g.,* in US Patent No. 6,413,777.

In other embodiments the polypeptides are expressed using polycistronic constructs. In these expression systems, multiple gene products of interest such as multiple polypeptides of multimer binding protein can be produced from a single polycistronic construct. These systems advantageously use an internal ribosome entry site (IRES) to provide relatively high levels of polypeptides in eukaryotic host cells. Compatible IRES sequences are disclosed in US Patent No. 6,193,980.

More generally, once the vector or DNA sequence encoding a polypeptide has been prepared, the expression vector can be introduced into an appropriate host cell. That is, the host cells can be transformed. Introduction of the plasmid into the host cell can be accomplished by various techniques well known to those of skill in the art, as discussed above. The transformed cells are grown under conditions appropriate for the production of the chimeric protein, and assayed for chimeric protein synthesis. Exemplary assay techniques include enzyme-linked immunosorbent assay (ELISA), radioimmunoassay (RIA), or fluorescence-activated cell sorter analysis (FACS), immunohistochemistry, and the like.

Having now described the present invention in detail, the same will be more clearly understood by reference to the following examples, which are included herewith for purposes of illustration only and are not intended to be limiting of the invention.

### EXAMPLE 1

### Long-term efficacy of rFVIIIFc prophylaxis in pediatric, adolescent, and adult subjects with target joints and severe hemophilia A

For people with hemophilia, frequent bleeding into the same joint (a target joint) may contribute to hemophilic arthropathy (chronic joint disease). rFVIIIFc was developed to extend the half-life of factor VIII (FVIII) as compared to conventional FVIII products (Peters et al., J. Thromb. Haemost. 11(1):132-41 (2013)). The completed a rFVIIIFc pivotal phase 3 trial (ClinicalTrials.gov Identifier: NCT01181128; Mahlangu, et al., Blood 123(3):317-25 (2014)) and Kids rFVIIIFc pivotal phase 3 trial (NCT01458106; Young et al., J. Thromb. Haemost. 13(6):967-77 (2015)) studies established the safety and efficacy of rFVIIIFc among adults/adolescents (patients 12-years-old or older) and children (patients less than 12 years old) with severe hemophilia A, respectively. Long-term safety and efficacy of rFVIIIFc are being evaluated in the ongoing rFVIIIFc extension study extension study (NCT01454739; Nolan et al., Haemophilia 22(1):72-80(2016)).

The objective of this study is to report cumulative data on the durable efficacy of rFVIIIFc and QOL in subjects with target joints at entry into rFVIIIFc pivotal phase 3 trial and Kids rFVIIIFc pivotal phase 3 trial, as of the second rFVIIIFc extension study interim data cut (December 8, 2014).

### Methods

Subjects with ≥1 target joint (major joint with ≥ 3 bleeding episodes in a 6-month period (*see* World Federation of Hemophilia, Guidelines for the Management of Hemophilia 2nd ed., Blackwell Publishing: Montréal, Canada (2012))) at entry into the parent study (*i.e.,* rFVIIIFc pivotal phase 3 trial or Kids rFVIIIFc pivotal phase 3 trial) who had available prestudy (*i.e*., pre-parent study) and on-study data (target joint-specific and overall) were evaluated. There are 4 treatment groups in rFVIIIFc extension study (Table 1).

**TABLE 1: rFVIIIFc Extension Study Treatment Regimens**

| **Treatment Regimen** | **Dosing Guidance per Protocol** |
|---|---|
| Individualized Prophylaxis | rFVIIIFc 25-65 IU/kg every 3-5 days OR twice-weekly rFVIIIFc (20-65 IU/kg on Day 1, 40-65 IU/kg on Day 4); pediatric subjects are allowed adjustments to dose and frequency (every 2-5 days) |
| Weekly Prophylaxis | rFVIIIFc 65 IU/kg every 7 days |
| Modified Prophylaxis | Investigators could personalize dosing for subjects in whom optimal prophylaxis could not be achieved with individualized or weekly prophylaxis |
| Episodic Treatment | rFVIIIFc dosing based on type and severity of bleeding episode |

| | |
|---|---|
| Subjects ≥12 years of age could participate in any treatment regimen in the rFVIIIFc extension study, while subjects <12 years of age could participate only in the individualized prophylaxis and modified prophylaxis treatment regimens. | |

Outcomes for subjects with target joints were analyzed post hoc over the cumulative duration of the parent study through the second rFVIIIFc extension study interim data cut. Outcomes included ABRs, number and resolution of target joint bleeding episodes, and prophylactic dose and dosing frequency. Analysis of target joint resolution was performed for subjects who had ≥ 12 months of consecutive follow-up time and who had not undergone major surgery (*i.e.*, replacement or removal) at the target joint since the start of the follow-up period. A target joint was considered clinically resolved if there were ≤ 2 spontaneous bleeding episodes in the target joint during a consecutive 12-month period (Blanchette et al., J Thromb Haemost. 12(11):1935-39 (2014)).

QOL measures were assessed by the Haem-A-QOL index in prophylaxis subjects who were ≥17 years of age, had ≥1 resolved target joint during the study, and had Haem-A-QOL scores at both rFVIIIFc pivotal phase 3 trial baseline and rFVIIIFc extension study Year 2. Among subjects from Kids rFVIIIFc pivotal phase 3 trial, QOL was measured by the Canadian Hemophilia Outcomes-Kids Life Assessment Tool (CHO-KLAT).

### Results

### Study Population

Of 113 subjects from rFVIIIFc pivotal phase 3 trial who had target joints at baseline, 111 subjects with prestudy prophylactic or episodic regimens and on-study data had 287 target joints at baseline (median age, 31.0 years; interquartile range (IQR), 24.0-44.0 years; Table 2). Thirteen subjects from Kids rFVIIIFc pivotal phase 3 trial had 15 target joints at baseline and had prestudy and on-study data (median age, 6.0 years; IQR, 5.0-8.0 years; Table 2).

**TABLE 2: Demographic and baseline characteristics^{a}**

| | | Parent Study | |
|---|---|---|---|
| Characteristic | | rFVIIIFc pivotal phase 3 trial n = 111 | Kids rFVIIIFc pivotal phase 3 trial n = 13 |
| Median (IQR) age, years | | 31.0 (24.0-44.0) | 6.0 (5.0-8.0) |

| Prestudy regimen | | | |
|---|---|---|---|
| | Episodic | 65 (58.6) | 3 (23.1) |
| | Prophylaxis | 46 (41.4) | 10 (76.9) |
| Total number of target joints, n | | 287 | 15 |

| Number of target joints, n (%)^{b} | | | |
|---|---|---|---|
| | 1 | 42 (37.8) | 11 (84.6) |
| | 2 | 27 (24.3) | 2 (15.4) |
| | 3 | 7 (6.3) | 0 |
| | >3 | 35 (31.5)^{c} | 0 |

| Target joint location, n (%) | | | |
|---|---|---|---|
| | Elbow | 69 (62.2) | 4 (30.8) |
| | Ankle | 65 (58.6) | 9 (69.2) |
| | Knee | 48 (43.2) | 1 (7.7) |
| | Shoulder | 14 (12.6) | 0 |
| | Wrist | 6 (5.4) | 0 |
| | Hip | 5 (4.5) | 0 |

| | | | |
|---|---|---|---|
| IQR - interquartile range. ^{a} Includes subjects with ≥ 1 target joint at entry into the parent study and with an efficiency period. A target joint is defined as a major joint (*e.g*., elbow, ankle, knee, shoulder, wrist, and hip) into which repeated bleading occurred (frequency ≥3 bleeding episodes into the same joint in a consecutive 6-month period). ^{b} Percentages may not total 100.0% due to rounding. ^{c} Mean age of subjects was 39.9 years. | | | |

### Bleeding Rates

Median (IQR) on-study overall ABRs with rFVIIIFc prophylaxis were lower than bleeding rates with prestudy prophylaxis for adults/adolescents and children, 12 years old or younger (FIGs. 1A-1D). Kids rFVIIIFc pivotal phase 3 trial data was further stratified by patient age, and median prestudy and on-study ABRs are shown in FIGs. 1E and 1F. Prestudy, patients less than 6 years old had a lower median (IQR) ABR than patients aged 6 to <12 years old (FG. 1E). On study, Patienst less than 6 years old had higher overall on-study ABR, overall target joint ABR, and target joinr spontaneous ABR than patients aged 6 to <12 years old (FG. 1F).

In rFVIIIFc pivotal phase 3 trial, 46.3% of individualized prophylaxis, 40.7% of weekly prophylaxis, and 21.4% of modified prophylaxis subjects had no target joint bleeding episodes, whereas 53.8% of individualized prophylaxis subjects in Kids rFVIIIFc pivotal phase 3 trial had no target joint bleeding episodes.

### Clinical Target Joint Resolution

Among subjects on prophylaxis who had target joints at baseline and 12 months of follow-up, 100% (93/93) of rFVIIIFc pivotal phase 3 trial and 100% (7/7) of Kids rFVIIIFc pivotal phase 3 trial subjects had ≥ 1 target joint resolved (*i.e.,* ≤ 2 spontaneous bleeding episodes during 12 consecutive months); and 98.3% (231/235) and 100% (9/9) of target joints (based on all bleeds) in rFVIIIFc pivotal phase 3 trial and Kids rFVIIIFc pivotal phase 3 trial subjects were resolved, respectively.

### Prophylactic Factor Consumption

The median (IQR) weekly prophylactic factor consumption among rFVIIIFc pivotal phase 3 trial (n = 105) and Kids rFVIIIFc pivotal phase 3 trial (n = 13) prophylaxis subjects with target joints at baseline was 76.0 (68.0-90.9) IU/kg and 83.5 (79.9-111.6) IU/kg, respectively.

Consumption was similar to subjects in the parent studies who were on prophylaxis prior to both rFVIIIFc pivotal phase 3 trial /Kids rFVIIIFc pivotal phase 3 trial and during rFVIIIFc pivotal phase 3 trial /Kids rFVIIIFc pivotal phase 3 trial and the rFVIIIFc extension study and had available prestudy and on-study dosing data (rFVIIIFc pivotal phase 3 trial, n = 79, 75.0 [70.0-113.8] IU/kg; Kids rFVIIIFc pivotal phase 3 trial, n = 54, 95.0 [75.0-113.0] IU/kg).

For both patient populations, median (IQR) dosing intervals were also similar between prophylaxis subjects with target joints at baseline (rFVIIIFc pivotal phase 3 trial, n = 105, 3.8 [3.5-5.6] days; Kids rFVIIIFc pivotal phase 3 trial, n = 13, 3.5 [3.5-3.5] days) and prophylaxis subjects in the parent study who had available prestudy and on-study dosing data (rFVIIIFc pivotal phase 3 trial, n = 79, 3.5 [3.0-5.0] days; Kids rFVIIIFc pivotal phase 3 trial, n = 54, 3.5 [3.5-3.5] days).

Kids rFVIIIFc pivotal phase 3 trial data was further stratified by patient age. Average weekly consumption in patients under the age of 6 was 89.6 (75.3-97.5; n = 6) IU/kg at a dosing interval of 3.5 (3.5-3.5) days. For patients aged 6 to less than 12 years old, average weekly consumption was 82.2 (79.4-113.2) IU/kg at a dosing interval of 3.5 (3.0-3.6) days.

### Quality of Life

QOL improved by 18% at rFVIIIFc extension study Year 2 among adults/adolescents (n = 48) compared with rFVIIIFc pivotal phase 3 trial baseline (Table 3). Among Kids rFVIIIFc pivotal phase 3 trial subjects who had self-reported CHO-KLAT scores at Kids rFVIIIFc pivotal phase 3 trial baseline and rFVIIIFc extension study Year 1 (n = 6), the mean (standard deviation [SD]) baseline score was 85.5 (12.1); at rFVIIIFc extension study Year 1, CHO-KLAT scores improved by 28% (mean [SD] improvement of 24.1 [15.3]).

**TABLE 3: Haem-A-QOL analysis in subjects with target joints at baseline**

| | Mean (SD) Score^{a} at rFVIIIFc Pivotal Phase 3 Trial Baseline N= 30-48^{c} | Mean (SD) Score^{a} at rFVIIIFc Extension Study Month 24 N= 30-48^{c} | Mean (SD) Change | *P* value^{b} |
|---|---|---|---|---|
| Total | 30.3 (15.7) | 24.9 (15.7) | -5.4 (10.8) | 0.001 |
| Sports & Leisure | 51.5 (26.9) | 40.8 (27.6) | -10.7 (21.7) | 0.008 |
| Physical health | 42.7 (21.7) | 32.1 (27.8) | -10.6 (19.8) | 0.0005 |
| Dealing with hemophilia | 17.0 (13.6) | 15.3 (15.9) | -1.7 (17.5) | NS |
| Family planning | 15.5 (19.8) | 11.5 (18.3) | -4.0 (16.3) | NS |
| Feeling (toward hemophilia) | 23.0 (21.8) | 15.4 (19.2) | -7.7 (16.0) | 0.002 |
| Future | 38.9 (20.9) | 33.8 (23.1) | -5.1 (15.4) | 0.03 |
| Partnership & sexuality | 12.6 (20.2) | 12.2 (21.4) | -0.4 (20.6) | NS |
| Treatment | 28.3 (16.6) | 23.3 (14.1) | -5.0 (15.7) | 0.03 |
| View (of yourself) | 34.2 (22.7) | 32.1 (21.1) | -2.1 (16.8) | NS |
| Work & school | 19.6 (20.0) | 13.7 (19.1) | -6.0 (12.1) | 0.002 |

| | | | | |
|---|---|---|---|---|
| NS, not significant ^{a} Haem-A-QOL scores range from 0 to 100, with higher scores representing a higher impairment in QOL for each subscore and the total score. ^{b} Based on 2-tailed paired t test. ^{c} 48 patients were evaluable for all outcomes other than sports & leisure ( n = 33), family planning (n = 30), partnership & sexuality (n = 45), and work & school (n = 44). | | | | |

### Conclusions

Efficacy data from the Phase 3 rFVIIIFc pivotal phase 3 trial and Kids rFVIIIFc pivotal phase 3 trial studies and the ongoing rFVIIIFc extension study show sustained low target joint annualized bleeding rates (ABRs) and effective target joint resolution in pediatric, adolescent, and adult subjects with severe hemophilia A on long-term rFVIIIFc prophylaxis. Weekly prophylactic factor consumption in this analysis of subjects with target joints at baseline was consistent with that in the overall populations of the previously published rFVIIIFc pivotal phase 3 trial and Kids rFVIIIFc pivotal phase 3 trial studies. Improvement in quality of life (QOL) was seen in subjects with target joint resolution with rFVIIIFc prophylaxis without changes in prophylactic factor consumption or dosing interval.

### EXAMPLE 2

### Longitudinal Modified Hemophilia Joint Health Scores (mHJHS) Outcomes With Recombinant Factor VIII Fc Fusion Protein (rFVIIIFc) Prophylaxis in Subjects With Severe Hemophilia A

Hemophilic arthropathy remains a challenge in the management of haemophilia (Knobe et al., J Comorbidity. 2011;1(1):51-59; Simpson et al., Expert Rev Hematol. 2012;5(4):459-68). Hemophilia Joint Health Score (HJHS) is a first-line tool that can be used to detect the development of hemophilic arthropathy (Oymak et al. J Pediatr Hematol Oncol. 2015;37(2):e80-5). Improvement in musculoskeletal outcomes is an important measure of the effectiveness of prophylaxis treatment for hemophilia A (Blanchette et al. Haemophilia. 2004;10 (Suppl. S4):97-104). The long-term safety and efficacy of rFVIIIFc among adults/adolescents and children who have completed the rFVIIIFc pivotal phase 3 trial (Mahlangu et al. Blood. 2014;123(3):317-325) and Kids rFVIIIFc pivotal phase 3 trial studies (Young et al. J Thromb Haemost. 2015;13(6):967-977), respectively, have been established using interim data from the ongoing rFVIIIFc extension study (Nolan et al. Haemophilia. 2016;22(1):72-80). Determining the long-term impact of rFVIIIFc on musculoskeletal outcomes will require further study.

The objective of this study is to report longitudinal joint health data from rFVIIIFc pivotal phase 3 trial and rFVIIIFc extension study using the modified Hemophilia Joint Health Scores (mHJHS).

### Study Participants and Design

The analysis population included adults/adolescents (≥12 years) who completed rFVIIIFc pivotal phase 3 trial and enrolled in the rFVIIIFc extension study for 2 years. Patients may have been on pre-study prophylaxis or episodic treatment. Joint health was assessed using the mHJHS at the rFVIIIFc pivotal phase 3 trial screening (after protocol amendment) and at baseline, and then annually thereafter for the rFVIIIFc extension study.

The mHJHS differs from the standard HJHS, version 2.1, in that the response options for joint pain and gait were condensed into fewer categories, an assessment for instability was added, and the total score is lower (range, 0-116; 0 indicates normal joint function, 116 indicates severe disease) compared with the standard HJHS (range, 0-124) (*see* Table 4). Scores preceded by a bleed within 2 weeks were excluded. Scores for joints that underwent surgical interventional were imputed using last observation carry forward. To evaluate year-by-year change, rFVIIIFc extension study subjects who had mHJHS data at 4 time points (rFVIIIFc pivotal phase 3 trial baseline, rFVIIIFc extension study baseline, rFVIIIFc extension study Year 1, and rFVIIIFc extension study Year 2) were included in this post hoc analysis. Change in mHJHS score from rFVIIIFc pivotal phase 3 trial baseline to rFVIIIFc extension study Year 2 (negative value indicates improvement) was summarized using descriptive statistics. Change in mHJHS score from rFVIIIFc pivotal phase 3 trial baseline to follow-up visits was summarized for: (1) total score (range, 0-116; by pre-study regimen (prophylactic versus episodic); by severity of functional impairment based on initial mHJHS; and by presence of target joints at baseline; (2) target joints (range, 0-19: sum of all questions for a single target joint); (3) Weight bearing (*e.g*., ankle and knee) and non-weight bearing (*e.g*., elbow) joints (range, 0-38: sum of right and left joints of a single location); and (4) individual components (range of motion (range, 0-36: combination of questions "Extension loss [dorsiflexion of ankles]" and "Flexion loss [plantarflexion of ankles]" of all joints); swelling (range, 0-24: combination of questions "Swelling" and "Duration of swelling" of all joints); and strength (range, 0-6: sum of all joints)).

**TABLE 4 - Differences between HJHS and mHJHS**

| **HJHS** | **mHJHS** |
|---|---|
| Crepitus of motion (Scored 0-2) | Crepitus of motion (Scored 0 or 1) |
| Joint pain (Scored 0-2) | Joint pain (Scored 0 or 1) |
| Global gait (Scored 0-4) | Global Gait (Scored descriptively) |
| - | Instability (Scored 0 or 1) |
| Total score (0-124) | Total score (0-116^{a}) |

| | |
|---|---|
| ^{a} 0 = normal joint function; 116 = severe disease | |

Differences between rFVIIIFc pivotal phase 3 trial baseline and rFVIIIFc extension study Year 2 were analyzed using a paired t-test. *P* values were not used to infer statistical significance because this analysis was ad hoc. For subgroup analyses, only descriptive statistics are provided.

### Results

### Baseline Characteristics

Baseline characteristics were similar between the population of completers included in this analysis (n = 47) and the patient population for whom mHJHS was collected at rFVIIIFc pivotal phase 3 trial baseline and who enrolled in the rFVIIIFc extension study (n = 74) (Table 5).

**TABLE 5: Baseline characteristics**

| | | **rFVIIIFc Pivotal Phase 3 Trial Baseline and Enrolled in the rFVIIIFc Extension Study n=74^{a}** | **rFVIIIFc PIVOTAL PHASE 3 TRIAL and rFVIIIFc Extension Study 2-year Completer n = 47^{b}** |
|---|---|---|---|
| Mean (SD) age, y | | 31.6 (11.9) | 32.3 (12.8) |
| Mean (SD) weight at parent study entry, kg | | 72.7 (15.1) | 73.5 (15.7) |
| Mean (SD) BMI, kg/m² | | 23.8 (4.3) | 24.0 (4.1) |
| Mean (SD) baseline mHJHS score | | 22.1 (18.0) | 23.4 (18.3) |
| Target joints, % | | 55.4 | 51.1 |
| Pre-study treatment, % | | 68.9 | 63.8 |
| | Prophylaxis | 31.1 | 36.2 |
| | On-demand | | |
| Mean (SD) pre-study ABR | | 15.8 (20.0) | 16.5 (18.4) |

| | | | |
|---|---|---|---|
| ^{a} 74 subjects on rFVIIIFc prophylaxis enrolled in rFVIIIFc pivotal phase 3 trial, have mHJHS scores, and subsequently entered the rFVIIIFc extension study. ^{b} 47 patients on prophylaxis reached year 2 in the rFVIIIFc extension study with data available at rFVIIIFc pivotal phase 3 trial baseline, rFVIIIFc extension study baseline, and Years 1 and 2. | | | |

### Longitudinal Joint Health

Continuous improvement was observed in mHJHS score from rFVIIIFc pivotal phase 3 trial base line through rFVIIIFc extension study year 2, with an average decrease in total score from 23.4 to 19.3 (FIG. 2A). Of the seventy-four subject, twenty-four were evaluated at extension study year 3, and these improvements in mHJHS scores were seen at extension study year 3 regardless of the presence of target joints at baseline for the rFVIIIFc pivotal phase 3 trial (FIG. 2B). The average follow-up duration was 2.8 (2.5-3.3) years. Continuous improvement was observed regardless of the prestudy treatment of the patient (FIG. 3) or the presence of target joints at rFVIIIFc pivotal phase 3 trial baseline (FIG. 4). In those patients evaluated at extension study year 3, mean mHJHS total score for adults/adolescents with data at all time points was 25.0 (standard error of the mean [SEM], 2.9) at baseline. Mean (SEM) change from baseline was -2.0 (1.2) at extension study baseline, -3.8 (1.5) at extension study year 1, -4.5 (1.6) at extension study year 2, and -5.1 (1.5) at extension study year 3 (FIG. 3B). The change from baseline to extension study year 3 was statistically significant (P<0.002). The Kids rFVIIIFc pivotal phase 3 trial analysis population (n = 24) also showed statistically significant mean (SEM) improvement from baseline to extension study year 2 (-1.2 [0.56]; P<0.05) (FIG. 3C). Subjects with the highest quartile of impairment in mHJHS scores at rFVIIIFc pivotal phase 3 trial baseline showed the greatest improvement in mHJHS total score from baseline to rFVIIIFc extension study year 2 (FIG. 5). Continuous improvement in mHJHS target joint scores was observed from rFVIIIFc pivotal phase 3 trial baseline to rFVIIIFc extension study year 2, with an improvement from an average of 7.0 at baseline to an average of 5.3 at rFVIIIFc extension study year 2 (FIG. 6). mHJHS joint scores for weight bearing joints decreased from an average of 8.0 to an average of 6.8 and for non-weight bearing joints from an average of 6.7 to an average of 4.8 from rFVIIIFc pivotal phase 3 trial baseline to rFVIIIFc extension study year 2 (FIG. 7). For calculation of weight bearing (ankle & knee) and non-weight bearing joint (elbow) score, a score was first derived as the sum of per-joint scores for a pair of right and left joints (ankle, knee, or elbow). Then weight bearing joint score was calculated as the average of the scores for ankle and knee, and non-weight bearing joint score was the same as the score for elbow. In addition, specific improvements in swelling, range of motion, and strength were also observed from rFVIIIFc pivotal phase 3 trial baseline to rFVIIIFc extension study year 2, which represented the most significant contributing factors to change in mHJHS total score (FIG. 8).

Statistically significant mean improvements from the rFVIIIFc pivotal phase 3 trial baseline were observed for both weight-bearing and non-weight-bearing joints (-1.1 [SEM, 0.5; P=0.036] and -3.0 [SEM, 0.8; P=0.001], respectively) at year 3. Individual components of the mHJHS that showed ≥20% reduction from baseline to year 3 were swelling (-47%), muscle atrophy (-26%), crepitus (-20%) (P<0.05 for all 3 components) and joint instability (-89%), joint pain (-31%), and strength (-26%) (FIG. 9).

### EXAMPLE 3 (not according to the invention)

### Target Joint Outcomes with Prophylaxis with rFIXFc in Adults and Adolescents with Hemophilia B

In patients with severe hemophilia B, repeated bleeding into joints without sufficient treatment may lead to crippling chronic joint disease, pain, and reduced quality of life (Djambas Khayat, *J Blood Med. 7*:275-82 (2016)). Prophylactic treatment with recombinant factor IX Fc fusion protein (rFIXFc) resulted in lower annualized bleeding rates (ABRs) and fewer spontaneous/traumatic bleeding events in adolescents and adults with severe hemophilia B (Kavakli et al., Haemophilia 22(3):381-88 (2016); Powell et al. N Engl J Med. 369(24):2313-23 (2013); Powell et al., Br J Haematol. 168(1):124-34 (2015); Powell et al., Br J Haematol. 168(1):113-23 2015)). In addition to prevention of joint damage and reduction in bleeding events, prophylactic treatment with both conventional and long-acting rFIX may result in less time out of work or school, fewer hospital admissions, less frequent monitoring, and improved quality of life (Kavakli et al., Haemophilia 22(3):381-88 (2016); Wyrwich et al., Haemophilia 22(6):866-72 (2016)). Prophylactic treatment initiated early in life reduces bleeding and prevents joint damage. When initiated later in life once joint damage has transpired, prophylactic treatment still can significantly reduce the number of bleeds, including bleeds into joints (Fischer et al., Haemophilia 20(Suppl 4):106-13 (2014)). Adults/adolescents with severe hemophilia B who completed the rFIXFc pivotal phase 3 trial (NCT01027364) could enroll in the long-term extension study (NCT01425723; Pasi et al., Thromb Haemost. 117(3):508-18 (2017)) that evaluated the safety and efficacy of rFIXFc. Longitudinal outcomes from subjects with target joints at entry into rFIXFc pivotal phase 3 trial throughout the long-term extension study are reported here.

The objective of this study is to present rFIXFc pivotal phase 3 trial longitudinal data outcomes from subjects with target joints at entry through the second interim data cut of the extension study (September 11, 2015).

### Methods

### Study Design and Population

Subjects with severe hemophilia B (≤2 IU/dL endogenous FIX) completing the rFIXFc pivotal phase 3 trial enrolled in 1 of 4 treatment groups in the extension study: (1) weekly prophylaxis (WP; 20-100 IU/kg every 7 days); (2) individualized interval prophylaxis (IP; 100 IU/kg every 8-16 days); (3) modified prophylaxis (MP; investigators could personalize dosing for subjects not achieving optimal dosing with IP or WP); and (4) episodic treatment (ET; on-demand dosing based on type and severity of bleeding episodes). Subjects could switch their treatment group at enrollment into the extension study and at any time during the study. Subjects who switched treatment groups were included in the analyses of each treatment group for the period spent on that treatment regimen, so individual subjects could be counted in more than 1 treatment group in the analysis. Subjects with ≥1 target joint (major joint with ≥3 bleeding episodes in a 3-month period) at pivotal phase 3 trial entry were evaluated.

### Outcome Measures and Statistical Analyses

Outcomes were analyzed over the cumulative duration of the rFIXFc pivotal phase 3 trial through the second B-YOND interim data cut (September 11, 2015). An analysis of target joint resolution was performed. Target joint resolution was defined as ≤2 spontaneous bleeds in the target joint over a consecutive 12-month period (Blanchette et al., J Thromb Haemost. 12(11):1935-39 (2014)).

### Results

Baseline characteristics for subjects with target joints are shown in Table 6. Of 117 rFIXFc pivotal phase 3 trial subjects with on-study data, sixty had a total of 166 target joints at baseline. These subjects received rFIXFc for a cumulative median (interquartile range [IQR]) duration of 3.4 (1.4-4.2) years.

**Table 6: Baseline characteristics.**

| **Characteristic** | | **N = 60** |
|---|---|---|
| Prestudy regimen | | |
| | Episodic | 44 (73.3) |
| | Prophylaxis | 16 (26.7) |

| Number of target joint(s) | | |
|---|---|---|
| | 1 | 20 (33.3) |
| | 2 | 13 (21.7) |
| | 3 | 7 (11.7) |
| | >3 | 20 (33.3) |

| Target joint location | | |
|---|---|---|
| | Knee | 42 (70.0) |
| | Ankle | 33 (55.0) |
| | Elbow | 28 (46.7) |
| | Hip | 6 (10.0) |
| | Shoulder | 3 (5.0) |
| | Wrist | 3 (5.0) |

All data are n (%). rFIXFc, recombinant factor IX Fc fusion protein. Includes subjects with ≥1 target joint at entry into B-LONG and with an efficacy period (defined as the sum of all intervals of time during which subjects were treated with rFIXFc according to treatment regimens of the study, excluding surgical rehabilitation periods); bA target joint is defined as a major joint (eg, knee, ankle, elbow, hip, shoulder, and wrist) into which repeated bleeding occurred (frequency of ≥3 bleeding episodes into the same joint in a consecutive 3-month period).

### Prophylactic Dosing

Average weekly dosing and dosing interval summarized in Table 7.

**Table 7: Summary of average rFIXFc prophylactic dosing and dosing interval**

| **Treatment Group** | **WP (n = 40)** | **IP (n = 12)** | **MP (n = 12)** |
|---|---|---|---|
| Average weekly dose, IU/kg | 45.2 (37.3-55.8) | 64.7 (46.7-82.3) | 59.7 (40.0-109.8) |
| Dosing interval, d | 6.98 (6.9-7.0) | 10.25 (8.9-13.2) | 6.57 (4.9-6.9) |

All data are median (IQR). IP, Individualized interval prophylaxis; MP, modified prophylaxis; rFIXFc, recombinant factor IX Fc fusion protein; WP, weekly prophylaxis. Only subjects with available on-study dose and dosing interval data were included in this analysis. Subjects are included in each treatment regimen they participated in for the duration of time on that regimen and, as such, may appear in more than one treatment regimen.

### Bleeding Rates

Prestudy and on-study bleeding data are shown in FIGs. 10A and 10B, respectively. Subjects receiving rFIXFc prophylaxis who did not have a target joint rebleed on-study were as follows: WP: 15 of 40 (37.5%); IP: 1 of 12 (8.3%); MP: 4 of 12 (33.3%); ET: 0 of 14 (0%). In subjects with target joints at baseline, on-study overall and target joint ABRs with rFIXFc prophylaxis were lower than bleeding rates with prestudy treatment (FIGs. 10A and 10B).

| Annualized bleeding rates for twenty-two subjects treated with a dosing interval ≥14 days, as well as sixteen subjects who ended treatment on a dosing interval ≥14 days are shown in Table 8. | | |
|---|---|---|
| Overall ABR | 1.7 (0.6-4.2) | 1.4 (0.6-2.0) |
| Spontaneous ABR | 0.7 (0.3-1.3) | 0.7 (0.3-1.0) |
| Joint ABR | 1.1 (0.3-2.7) | 0.6 (0.2-1.5) |
| Spontaneous Joint ABR | 0.3 (0.0-1.3) | 0.3 (0.0-0.8) |

### Target Joint Resolution

Overall, 100% (93/93) of target joints (in 37 subjects) resolved, as indicated two or less spontaneous bleeds in a twelve-month period (FIG. 11)

### Conclusions

In adults/adolescents with severe hemophilia B, long-term rFIXFc prophylaxis resulted in target joint resolution in 100% of subjects with evaluable target joints at baseline and in low target joint ABRs across all treatment groups. Clinicians should consider the beneficial and significantly improved long-term outcomes for patients with target joints achieved with rFIXFc when designing treatment prophylaxis plans.

### EXAMPLE 4 (not according to the invention)

### Biodistribution of ¹²⁵I-labeled FIXFc, FIX, and GlycoPEGylated FIX in HemB mice by in vivo Single Photon Emission Tomography (SPECT)

An *in vivo* mouse study was conducted to evaluate biodistribution of FIX proteins at multiple times post-dosing by *in vivo* Single Photon Emission Tomography (SPECT). FIX proteins were labeled at lysine residues using a ¹²⁵I-labeled SIB linker. Labeled FIX proteins were administered to 7-12 week old HemB mice by a single therapeutically relevant dose. ¹²⁵I-SIB-FIX was administered as a single dose of 1 mg/kg (n = 3); ¹²⁵I-SIB-FIXFc was administered as a single dose of 2 mg/kg (n = 3), and ¹²⁵I-SIB-FIX-PEG was administered as a single dose of 1 mg/kg (n = 3).

Localization of the labeled FIX proteins was imaged at 0.5 hrs, 2.5 hrs, 20 hrs, 48hrs, 92 hrs, 120 hrs, 168 hrs, and/or 216 hrs (FIGs. 10A-10C). Specific localization to joint areas (*e.g.*, right/left knee and right/left shoulder), the heart (left ventricle), and the liver was analyzed using region of interest (ROI) placement. Left Ventricle and Liver ROI were fixed volume objects, placed within the correct organ. Knee-joint ROI was defined by placing a cylinder around the bone from approximately half way down the femur to half way down the tibia/fibula. Shoulder ROI was defined by placing a uniform sphere at the center of the shoulder, then thresholding for bone within that sphere, followed by a dilation of the ROI.

Labeled FIXFc was found to distribute to areas around the joints at 30 minutes (FIG. 12D) and 2.5 hrs (FIG. 12E) post-dosing, which persisted for 48-216 hrs post-dosing (FIGs. 12F-12G), representing the half-life and five-times the half-life, respectively. FIXFc localization to the knees (FIG. 13A) and the shoulders (FIG. 13B) was more pronounced than localization of FIX and GlycoPEG-FIX to the same joint areas.

¹²⁵I-SIB labeling did not affect FIX activity of the labeled FIX proteins (FIG. 14A), nor did labeling affect the pharmacokinetic properties of the FIX proteins, as compared to unlabeled FIX proteins, following administration to HemB mice (FIG. 14B).

### EXAMPLE 5

### Baseline Joint Assessment

### Baseline Joint Assessment

Six joints (left ankle-LA, right ankle-RA, left elbow-LE, right elbow-RE, left knee-LK, right knee-RK) will be scored on a scale from 0 to 19 according to the following criteria: swelling, duration, muscle atrophy, crepitus, flexion loss, extension loss, instability, joint pain, and strength. Gait will be scored on a scale from 0 to 2 based on walking and climbing stairs. The total score will be the sum of scores from all 6 joints plus the gait score (range of 0-116 with 0 being normal and 116 being the most severe disease).

### Screening Visit

The elbow, knee and ankle on each side of the body will be evaluated for joint disease at screening. Joint function should be evaluated in the absence of an active bleeding episode. A joint score of zero reflects a normal status.

### SCORING DETAILS

1. Joint scoring will be done separately for the 6 joints (LA, RA, LE, RE, LK, RK) according to these categories and scales (range is 0-19 for each joint and 0-114 for all six joints): swelling (0 = none; 1 = mild; 2 = moderate; 3 = severe); duration of swelling (0 = no swelling or ≤ 6 months; 1 = >6 months); muscle atrophy (0 = none; 1 = mild; 2 = severe); crepitus on motion (0 = absent; 1 = present); flexion loss, including loss of plantarflexion of ankles (0 = none; 1 = mild; 2 = moderate; 3 = severe); extension loss, including loss of dorsiflexion of ankles (0 = none; 1 = mild; 2 = moderate; 3 = severe); instability (0 = none; 1 = significant pathologic joint laxity); joint pain (0 = no pain, either through range or at end range of motion; 1 = present); strength (0 = normal (holds position against gravity and maximum resistance); 1 = minimal decrease (holds position against gravity and moderate resistance, but not maximum resistance); 2 = mild decrease (holds position against gravity or minimal resistance); 3 = moderate decrease (able to move joint if gravity eliminated); 4 = severe decrease (trace or no muscle contraction). For scoring flexion loss and extension loss at knees and elbows, the following applies: none = approximately 0-5 degrees; mild = approximately 5-10 degrees; moderate = approximately 11-20 degrees; and severe = approximately >20 degrees.
2. Gait will be scored once (range is 0-2), wherein 0 = no difficulty with walking or climbing up/down stairs; 1 = no difficulty with walking, but difficulty with stairs; and 2 = difficulty with walking and with stairs.

This modified hemophilia joint health score (HJHS) is based on the scoring system used in a joint scoring reliability study in boys with hemophilia (Hilliard, Funk et al., Haemophilia 12(5):518-525 (2006)). It has been used as a tool to evaluate musculoskeletal outcomes in a cohort of 20 boys ages 4-17 years (Saulyte Trakymiene, Ingerslev et al., Haemophilia 16(3):479-486 (2010)). The modifications were done to adapt the scoring system to an adult hemophilia population and according to comments in a recent validation study by the international hemophilia prophylaxis study group (Feldman, Funk et al., Arthritis Care Res (Hoboken), 2010).

### EXAMPLE 6

The main complication of replacement therapy with factor in hemophilia A is the formation of inhibitors (neutralizing anti-factor VIII antibodies) in ~30% of patients with severe hemophilia A. Inhibitor development impacts treatment efficacy as well as the quality of life of affected individuals. Further understanding of how the immune system responds to recombinant factor III (rFVIII) is an ongoing effort in hemophilia research to effectively eradicate inhibitors. The extended half-life rFVIII Fc fusion protein (rFVIIIFc) is an efficacious and well-tolerated therapy to prevent and control bleeding episodes. The Fc region of this molecule is not only responsible for increasing rFVIII half-life but may promote antigen-specific tolerance, as shown in a preclinical animal model (Krishnamoorthy S, et al., Cell Immunol. 301:30-39 (2016)) and as suggested by immune tolerance induction case reports (Groomes CL, et al., Pediatr Blood Cancer 63(5):922-24 (2016); Malec LM, et al., Haemophilia 22(6):e552-e554 (2016); Ragni MV, et al., Haemophilia 22(5):e462-e464 (2016)).

### Methods

Peripheral blood-derived human APCs or THP-1 monocytic cells were used to investigate the effects of rFVIIIFc on FcγR binding, internalization, signaling and cytokine production, and gene expression changes, as well as subsequent interactions and effects on T cells *in vitro* (FIG. 16).

### Results

Decreased cell surface expression of FcγR indicates internalization upon rFVIIIFc treatment (FIGs. 17A-17C). Monocyte-derived macrophages and dendritic cells were treated with horseradish peroxidase immune complexes (HRP-IC) as positive control, human immunoglobulin G1 (IgG1) as negative control, and with recombinant factor VIII (rFVIII) or rFVIII Fc fusion protein (rFVIIIFc) at equimolar concentrations (200 nM) for 24 hours. The cell surface expression of the Fcy receptors (FcγR) CD16 (FIG. 17A), CD32 (FIG. 17B), and CD64 (FIG. 17C) was measured by flow cytometry (n=3; **P≤0.01, ***P≤0.005, significance for HRP-IC to other treatments not shown). Treatment with rFVIIIFc correlated with decreased cell surface expression of CD16 (FIG. 17A), CD32 (FIG. 17B), and CD64 (FIG. 17C), as compared to surface expression following treatment with rFVIII.

rFVIIIFc engages FcγR and induces signaling in monocytes and macrophages, without subsequent proinflammatory cytokine production (FIGs. 18A-18C). THP-1 monocytic cell line, monocytes, peripheral blood monocyte-derived macrophages, and peripheral blood monocyte-derived dendritic cells were treated with HRP-IC, IgG1, rFVIII or rFVIIIFc for 15 minutes (FIG. 18A). Syk phosphorylation was measured in cell lysates using the MSD platform (n=3-7, *P≤0.05). Syk phosphorylation was measured after treating macrophages with rFVIIIFc (WT), with mutant rFVIIIFc that is unable to bind to neonatal Fc receptor (FcRn mutant), or with mutant rFVIIIFc that is unable to bind to FcγR (FcyR mutant) (n=4, *P≤0.05) (FIG. 18B). Proinflammatory cytokine production of the twenty-four-hour treated macrophages were measured by MSD ELISA (n=4, significance not shown) (FIG. 18C).

rFVIIIFc phosphorylates molecules taking part in immunoregulation, rather than molecules playing role in activation and proinflammatory cytokine production (Table 9 and FIG. 19). Phosphorylated proteins in lysates from monocyte-derived macrophages treated with rFVIIIFc for fifteen minutes were queried using the Proteome Profiler phospho-kinase and phospho-immunoreceptor arrays. A list of phosphorylated molecules in rFVIIIFc-treated macrophages identified by the Proteome Profiler assays is shown in Table 9. Phosphorylation of phosphatases responsible for inhibitory signaling were measured using the MSD platform (n=3; **P≤0.01, ***P≤0.005) (FIG. 19).

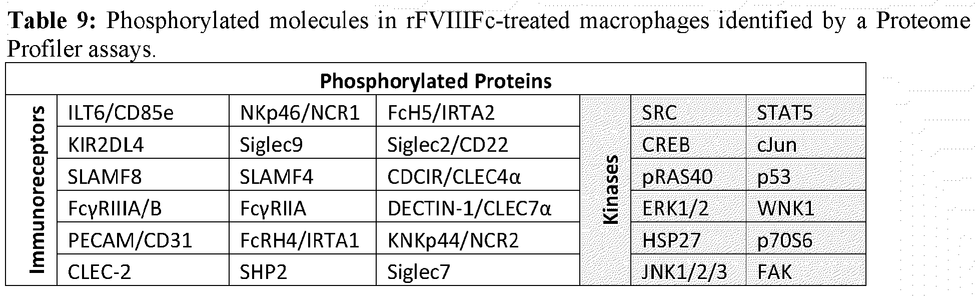

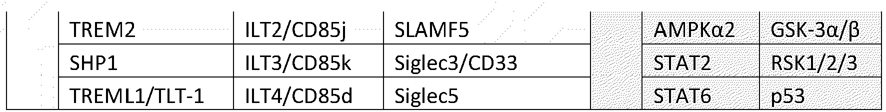

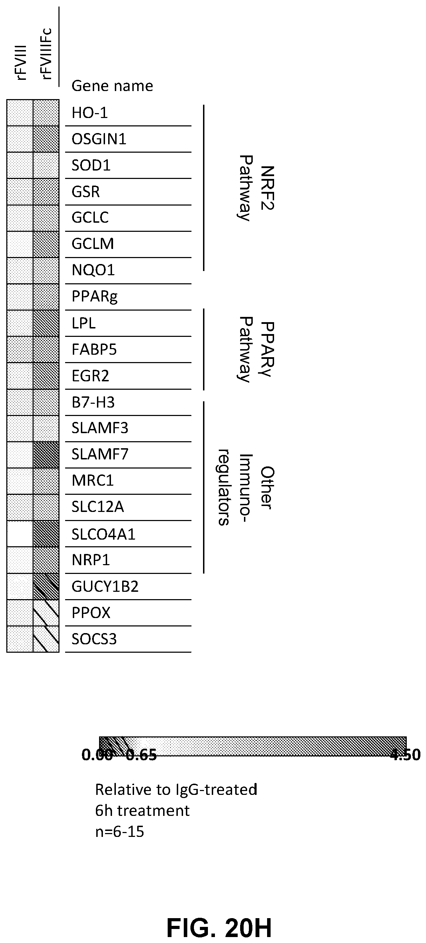

rFVIIIFc induces gene expression pattern characteristic of tolerogenic macrophages (FIGs. 20A-20G). Exploratory RNA sequencing was performed on monocyte-derived macrophages treated with IgG1, rFVIII, or rFVIIIFc for six hours (n=3) for genes that were significantly downregulated (FIG. 20A) and for genes that were significantly upregulated (FIG. 20B), and a pathway analysis was run on the rFVIIIFc-upregulated genes to investigate the molecular pathways represented selectively in these cells, compared to rFVIII-treated cells (Table 9). Various genes of the NRF2 and PPAR-gamma pathways were found to be upregulated, as well as various other immunoregulators (FIG. 20H). Selected genes of the NRF2 and lipid metabolism pathways were validated by Q-PCR (n=8; *P≤0.05, **P≤0.01, ***P≤0.005) (FIGs. 20C-20G). In addition, rFVIIIFc-educated macrophages were found to exhibit a characteristic M2-like phenotype (FIGs. 20I-20M). In particular, macrophages treated with rFVIIIFc had higher relative CD206 expression than cells treated with rFVIII after 6 hours (FIG. 20I) and after 24 hours (FIG. 20J), and macrophages treated with rFVIIIFc had higher relative ARG1 expression than cells treated with rFVIII after 24 hours (FIG. 20M).

**Table 9: Run pathway analysis on the rFVIIIFc-upregulated genes to investigate the molecular pathways represented selectively in these cells, compared to rFVIII-treated cells.**

| **Pathway Name** | **Set Size** | **Candidates Contained** | ***P*-value** | **q-value** |
|---|---|---|---|---|
| NRF2 pathway | 142 | 10 (7.0%) | 4.07e-06 | 0.000273 |
| Liproprotein metabolism | 68 | 7 (10.3%) | 1.01e-05 | 0.000338 |
| Lipid digestion, mobilization, and transport | 110 | 8 (7.3%) | 3.06e-05 | 0.000684 |
| Cysteine and methionine metabolism-Homo sapien (human) | 45 | 5 (11.1%) | 0.000137 | 0.00229 |
| C-MYB transcription factor network | 86 | 6 (7.0%) | 0.000389 | 0.00521 |
| Nuclear receptors meta-pathway | 316 | 11 (3.5%) | 0.000813 | 0.00908 |

rFVIIIFc-treated antigen presenting cells influence regulatory T-cell differentiation that requires APC-T cell-cell contact (FIGs. 21A-21C). Peripheral blood monocyte-derived macrophages were treated with IgG1, rFVIII, or rFVIIIFc, then placed into co-culture with naive CD4 positive T cells isolated from peripheral blood from the same donor. After six days in co-culture (FIG. 21A), the percent of regulatory T cells (CD4+ CD25+ FoxP3+) was quantified using flow cytometry (n=4) (FIG. 21B). The percent of regulatory T cells were also quantified when naïve T cells were cultured in the conditioned media of APCs pretreated with IgG1, rFVIII, or rFVIIIFc (n=4) (FIG. 21C).

### Conclusion

rFVIIIFc appears to bind and induce internalization and signaling through Fcy receptors on APCs. This signaling does not translate to proinflammatory cytokine production and does not activate the APCs (data not shown). Immunomodulatory signaling events are initiated upon rFVIIIFc treatment. These events appear to drive macrophage differentiation towards an M2-like phenotype characterized by the upregulation of NRF2 and PPARγ pathways (FIG. 20H) as well as the upregulation of CD206 and arginase 1 molecules. Various other immunoregulators also showed increased expression, while at least guanylate cyclase 1 soluble subunit beta (2GUCY1B2), protoporphyrinogen oxidase (PPOX), and suppressor of cytokine signaling 3 (SOCS3) showed decreased expression in rFVIIIFc treated cells (FIG. 20H). These macrophages may execute the beneficial immunological effects previously reported, such as regulatory T-cell differentiation, FVIII tolerization, and anti-FVIII inhibitor reduction (FIGs. 22 and 23).

### EXAMPLE 7 (not according to the invention)

The pharmacokinetic (PK) profile of factor IX (FIX) is consistent with a two-compartment model where FIX largely distributes to a space outside of the plasma compartment (extravascular space). We have previously shown using SPECT imaging that the rFIXFc and rFIX biodistribution profiles in hemophilia B mice are consistent with the hypothesis that FIX can distribute to tissues outside of the plasma compartment, while glycoPEGylated-rFIX remains mostly in the plasma compartment due to modifications imposed by the PEG moiety. In this study we evaluated the distribution of rFIX and rFIXFc in nonhuman primates (NHP)

rFIXFc and rFIX were labeled at lysine residues using an 124I-SIB (succinimidyl iodobenzoate). Cynomolgus monkeys received a single IV bolus injection of ~2 mCi tracer dose of 124I-SIB-rFIXFc (2 mg/kg) or 124I-SIB-rFIX (1 mg/kg). Reconstructed whole-body PET/CT scans were used for generating maximum-intensity projection (MIP) images and in vivo biodistribution was determined for regions of interest (ROIs) analysis.

PET imaging in NHP showed an early distribution of 124I-SIB-rFIXFc and 124I-SIB-rFIX in the blood pool, heart, liver and kidneys. At later time points both rFIXFc and rFIX showed distribution to shoulder joints as well as other bone joints (wrist, ankle and jaw) with rFIXFc displaying significantly higher distribution to these areas, even at time points when FIX plasma levels were below the detection level. Both in vivo and ex vivo data showed blood pool clearance of rFIXFc and rFIX. TCA precipitation data showed >95% radioactivity was associated with the FIX.

The rFIXFc and rFIX biodistribution profiles NHP are similar to those observed in hemophilia B mice and are consistent with the hypothesis that FIX can distribute to tissues outside of the plasma compartment. The significantly higher distribution of rFIXFc to joints areas compared to rFIX could potentially reflect retention at these tissues by an Fc-mediated mechanism or improved PK. While the role of extravascular distribution in prevention of bleeding and overall protection of joint health remains an area of investigation, this study is consistent with the observations in mice that demonstrated biodistribution differences for FIX variants suggesting plasma levels may not be comparable, or have the same meaning, across FIX variants.

Other embodiments of the invention will be apparent to those skilled in the art from consideration of the specification and practice of the invention disclosed herein. It is intended that the specification and examples be considered as exemplary only.

## Claims

1. A chimeric protein comprising a factor VIII (FVIII) and a first Fc region derived from human IgG1, for use in reversing hemophilic arthropathy of a target joint in a human subject who has hemophilia A, wherein the hemophilic arthropathy comprises synovitis, wherein the chimeric protein is administered at a dose from 20 IU/kg to 300 IU/kg, wherein the hemophilic arthropathy is reversed in the subject without surgical intervention, and wherein the administration improves a joint health score in the human subject.

2. The chimeric protein for use according to claim 1, wherein the hemophilic arthropathy comprises a microbleed.

3. The chimeric protein for use according to claim 1 or 2, wherein the administration of the chimeric protein reduces angiogenesis and occurrence of microbleeds in the joints.

4. The chimeric protein for use according to any one of claims 1 to 3, wherein the human is 12 years or older.

5. The chimeric protein for use according to any one of claims 1 to 4, wherein the chimeric protein is administered at a dose from 20 IU/kg to 100 IU/kg.

6. The chimeric protein for use according to any one of claims 1 to 5, wherein the chimeric protein is administered at a dose from 25 IU/kg to 65 IU/kg.

7. The chimeric protein for use according to any one of claims 1 to 5, wherein the chimeric protein is administered at a dose from 50 IU/kg to 100 IU/kg.

8. The chimeric protein for use according to any one of claims 1 to 7, wherein the administration reduces joint pain in the human.

9. The chimeric protein for use according to one of claims 1 to 8, wherein the joint is selected from the group consisting of one or both elbows, one or both knees, one or both ankles, one or both shoulders, one or both hips, one or both wrists, one or more joints of the hand, one or more joints of the foot, and any combination thereof.

10. The chimeric protein for use according to any one of claims 1 to 9, further comprising identifying the human in need of the treatment using an imaging system selected from the group consisting of a radiography, a magnetic resonance imaging, an ultrasonography, power Doppler sonography, and any combination thereof

11. The chimeric protein for use according to any one of claims 1 to 10, wherein the chimeric protein is administered at a dosing interval of 3 days to 5 days.

12. The chimeric protein for use according to any one of claims 1 to 11, wherein the FVIII comprises a deletion of all or of part of the B domain of FVIII.

13. The chimeric protein for use according to any one of claims 1 to 12, wherein the chimeric protein comprises a second Fc region that is associated with the first Fc region by a disulfide bond.

14. The chimeric protein for use according to any one of claims 1 to 13, wherein the hemophilic arthropathy is stage I hemophilic arthropathy.

15. The chimeric protein for use according to any one of claims 1 to 13, wherein the hemophilic arthropathy is stage II hemophilic arthropathy.

16. The chimeric protein for use according to any one of claims 1 to 13, wherein the hemophilic arthropathy is stage III hemophilic arthropathy.

## Patentansprüche

1. Chimäres Protein, umfassend einen Faktor VIII (FVIII) und eine erste Fc-Region, die von humanem IgG1 abgeleitet ist, zur Verwendung beim Rückgängigmachen von hämophiler Arthropathie eines Zielgelenks bei einem menschlichen Individuum, das Hämophilie A aufweist, wobei die hämophile Arthropathie Synovitis umfasst, wobei das chimäre Protein in einer Dosis von 20 IE/kg bis 300 IE/kg verabreicht wird, wobei die hämophile Arthropathie in dem Individuum ohne chirurgischen Eingriff rückgängig gemacht wird und wobei die Verabreichung einen Gelenk-Gesundheitsscore in dem menschlichen Individuum verbessert.

2. Chimäres Protein zur Verwendung nach Anspruch 1, wobei die hämophile Arthropathie eine Mikroblutung umfasst.

3. Chimäres Protein zur Verwendung nach Anspruch 1 oder 2, wobei die Verabreichung des chimären Proteins die Angiogenese und das Auftreten von Mikroblutungen in den Gelenken reduziert.

4. Chimäres Protein zur Verwendung nach einem der Ansprüche 1 bis 3, wobei der Mensch 12 Jahre oder älter ist.

5. Chimäres Protein zur Verwendung nach einem der Ansprüche 1 bis 4, wobei das chimäre Protein in einer Dosis von 20 IE/kg bis 100 IE/kg verabreicht wird.

6. Chimäres Protein zur Verwendung nach einem der Ansprüche 1 bis 5, wobei das chimäre Protein in einer Dosis von 25 IE/kg bis 65 IE/kg verabreicht wird.

7. Chimäres Protein zur Verwendung nach einem der Ansprüche 1 bis 5, wobei das chimäre Protein in einer Dosis von 50 IE/kg bis 100 IE/kg verabreicht wird.

8. Chimäres Protein zur Verwendung nach einem der Ansprüche 1 bis 7, wobei die Verabreichung Gelenkschmerzen beim Menschen reduziert.

9. Chimäres Protein zur Verwendung nach einem der Ansprüche 1 bis 8, wobei das Gelenk ausgewählt ist aus der Gruppe bestehend aus einem oder beiden Ellbogen, einem oder beiden Knien, einem oder beiden Fußknöcheln, einer oder beiden Schultern, einer oder beiden Hüften, einem oder beiden Handgelenken, einem oder mehreren Gelenken der Hand, einem oder mehreren Gelenken des Fußes und einer beliebigen Kombination davon.

10. Chimäres Protein zur Verwendung nach einem der Ansprüche 1 bis 9, ferner umfassend das Identifizieren eines Menschen, der die Behandlung benötigt, unter Verwendung eines Bildgebungssystems, das ausgewählt ist aus der Gruppe bestehend aus einer Radiographie, einer Magnetresonanzbildgebung, einer Ultrasonographie, einer Leistungs-Doppler-Sonographie und einer beliebigen Kombination davon.

11. Chimäres Protein zur Verwendung nach einem der Ansprüche 1 bis 10, wobei das chimäre Protein in einem Dosierungsintervall von 3 Tagen bis 5 Tagen verabreicht wird.

12. Chimäres Protein zur Verwendung nach einem der Ansprüche 1 bis 11, wobei der FVIII eine Deletion der gesamten oder eines Teils der B-Domäne von FVIII umfasst.

13. Chimäres Protein zur Verwendung nach einem der Ansprüche 1 bis 12, wobei das chimäre Protein eine zweite Fc-Region umfasst, die durch eine Disulfidbindung mit der ersten Fc-Region assoziiert ist.

14. Chimäres Protein zur Verwendung nach einem der Ansprüche 1 bis 13, wobei die hämophile Arthropathie eine hämophile Arthropathie des Stadiums I ist.

15. Chimäres Protein zur Verwendung nach einem der Ansprüche 1 bis 13, wobei die hämophile Arthropathie eine hämophile Arthropathie des Stadiums II ist.

16. Chimäres Protein zur Verwendung nach einem der Ansprüche 1 bis 13, wobei die hämophile Arthropathie eine hämophile Arthropathie des Stadiums III ist.

## Revendications

1. Protéine chimérique comprenant un facteur VIII (FVIII) et une première région Fc dérivée d'IgG1 humaine, pour utilisation dans l'inversion de l'arthropathie hémophile d'une articulation cible chez un sujet humain atteint d'hémophilie A, dans laquelle l'arthropathie hémophile comprend une synovite, dans laquelle la protéine chimérique est administrée à une dose de 20 UI/kg à 300 UI/kg, dans laquelle l'arthropathie hémophile est inversée chez le sujet sans intervention chirurgicale, et l'administration améliorant un score de santé articulaire chez le sujet humain.

2. Protéine chimérique pour utilisation selon la revendication 1, dans laquelle l'arthropathie hémophile comprend un microsaignement.

3. Protéine chimérique pour utilisation selon la revendication 1 ou 2, dans laquelle l'administration de la protéine chimérique réduit l'angiogenèse et l'apparition de microsaignements dans les articulations.

4. Protéine chimérique pour utilisation selon l'une quelconque des revendications 1 à 3, dans laquelle l'être humain a 12 ans ou plus.

5. Protéine chimérique pour utilisation selon l'une quelconque des revendications 1 à 4, dans laquelle la protéine chimérique est administrée à une dose de 20 UI/kg à 100 UI/kg.

6. Protéine chimérique pour utilisation selon l'une quelconque des revendications 1 à 5, dans laquelle la protéine chimérique est administrée à une dose de 25 UI/kg à 65 UI/kg.

7. Protéine chimérique pour utilisation selon l'une quelconque des revendications 1 à 5, dans laquelle la protéine chimérique est administrée à une dose de 50 UI/kg à 100 UI/kg.

8. Protéine chimérique pour utilisation selon l'une quelconque des revendications 1 à 7, dans laquelle l'administration réduit la douleur articulaire chez l'être humain.

9. Protéine chimérique pour utilisation selon l'une des revendications 1 à 8, dans laquelle l'articulation est choisie dans le groupe constitué d'un ou des deux coudes, d'un ou des deux genoux, d'une ou des deux chevilles, d'une ou des deux épaules, d'une ou des deux hanches, d'un ou des deux poignets, d'une ou plusieurs articulations de la main, d'une ou de plusieurs articulations du pied, et de toute combinaison de ceux-ci.

10. Protéine chimérique pour utilisation selon l'une quelconque des revendications 1 à 9, comprenant en outre l'identification de l'être humain nécessitant le traitement en utilisant un système d'imagerie choisi dans le groupe constitué d'une radiographie, d'une imagerie par résonance magnétique, d'une échographie, d'une échographie Doppler de puissance et de toute combinaison de ceux-ci.

11. Protéine chimérique pour utilisation selon l'une quelconque des revendications 1 à 10, dans laquelle la protéine chimérique est administrée à un intervalle de dosage de 3 jours à 5 jours.

12. Protéine chimérique pour utilisation selon l'une quelconque des revendications 1 à 11, dans laquelle le FVIII comprend une délétion de tout ou partie du domaine B de FVIII.

13. Protéine chimérique pour utilisation selon l'une quelconque des revendications 1 à 12, dans laquelle la protéine chimérique comprend une seconde région Fc qui est associée à la première région Fc par une liaison disulfure.

14. Protéine chimérique pour utilisation selon l'une quelconque des revendications 1 à 13, dans laquelle l'arthropathie hémophile est une arthropathie hémophile de stade I.

15. Protéine chimérique pour utilisation selon l'une quelconque des revendications 1 à 13, dans laquelle l'arthropathie hémophile est l'arthropathie hémophile de stade II.

16. Protéine chimérique pour utilisation selon l'une quelconque des revendications 1 à 13, dans laquelle l'arthropathie hémophile est une arthropathie hémophile de stade III.
